(19) Europäisches Patentamt
European Patent Office
Office européen des brevets

(11) Publication number: **0 405 436 B1**

(12) # EUROPEAN PATENT SPECIFICATION

(45) Date of publication of patent specification: **15.11.95**

(51) Int. Cl.$^6$: **C07D 295/033**, C07D 295/088, C07D 295/182, C07D 239/42, C07D 487/04, A61K 31/495

(21) Application number: **90112113.7**

(22) Date of filing: **26.06.90**

The file contains technical information submitted after the application was filed and not included in this specification

(54) **Dihetero nitrogen-containing cycloheteroethanoanthracene derivatives as antipsychotic agents.**

(30) Priority: **29.06.89 US 374318**

(43) Date of publication of application:
**02.01.91 Bulletin 91/01**

(45) Publication of the grant of the patent:
**15.11.95 Bulletin 95/46**

(84) Designated Contracting States:
**AT BE CH DE DK ES FR GB GR IT LI LU NL SE**

(56) References cited:
**DE-A- 2 626 293**
**FR-M- 6 317**

**CHEMICAL ABSTRACTS, vol. 95, no. 3, July 20, 1981, Columbus, Ohio, USA SHIONOGI AND CO. LTD "Dihydroethanoanthracene derivatives" page 701, column 1 abstract-no. 25 059a.**

**CHEMICAL ABSTRACTS, vol. 88, no. 25, June 18, 1978 Columbus, Ohio, USA SUMITOMO CHMICAL CO "Morpholine derivatives" page 770, column 1 abstract-no. 190 849w**

**JOUNAL OF MEDICINAL CHEMISTRY vol. 10, 1967, BOISSIER J.R. et al "Synthesis and Pharma-cological properties of New 9,10-Dihydro-9-10-ethano-anthracene Derivatives" pages 86-91**

(73) Proprietor: **G.D. Searle & Co.**
**P.O. Box 5110**
**Chicago**
**Illinois 60680 (US)**

(72) Inventor: **Gray, Nancy M.**
**16523 West Glen Farms Drive**
**Ellisville,**
**Missouri 63011 (US)**

(74) Representative: **Beil, Hans Chr., Dr. et al**
**BEIL, WOLFF & BEIL,**
**Rechtsanwälte,**
**Postfach 80 01 40**
**D-65901 Frankfurt (DE)**

EP 0 405 436 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

This invention is in the field of clinical neurology and relates to a class of compounds, compositions and methods useful for treatment of Central Nervous System (CNS) dysfunctions. Of particular interest is a class of ethanoanthracene derivatives useful as antipsychotics, as anticonvulsives and to treat dystonic disorders.

### BACKGROUND OF THE INVENTION

There are many classes of compounds known for treatment of psychotic disorders. For example, current therapeutic treatments for psychoses use compounds classifiable as phenothiazine-thioxanthenes, as phenylbutylpiperidines and also as certain alkaloids. An example of a piperazine-substituted tricyclic compound of current use in psychotic treatment therapy is fluphenazine [A.F. Gilman et al, The Pharmacological Basis of Therapeutics, 7th Edn., p. 403, MacMillan (1985)].

Tricyclic compounds have been investigated for various CNS uses. For example, Belgian Patent No. 706,262 describes a class of diphenylenemethane amine and amide derivatives mentioned for use as anticonvulsants, as well as for anti-depressive, anti-inflammatory and analgesic uses, and mentions in particular the compound 2-[fluorene-9-yl)amino]-acetamide. U.S. Patent No. 3,821,249 describes a series of dibenzothiazepin derivatives asserted to possess psychostimulant, antidepressive, analgesic, antitussive, antihistaminic and gastric antisecretory properties, such series including certain specific 7-[dibenzo(a,d)-cycloheptadien-5-yl]aminoheptanoic acid derivatives and certain specific 7-[chlorodibenzo(b,e)thiepin-11-yl]-aminoheptanoic acid derivatives.

Ethanoanthracene-type compounds have been identified for other pharmaceutical uses. For example, tricyclic oligoamine compounds, such as N,N'-bis-(4-phenylbutyl)-9,10-dihydro-9,10-ethanoanthracene-11,12-bis-methylamine, have been mentioned for use as inhibitors of platelet aggregation (K. Rehse et al, Arch. Pharm. (Neinheim), 320, 829-836 (1987)]. Certain 9,10-dihydro-9,10-ethanoanthracene derivatives, including 4-[(9,10-dihydro-9,10-ethanoanthracenyl)methyl]-1-methylpiperizine, have been described as having anticholinergic, antihistaminic, local anesthetics or hypotensive properties [J. R. Boissier et al, J. Med. Chem., 10, 86-91 (1967)]. U.S. Patent No. 3,422,104 describes 9,10-dihydro-9,10-ethanoanthracene compounds, including 4-[(9,10-dihydro-9,10-ethanoanthracenyl)methyl]-1-(2-hydroxyethyl)piperizine, for use in treatment of depression. Swiss Patent No. 482,642 describes certain 11-aminoalkyl-9,10-dihydro-9,10-ethanoanthracenes as anesthetics. Netherlands Patent Application No. 6,412,205 describes ethanoanthracene derivatives, including 4-methylpiperazide of 9,10-dihydro-9,10-ethanoanthracene-11-carboxylic acid, for antiemetic and anesthetic uses.

### DESCRIPTION OF THE INVENTION

Treatment of a patient afflicted by or susceptible to a central nervous system disorder, such as psychotic, convulsive and dystonic disorders, is provided by administering to the patient a therapeutically-effective amount of a compound of the formula

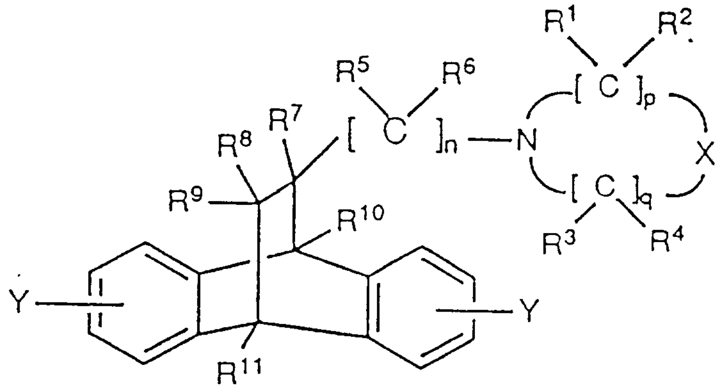

(I)

wherein each of $R^1$ through $R^6$ is independently selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phenyl-$C_1$-$C_{20}$-alkyl, phenyl, $C_1$-$C_{10}$-alkoxyalkyl, $C_1$-$C_{20}$-hydroxyalkyl and halo;

wherein $R^1$ and $R^2$ may be taken together to form oxo; wherein $R^3$ and $R^4$ may be taken together to form oxo; wherein $R^5$ and $R^6$ may be taken together to form oxo; wherein each of $R^7$ through $R^{11}$ is independently selected from hydrido, $C_1$-$C_{20}$-alkyl, hydroxy, $C_3$-$C_{10}$-cycloalkyl, $C_3$-$C_{10}$-cycloalkyl-$C_1$-$C_{20}$-alkyl, phenyl-$C_1$-$C_{20}$-alkyl, phenyl, $C_1$-$C_{10}$-alkoxy, $C_1$-$C_{10}$-alkoxyalkyl, phenyloxy, phenyl-$C_1$-$C_{10}$-alkoxy, $C_1$-$C_{20}$-hydroxyalkyl, halo and $C_1$-$C_{20}$-haloalkyl; wherein $R^8$ and $R^9$ may be taken together to form oxo; wherein n is a number selected from zero to five, inclusive; wherein each of p and q is a number selected from one to four, inclusive; wherein X is selected from O, S, $>$N-$R^{12}$, $>$SO and $>SO_2$; wherein $R^{12}$ may be selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, Phenyl, phenyl-$C_1$-$C_{20}$-alkyl, an aromatic ring system containing one or two heteroatoms selected from oxygen, nitrogen, sulfur in a ring system having five or six ring members, $C_1$-$C_{10}$-alkoxyalkyl, $C_1$-$C_{20}$-hydroxyalkyl, alkanoyl, aralkanoyl, aroyl, amino-$C_1$-$C_{20}$-alkyl, mono-$C_1$-$C_{20}$-alkylamino-$C_1$-$C_{20}$-alkyl and di-$C_1$-$C_{20}$-alkylamino-$C_1$-$C_{20}$-alkyl; wherein $R^{12}$ together with one of $R^1$ through $R^4$ may form a fused heterocyclic ring containing five to eight ring members; wherein each Y is independently one or more groups selected from hydrido, hydroxy, $C_1$-$C_{20}$-alkyl, $C_3$-$C_{10}$-cycloalkyl, $C_3$-$C_{10}$-cycloalkyl-$C_1$-$C_{20}$-alkyl, phenyl-$C_1$-$C_{20}$-alkyl, phenyl, $C_1$-$C_{10}$-alkoxy, phenyl-$C_1$-$C_{10}$-alkoxy, phenyloxy, $C_1$-$C_{10}$-alkoxyalkyl, $C_1$-$C_{20}$-haloalkyl, $C_1$-$C_{20}$-hydroxyalkyl, halo, cyano, amino, mono-$C_1$-$C_{20}$-alkylamino, di-$C_1$-$C_{20}$-alkylamino, nitro, carboxy, carboxy-$C_1$-$C_{20}$-alkyl and alkanoyl; or a pharmaceutically-acceptable salt thereof.

The family of compounds defined by Formula I is believed to embrace novel compounds when Formula I is qualified by the following proviso limitations:

when X is $>NR^{12}$, and each of p and q is two and each of $R^1$ through $R^4$ is hydrido and each of $R^7$ through $R^{11}$ is hydrido, and $R^{12}$ is selected from hydrido, methyl, hydroxyethyl and acetoxy, then $R^5$ together with $R^6$ cannot be oxo and at least one of $R^5$ and $R^6$ must be other than hydrido; and

with the further proviso that when X is oxygen atom and each of p and q is two, then each of the following selections cannot be made:

- $R^5$ and $R^6$ together being oxo when each of $R^1$ through $R^4$, $R^7$ through $R^{11}$ and Y is hydrido;
- each of $R^1$ through $R^{11}$ and Y being hydrido;
- $R^8$ and $R^9$ together being oxo when each of $R^1$ through $R^4$, $R^7$ through $R^{11}$ and Y is hydrido;
- $R^8$ and $R^9$ together being oxo with each of $R^{10}$ and $R^{11}$ being methyl when each of $R^1$ through $R^4$, $R^7$ through $R^9$ and Y is hydrido.

Also excluded from Formula I are quaternary ammonium salts of the compounds embraced by Formula I.

A preferred class of compounds within Formula I consists of those compounds wherein each of $R^1$ through $R^6$ is independently selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxyalkyl, $C_1$-$C_{20}$-hydroxyalkyl and halo; wherein $R^5$ and $R^6$ may be taken together to form oxo; wherein each of $R^7$ through $R^{11}$ is independently selected from hydrido, $C_1$-$C_{20}$-alkyl, $C_1$-$C_{10}$-hydroxy, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxy, $C_1$-$C_{10}$-alkoxyalkyl, phenoxy, phenyl-$C_1$-$C_{10}$-alkoxy, $C_1$-$C_{20}$-hydroxyalkyl, halo and $C_1$-$C_{20}$-haloalkyl; wherein $R^8$ and $R^9$ may be taken together to form oxo; wherein n is a number selected from zero to five, inclusive; wherein each of p and q is a member selected from one to four, inclusive; wherein X is selected from O, S and $>$N-$R^{12}$; wherein $R^{12}$ may be selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phenyl, phen-$C_1$-$C_{20}$-alkyl, an aromatic ring system containing one or two hetero atoms selected from oxygen, nitrogen, sulfur in a ring system having five or six ring members, $C_1$-$C_{10}$-alkoxyalkyl, $C_1$-$C_{20}$-hydroxyalkyl, alkanoyl, phenalkanoyl, aroyl, amino-$C_1$-$C_{20}$-alkyl, mono-$C_1$-$C_{20}$-alkylamino-$C_1$-$C_{20}$-alkyl and di-$C_1$-$C_{20}$-alkylamino-$C_1$-$C_{20}$-alkyl; wherein $R^{12}$ together with one of $R^1$ through $R^4$ form a fused heterocyclic ring containing five to eight ring members; wherein each Y is independently one or more groups selected from hydrido, hydroxy, $C_1$-$C_{20}$-alkyl, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxy, phen-$C_1$-$C_{10}$-alkoxy, phenoxy, $C_1$-$C_{10}$-alkoxyalkyl, $C_1$-$C_{20}$-haloalkyl, $C_1$-$C_{20}$-hydroxyalkyl, halo, cyano, amino, mono-$C_1$-$C_{20}$-alkylamino, di-$C_1$-$C_{20}$-alkylamino, carboxy, carboxy-$C_1$-$C_{20}$-alkyl and alkanoyl; or a pharmaceutically-acceptable salt thereof.

A more preferred class of compounds within Formula I consists of those compounds wherein each of $R^1$ through $R^4$ is independently selected from hydrido, $C_1$-$C_5$-alkyl, cycloalkyl of three to eight carbon atoms, cycloalkylalkyl of four to eight carbon atoms, phenyl-$C_1$-$C_5$-alkyl, phenyl, $C_1$-$C_5$-alkoxy-$C_1$-$C_5$-alkyl, hydroxy-$C_1$-$C_5$-alkyl and halo; wherein each of $R^5$ and $R^6$ is independently selected from hydrido, $C_1$-$C_5$-alkyl, phenyl-$C_1$-$C_5$-alkyl, phenyl and $C_1$-$C_5$-alkoxy-$C_1$-$C_5$-alkyl; wherein $R^5$ and $R^6$ may be taken together to form oxo; wherein each of $R^7$ through $R^{11}$ is independently selected from hydrido, $C_1$-$C_5$-alkyl, hydroxy, cycloalkyl of three to eight carbon atoms, cycloalkylalkyl of four to eight carbon atoms, phenyl-$C_1$-$C_5$-alkyl, phenyl, $C_1$-$C_5$-alkoxy, $C_1$-$C_5$-alkoxy-$C_1$-$C_5$-alkyl, phenoxy, phenyl-$C_1$-$C_{10}$-alkoxy, hydroxy-$C_1$-$C_5$-alkyl, halo and halo-$C_1$-$C_5$-alkyl; wherein $R^8$ and $R^9$ may be taken together to form oxo; wherein n is a number

selected from zero to five, inclusive; wherein each of p and q is two or three; wherein X is selected from O, s, >N-$R^{12}$; wherein $R^{12}$ may be selected from hydrido, $C_1$-$C_5$-alkyl, cycloalkyl of three to eight carbon atoms, cycloalkylalkyl of four to eight carbon atoms, phenyl, phenyl-$C_1$-$C_5$-alkyl, $C_1$-$C_5$-alkoxy-$C_1$-$C_5$-alkyl, hydroxy-$C_1$-$C_5$-alkyl, lower-alkanoyl and heteroaryl selected from saturated, partially unsaturated and fully unsaturated heterocyclic rings containing five to seven ring members of which one or two ring members are selected from oxygen atom and nitrogen atom; wherein $R^{12}$ together with one of $R^1$ through $R^4$ may form a fused heterocyclic ring containing five or six ring members; wherein each Y is independently one or more groups selected from hydrido, hydroxy, $C_1$-$C_5$-alkyl, cycloalkyl of three to eight carbon atoms, cycloalkylalkyl of four to eight carbon atoms, phenyl-$C_1$-$C_5$-alkyl, phenyl, $C_1$-$C_5$-alkoxy, phenoxy, $C_1$-$C_5$-alkoxy-$C_1$-$C_5$-alkyl, halo-$C_1$-$C_5$-alkyl, hydroxy-$C_1$-$C_5$-alkyl, halo, carboxy, carboxy-$C_1$-$C_5$-alkyl and loweralkanoyl; or a pharmaceutically-acceptable salt thereof.

A further preferred class of compounds within Formula I consists of those compounds of Formula II

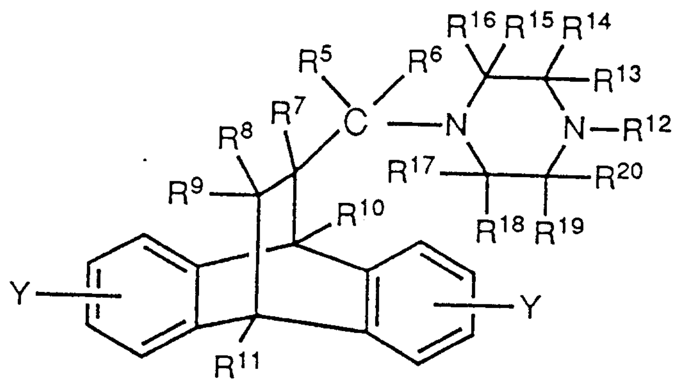

(II)

wherein each of $R^5$ and $R^6$ is independently selected from hydrido, $C_1$-$C_5$-alkyl, benzyl and phenyl; wherein each of $R^7$ through $R^{11}$ is independently selected from hydrido, $C_1$-$C_5$-alkyl, hydroxy, benzyl, phenyl, $C_1$-$C_5$-alkoxy, phenoxy, benzyloxy, halo and halo-$C_1$-$C_5$-alkyl; wherein $R^{12}$ may be selected from hydrido, $C_1$-$C_5$-alkyl, cycloalkyl of five or six carbon atoms, cycloalkylalkyl of six or seven carbon atoms, phenyl, benzyl, hydroxy-$C_1$-$C_5$-alkyl, and heteroaryl selected from saturated or fully unsaturated heterocyclic rings containing five to seven ring members of which one or two ring members are nitrogen atom; wherein each Y is independently one or more groups selected from hydrido, hydroxy, $C_1$-$C_5$-alkyl, benzyl, phenyl, $C_1$-$C_5$-alkoxy, phenoxy, halo-$C_1$-$C_5$-alkyl, halo, and loweralkanoyl; and wherein each of $R^{13}$ through $R^{20}$ is independently selected from hydrido, $C_1$-$C_5$-alkyl, benzyl, phenyl and halo; wherein $R^{12}$ together with one of $R^{13}$, $R^{14}$, $R^{19}$ or $R^{20}$ may form a fused heterocyclic ring containing five or six ring members; or a pharmaceutically-acceptable salt thereof.

A more highly preferred class of compounds consists of those compounds of Formula II wherein each of $R^5$ and $R^6$ is independently hydrido or methyl; wherein each of $R^7$ through $R^{11}$ is independently selected from hydrido, methyl, ethyl, hydroxy, methoxy, halo and trihalomethyl; wherein $R^{12}$ is selected from hydrido, methyl, ethyl, hydroxyethyl, benzyl, pyridyl and pyrimidyl; wherein each of $R^{13}$ through $R^{20}$ is independently selected from hydrido, methyl, ethyl and trihalomethyl; wherein $R^{12}$ together with one of $R^{13}$, $R^{14}$, $R^{19}$ or $R^{20}$ may form a fused heterocyclic ring containing five or six ring members; and wherein each Y is independently one or more groups selected from hydrido, methyl, ethyl, hydroxy, methoxy, trihalomethyl and halo.

Most preferred are the following compounds: 4-[(9,10-dihydro-9,10-ethanoanthracenyl)methyl]-1-(2-pyrimidyl)piperazine; 4-[(9,10-dihydro-9,10-ethanoanthracenyl)methyl]-1,4-diazabicyclo[4.3.0]nonane; and 4-[(9,10-dihydro-9,10-ethanoanthracenyl)methyl]-1-benzylpiperazine.

The term "hydrido" denotes a single hydrogen atom (H) which may be attached, for example, to a an oxygen atom to form hydroxyl group. Where the term "alkyl" is used, either alone or within other terms such as "haloalkyl" and "hydroxyalkyl", the term "alkyl" embraces linear or branched radicals having one to about twenty carbon atoms or, preferably, one to about ten carbon atoms. More preferred alkyl radicals are "lower alkyl" radicals having one to about five carbon atoms. The term "cycloalkyl" embraces cyclic radicals having three to about ten ring carbon atoms, preferably three to about six carbon atoms, such as cyclopropyl and cyclobutyl. The term "haloalkyl" embraces radicals wherein any one or more of the alkyl carbon atoms is substituted with one or more halo groups, preferably selected from bromo, chloro and fluoro. Specifically embraced by the term "haloalkyl" are monohaloalkyl, dihaloalkyl and polyhaloalkyl

groups. A monohaloalkyl group, for example, may have either a bromo, a chloro, or a fluoro atom within the group. Dihaloalkyl and polyhaloalkyl groups may be substituted with two or more of the same halo groups, or may have a combination of different halo groups. A dihaloalkyl group, for example, may have two bromo atoms, such as a dibromomethyl group, or two chloro atoms, such as a dichloromethyl group, or one bromo atom and one chloro atom, such as a bromochloromethyl group. Examples of a polyhaloalkyl are trifluoromethyl, 2,2,2-trifluoroethyl, perfluoroethyl and 2,2,3,3-tetrafluoropropyl groups. The terms "alkylol" and "hydroxyalkyl" embrace linear or branched alkyl groups having one to about ten carbon atoms any one of which may be substituted with one or more hydroxyl groups. The term "alkenyl" embraces linear or branched radicals having two to about twenty carbon atoms, preferably three to about ten carbon atoms, and containing at least one carbon-carbon double bond. The term "alkynyl" embraces linear or branched radicals having two to about twenty carbon atoms, preferably two to about ten carbon atoms, and containing at least one carbon-carbon triple bond. The terms "cycloalkenyl" and "cycloalkynyl" embrace cyclic radicals having three to about ten ring carbon atoms including, respectively, one or more double or triple bonds involving adjacent ring carbons. The terms "alkoxy" and "alkoxyalkyl" embrace linear or branched oxy-containing radicals each having alkyl portions of one to about ten carbon atoms, such as methoxy group. The "alkoxy" or "alkoxyalkyl" radicals may be further substituted with one or more halo atoms, such as fluoro, chloro or bromo, to provide haloalkoxy or haloalkoxyalkyl groups. The term "heteroaryl" embraces aromatic ring systems containing one or two hetero atoms selected from oxygen, nitrogen and sulfur in a ring system having five or six ring members, examples of which are thienyl, furanyl, pyridinyl, thiazolyl, pyrimidyl and isoxazolyl. The term "alkylene chain" describes a chain of two to six methylene ($-CH_2-$) groups which may form a cyclic structure with or without a hetero atom in the cyclic structure. The phrase, as used above, "$R^{12}$ together with one of $R^{13}$, $R^{14}$, $R^{19}$ and $R^{20}$ may form a fused heterocyclic ring containing five or six members", is intended to embrace a bicyclic fused heterocyclic ring system which includes both hetero atoms contained in Formula I and Formula II, which ring system may be further substituted as described herein. An example of such bicyclic fused ring system is shown as Compound No. 4, herein.

Specific examples of alkyl groups are methyl, ethyl, n-propyl, isopropyl, n-butyl, sec-butyl, iso-butyl, tert-butyl, n-pentyl, iso-pentyl, methylbutyl, dimethylbutyl and neopentyl. Typical alkenyl and alkynyl groups may have one unsaturated bond, such as an allyl group, or may have a plurality or unsaturated bonds, with such plurality of bonds either adjacent, such as allene-type structures, or in conjugation, or separated by several saturated carbons.

Included within the family of compounds of Formulas I-II are the tautomeric forms of the described compounds, isomeric forms including diastereoisomers, and the pharmaceutically-acceptable salts thereof. The term "pharmaceutically-acceptable salts" embraces salts commonly used to form alkali metal salts and to form addition salts of free acids or free bases. Since the compounds of Formulas I-II contain basic nitrogen atoms, such salts are typically acid addition salts. The phrase "pharmaceutically-acceptable salts" is not intended to embrace quaternary ammonium salts. The nature of the salt is not critical, provided that it is pharmaceutically acceptable, and acids which may be employed to form such salts are, of course, well known to those skilled in this art. Examples of acids which may be employed to form pharmaceutically acceptable acid addition salts include such inorganic acids as hydrochloric acid, sulfuric acid and phosphoric acid, and such organic acids as maleic acid, succinic acid and citric acid. Other pharmaceutically acceptable salts include salts with alkali metals or alkaline earth metals, such as sodium, potassium, calcium and magnesium, or with organic bases, such as dicyclohexylamine All of these salts may be prepared by conventional means by reacting, for example, the appropriate acid or base with the corresponding compound of Formulas I-II.

## General Synthetic Procedures

Compounds of Formulas I and II may be prepared in accordance with the following generic procedures, within which specific schemes are shown for Formula II type compounds.

Generic Procedure I

Step 1(a):

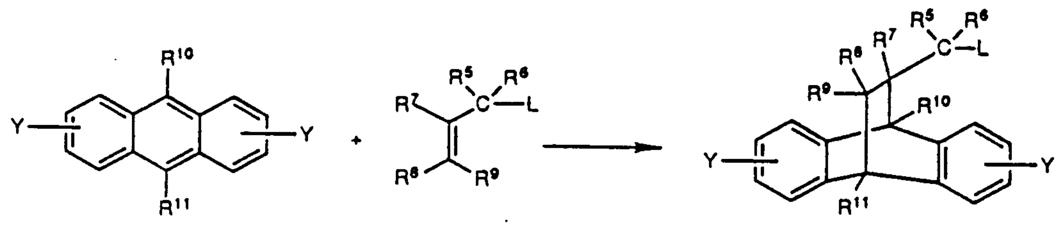

1 2 3

wherein Y and $R^5$ through $R^{11}$ are as previously described; and wherein L is halogen, tosylate, mesylate, brosylate or OH.

A process for preparing the compounds of the invention starts with anthracenes of general structure 1 where Y, $R^{10}$ and $R^{11}$ have the value assigned previously. The anthracene is combined with alkenes of general structure 2 where $R^5$ through $R^9$ have the value assigned previously and L is a good leaving group such as chloro, bromo, mesylate, tosylate or OH. The reaction is best achieved by mixing the reagents neat or in a solvent like benzene, toluene, or xylenes. The reaction temperature can vary from about 150 °C to about 250 °C.

Step 1(b):

Alternately, compounds of general structure 3 can be prepared according to the following generic procedure:

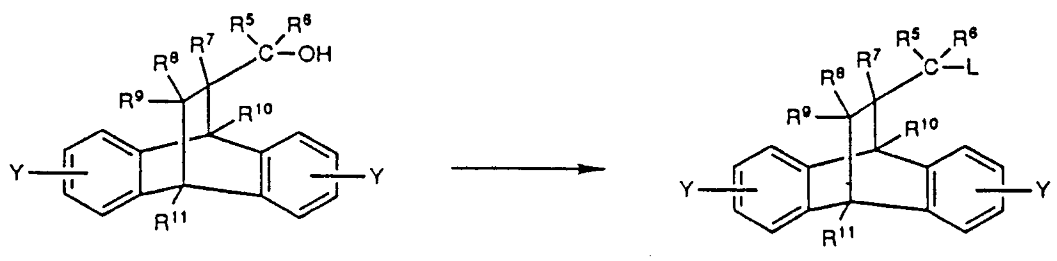

4 3

wherein Y and $R^5$ through $R^{11}$ are as previously described; and wherein L is halogen, tosylate, mesylate, or brosylate. The compounds of general structure 3 thus can be prepared by mixing the alcohol 4 with a reagent such as thionyl chloride, phosphorous oxychloride, triphenylphosphine dibromide, methanesulfonyl chloride and p-toluenesulfonyl chloride. The reagents can be combined neat or in a variety of aprotic solvents such as carbon tetrachloride, toluene, tetrahydrofuran, or ether. The temperature of the reaction may vary from room temperature to reflux of the reaction mixture.

Step 2(a):

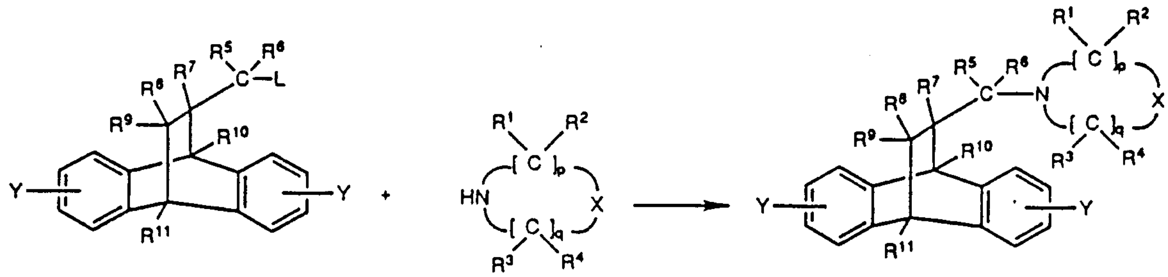

3 5 6

wherein p, q, X, Y, L and $R^1$ through $R^{11}$ are as previously described.

In the second step of the process, amines of general structure 6 are prepared by combining compounds of general structure 3 with amines of general structure 5, where p, q, X, and $R^1$ through $R^4$ are as previously defined. The compounds can be combined in a variety of solvents such as toluene, xylenes, dimethylformamide, hexamethylphosphoramide or ethanol. The temperature of the reaction can vary from room temperature to reflux of the reaction mixture.

Step 2(b):

Alternately, amines of general structure 6 can be prepared according to the following generic procedure:

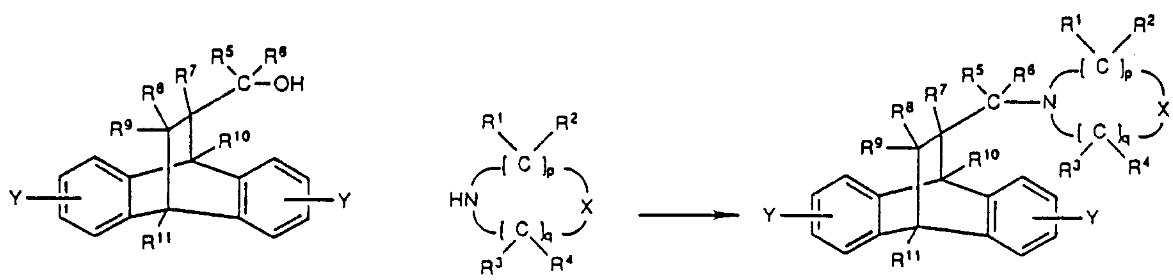

4 5 6

wherein p, q, X, Y and $R^1$ through $R^{11}$ are as previously described.

In Step 2(b) of the process, amines of general structure 6 are prepared by combining an alcohol of general structure 4 with amines of general structure 5, where p, q, X and $R^1$ through $R^4$ are as previously defined. The compounds can be combined in a variety of aprotic solvents such as toluene, xylenes, dimethylformamide, or hexamethylphosphoramide. The conversion requires combining the two reagents in the solvent in the presence of a strong base such as sodium hydride and in the presence of an activating agent such as triphenylphosphine, N-methyl-N-phenylaminotriphenylphosphonium iodide or similar reagents. The temperature of the reaction can vary from room temperature to about 100 °C.

A preferred method for preparing compounds by Generic Procedure I involves the displacement reaction of Step 2 wherein compound of Formula II:

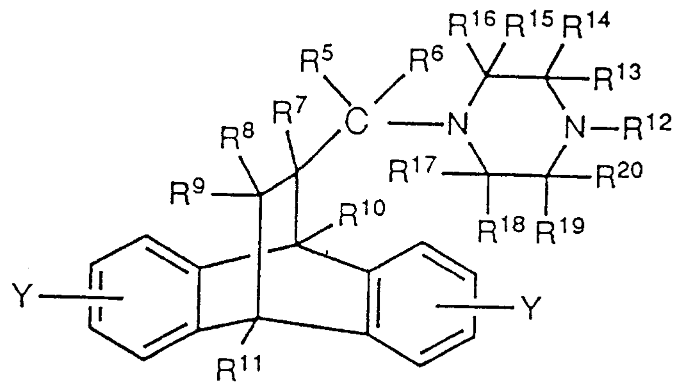

(II)

wherein each of $R^5$ and $R^6$ is independently selected from hydrido, $C_1$-$C_5$-alkyl, benzyl and phenyl; wherein each of $R^7$ through $R^{11}$ is independently selected from hydrido, $C_1$-$C_5$-alkyl, hydroxy, benzyl, phenyl, $C_1$-$C_5$-alkoxy, phenoxy, benzyloxy, halo and halo-$C_1$-$C_5$-alkyl; wherein $R^{12}$ may be selected from hydrido, $C_1$-$C_5$-alkyl, cycloalkyl of five or six carbon atoms, cycloalkylalkyl of six or seven carbon atoms, phenyl, benzyl, hydroxy-$C_1$-$C_5$-alkyl, and heteroaryl selected from saturated or fully unsaturated heterocyclic rings containing five to seven ring members of which one or two ring members are nitrogen atom; wherein each Y is independently one or more groups selected from hydrido, hydroxy, $C_1$-$C_5$-alkyl, benzyl, phenyl, $C_1$-$C_5$-alkoxy, phenoxy, halo-$C_1$-$C_5$-alkyl, halo, and loweralkanoyl; and wherein each of $R^{13}$ through $R^{20}$ is independently selected from hydrido, $C_1$-$C_5$-alkyl, benzyl, phenyl and halo; wherein $R^{12}$ together with one of $R^{13}$, $R^{14}$, $R^{19}$ and $R^{20}$ may form a fused heterocyclic ring containing five or six ring members; or a pharmaceutically-acceptable salt thereof;
said method comprising reacting a compound of the formula

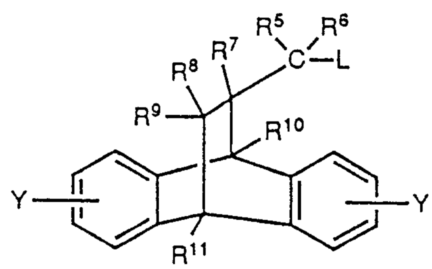

3

wherein each of $R^5$ through $R^{11}$ and Y is defined above; and wherein L is selected from halo, hydroxy, para-toluenesulfonyloxy, methylsulfonyloxy and para-bromotoluenesulfonyloxy; with an amine of the formula

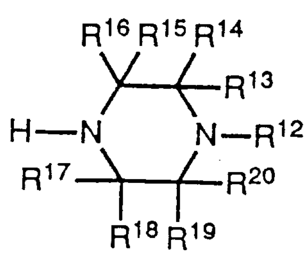

wherein each of $R^{12}$ through $R^{20}$ is as defined above.

The process is especially useful in preparing the compounds 4-[(9,10-dihydro-9,10-ethanoanthracenyl)-methyl]-1-methylpiperazine; 4-[(9,10-dihydro-9,10-ethanoanthracenyl)methyl]-1-(2-hydroxyethyl)-piperazine; 4-[(9,10-dihydro-9,10-ethanoanthracenyl)methyl]-1-(2-pyrimidyl)piperzaine; 4-[(9,10-dihydro-9,10-ethanoanthracenyl)methyl]-1,4-diazabicyclo[4.3.0]nonane; and 4-[(9,10-dihydro-9,10-ethanoanthracenyl)methyl]-1-benzylpiperazine.

Generic Procedure II

Step 1:

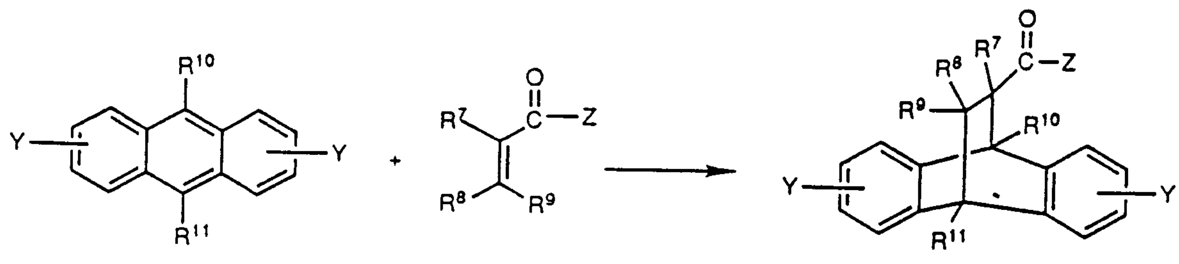

1 7 8

wherein Y and $R^7$ through $R^{11}$ are as defined before;
and wherein Z is selected from lower alkoxy or benzyloxy.

An alternate process that can be used to synthesize the products of the invention starts with anthracenes of general structure 1 where Y, $R^{10}$ and $R^{11}$ have the value assigned previously. The anthracene 1 is combined with acrylates of general structure 7 where Z and $R^7$ through $R^9$ have the value assigned previously. The reaction is best achieved by mixing the reagents neat or in a solvent like benzene, toluene, or xylenes. The reaction temperature can vary from about 150 °C to about 250 °C.

Step 2:

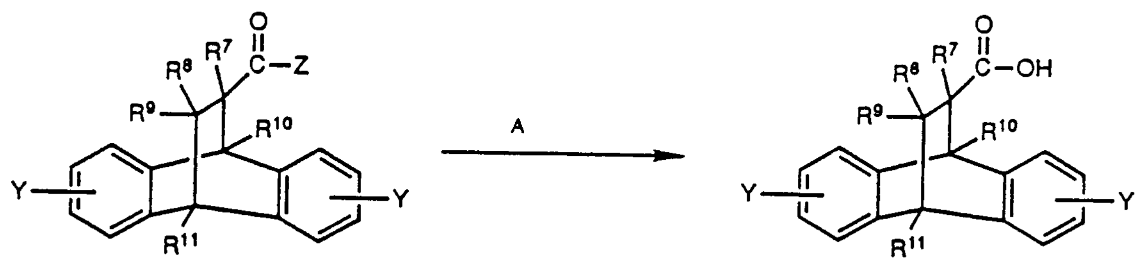


8 9

wherein Y and $R^7$ through $R^{11}$ are as defined before;
wherein Z is selected from lower alkoxy or benzyloxy;
wherein A is selected from a variety of bases such as sodium hydroxide, lithium hydroxide or potassium hydroxide.

In the second step of the process, the ester 8 is hydrolyzed to the acid 9 by mixing the ester with water in the presence of a base such as sodium hydroxide, lithium hydroxide or potassium hydroxide. The reaction is best achieved by mixing the reagents neat or in a solvent such as ethanol or methanol. The reaction temperature can vary from about room temperature to reflux of the reaction mixture.

Step 3

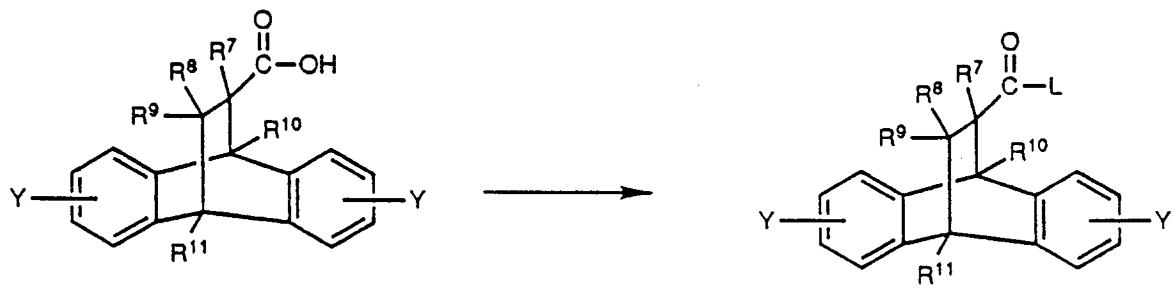

9 10

wherein Y and $R^7$ through $R^{11}$ are as defined before;
and wherein L represents a good leaving group such as chloro, bromo, or acyl.

In the third step of the process, the acid 9 is converted to a compound of the general structure 10, where L is a good leaving group such as chloro, bromo or acyl. The conversion can be best achieved by mixing the acid 9 with reagents such as thionyl chloride, phosphorous oxychloride, phosphorous tribromide, or other reagents. This conversion is best achieved by mixing the reagents neat or in an aprotic solvent such as tetrahydrofuran, methylene chloride, or ether.

The temperature of the reaction can vary from room temperature to reflux of the reaction mixture.

Step 4:

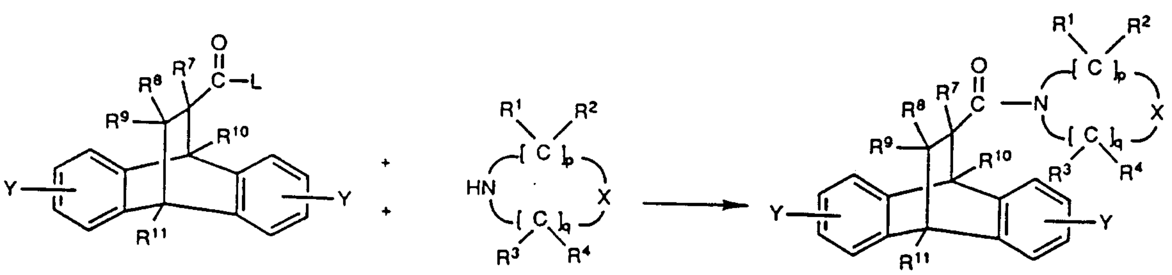

10 5 11

wherein p, q, X, Y, L, $R^1$ through $R^4$ and $R^7$ through $R^{11}$ are as defined before.

In the fourth step of the process, compounds of general structure 10 are converted to amides of general structure 11 by reaction with amines of general structure 5, where p, q, X and $R^1$ through $R^4$ are as defined before. This conversion is best achieved by mixing the reagents neat or in an aprotic solvent such as tetrahydrofuran, methylene chloride, or ether. The temperature of the reaction can vary from 0 °C to reflux of the reaction mixture.

Step 5:

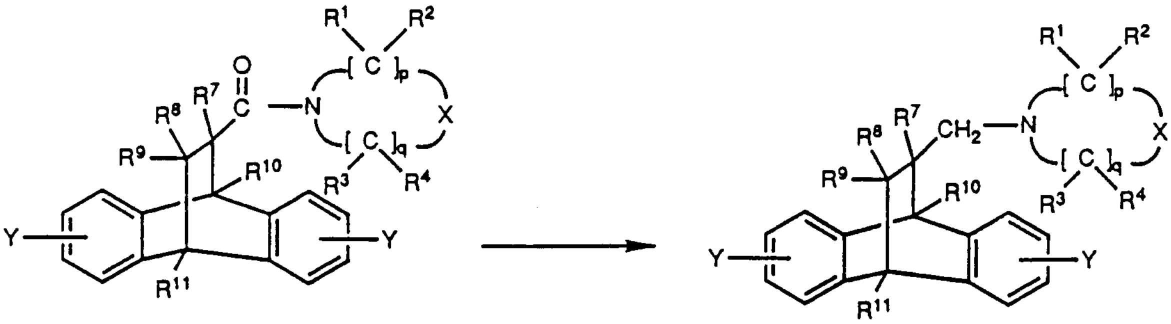

11 6

wherein p, q, X, Y, L, $R^1$ through $R^4$ and $R^7$ through $R^{11}$ are as defined before.

In the fifth step of the process, amides of general structure 11 are converted to amines of general structure 6 by employing reducing agents such as lithium aluminum hydride, sodium borohydride, sodium cyanoborohydride, or other reducing agents familiar to those skilled in the art. This reduction can be accomplished in either protic or aprotic solvents, depending on the reducing agent of choice, and at temperatures ranging from room temperature to reflux of the reaction mixture.

Example I

9,10-Dihydro-9,10-ethanoanthracene-11-methanol

Anthracene (45.4 gm) was combined with allyl alcohol (90.8 gm) and benzene (260 ml) in a Parr bomb and heated to 210 °C for 12 hours. The benzene was removed on a rotary evaporator and the residue was recrystallized from n-heptane to provide the product as a white solid (mp = 104-105 °C).

Example II

11-Bromomethyl-9,10-dihydro-9,10-ethanoanthracene

9,10-Dihydro-9,10-ethanoanthracene-11-methanol (2 gm) was combined with triphenylphosphine dibromide (5.3 gm) in carbon tetrachloride (60 ml). The mixture was heated to reflux 1 hour, filtered, and the filtrate was concentrated on a rotary evaporator. The residue was boiled in n-heptane (75 ml), filtered hot, and the filtrate cooled. The white precipitate was filtered and air dried to provide the product (mp = 103-106 °C).

Example III

11-Bromomethyl-9,10-dihydro-9,10-ethanoanthracene (Alternate Procedure)

9,10-Dihydro-9,10-ethanoanthracene-11-methanol (1.2 gm), imidazole (0.7 gm) and chlorodiphenylphosphine (1.4 gm) were combined in toluene (80 ml) and treated dropwise with bromine (1.0 gm). The mixture was stirred 10 minutes, then extracted with 10% sodium hydroxide (50 ml) and water (50 ml). The toluene was removed on a rotary evaporator and the residue was dissolved in 1:1 methylene chloride/hexane and placed on a silica gel column. The column was eluted with 1:1 methylene chloride/hexane and the eluant removed on a rotary evaporator to give the product.

Example IV

4-[(9,10-Dihydro-9,10-ethanoanthracenyl)methyl]-1-methylpiperazine (Compound No. 1).

11-Bromomethyl-9,10-dihydro-9,10-ethanoanthracene (0.51 gm), 1-methylpiperazine (1.9 ml) and potassium carbonate (0.20 gm) were combined in hexamethylphosphoramide (5 ml) in a sealed tube and heated to 90 °C for 48 hours. The mixture was extracted between water (25 ml) and ether (50 ml). The ether solution was washed with water (25 ml), then extracted with 3.6 N sulfuric acid (3 X 25 ml). The combined acid solutions were made basic with the addition of concentrated aqueous ammonia and the resulting mixture was extracted with ether (3 X 25 ml). The combined ether solutions were dried over magnesium sulfate and the ether removed on a rotary evaporator. The residue was dissolved in anhydrous ether (15 ml) and treated with 3.5% hydrochloric acid in isopropyl alcohol (0.79 ml). The resulting precipitate was filtered, washed with ether (25 ml), and air dried to give the product as a white solid. Analytical data are reported in Table I.

Example V

Methyl 9,10-Dihydro-9,10-ethanoanthracene-11-carboxylate

Anthracene (89.0 gm) was combined with methyl acrylate (51.5 gm) and xylenes (500 ml) in a Parr bomb and heated to 210 °C for 12 hours. The solvent was removed on a rotary evaporator and the residue was recrystallized from methanol to provide the product as a white solid (mp = 113-114 °C).

Example VI

9,10-Dihydro-9,10-ethanoanthracene-11-carboxylic acid

Methyl 9,10-Dihydro-9,10-ethanoanthracene-11-carboxylate (40 gm) was disolved in methanol (500 ml) and combined with 10% sodium hydroxide in water (60 ml). The mixture was heated to reflux for 1.5 hours, cooled to room temperature and treated with water (100 ml). The methanol was removed on a rotary evaporator and the residual solution was diluted with water (200 ml). The solution was washed with ether (2 X 50 ml). The aqueous solution was made acidic by the addition of concentrated hydrochloric acid and the resulting precipitate was filtered. The white solid was dried in vacuo to provide the product (mp = 189-191 °C).

Example VII

4-[(9,10-Dihydro-9,10-ethanoanthracenyl)carbonyl]-1-(2-pyrimidyl)piperazine

9,10-Dihydro-9,10-ethanoanthracene-11-carboxylic acid (1.0 gm) was combined with thionyl chloride (10 ml) and heated to reflux 1 hour. The excess thionyl chloride was removed by distillation and the residue was dissolved in anhydrous ether (10 ml). The acid chloride solution was added dropwise to a solution of 1-(2-pyrimidyl)piperazine (2 gm) in ether (90 ml). The resulting mixture was stirred at room temperature for 1 hour. The mixture was washed with 5% sodium bicarbonate solution (2 X 50 ml) and water (2 X 50 ml) and the ether solution dried over magnesium sulfate. The ether was removed on a rotary evaporator and the crude material purified using preparative centrifugally accelerated radial thin layer chromatography on silica gel using 5% ethanol in methylene chloride as the eluant to provide the product as a white foam.

Example VIII

4-[(9,10-Dihydro-9,10-ethanoanthracenyl)methyl]-1-(2-pyrimidyl)piperazine (Compound No. 3)

4-[(9,10-Dihydro-9,10-ethanoanthracenyl)acetyl]-1-(2- pyrimidyl)piperazine (0.85 gm) was dissolved in anhydrous tetrahydrofuran and treated with lithium aluminum hydride (0.081 gm). The mixture was heated to reflux for 15 hours. The mixture was cooled in an ice bath and treated with 1 N hydrochloric acid (25 ml). The resulting mixture was washed with ether (2 X 25 ml) and the combined ether layers extracted with additional 1 N hydrochloric acid (25 ml). The combined acid solutions were made basic by the addition of excess 50% sodium hydroxide solution and extracted with ether (3 X 35 ml). The combined ether solutions

were dried over magnesium sulfate and the ether removed on a rotary evaporator. The residue was dissolved in isopropyl alcohol (10 ml) and treated with 6 N hydrochloric acid in isoporopyl alcohol (3 ml). The solution was allowed to stand at room temperature for 1 hour, then it was added dropwise to 200 ml ether. The resulting precipitate was filtered and recrystallized from isopropanol to provide the product as a white solid. Analytical data are reported in Table I.

Example IX

4-[(9,10-Dihydro-9,10-ethanoanthracenyl)methyl]-1-benzylpiperazine (Compound No. 6).

9,10-Dihydro-9,10-ethanoanthracene-11-methanol (1.2 gm) was combined with dimethylformamide (5 ml) and treated with a 60% dispersion of sodium hydride in mineral oil (200 mg). The mixture was stirred at room temperature for 15 minutes.

The resulting solution was then treated with a mixture of 1-benzylpiperazine (1.8 gm) and N-methyl-N-phenylaminotriphenylphosphonium iodide (2.5 gm) in dimethylformamide (10 ml). The reaction mixture was heated to 80 °C for 24 hours. The reaction solution was cooled to room temperature, poured into water (75 ml), and the aqueous mixture was extracted with ether (3 X 50 ml). The combined ether solutions were extracted with 3.6 N sulfuric acid (3 X 30 ml) and the combined acid extracts were made basic by the addition of excess concentrated aqueous ammonia. The resulting mixture was extracted with ether (3 X 50 ml) and the combined ether extracts were dried over magnesium sulfate. After removal of the ether on a rotary evaporator, the crude product was purified by preparative centrifugally accelerated radial thin layer chromatography on silica gel using 5% ethanol in methylene chloride as the eluant to provide a white solid. The solid was dissolved in anhydrous ether (50 ml), treated with 3 N hydrochloric acid in isopropyl alcohol (1 ml), and the resulting precipitate was filtered. The precipitate was recrystallized from ethanol to provide the product as a white solid. Analytical data are reported in Table I.

Table I

| Compound Number | Name | Structure | Method of Preparation | Elemental Analysis | Theor. | Found | Melting Point |
|---|---|---|---|---|---|---|---|
| 1 | 4-[(9,10-dihydro-9,10-ethanoanthracenyl)methyl]-1-methylpiperazine·1.5HCl·0.8$H_2O$ | 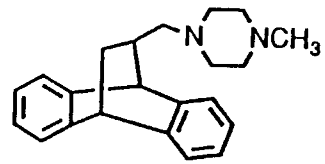 | I, II, IV or V-VIII | C<br>H<br>N | 68.24<br>7.57<br>.7.23 | 68.25<br>7.53<br>7.11 | 236-241°C |
| 2 | 4-[(9,10-dihydro-9,10-ethanoanthracenyl)methyl]-1-(2-hydroxyethyl)-piperazine·1.9HCl·0.6$H_2O$ | 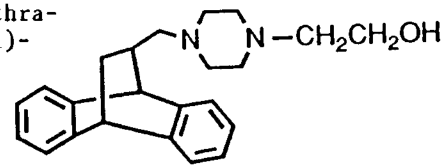 | I, II, IV | C<br>H<br>N | 64.13<br>7.30<br>6.50 | 64.13<br>7.29<br>6.50 | 282-286°C |
| 3 | 4-[(9,10-dihydro-9,10-ethanoanthracenyl)methyl]-1-(2-pyrimidyl)piperazine·2HCl·0.9$H_2O$ | 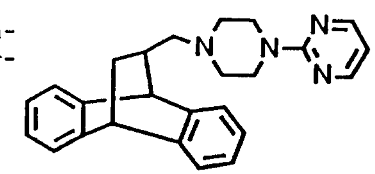 | V, VIII | C<br>H<br>N | 63.58<br>6.36<br>11.80 | 63.66<br>6.36<br>11.27 | 276-279°C |
| 4 | 4-[(9,10-dihydro-9,10-ethanoanthracenyl)methyl]-1,4-diazabicyclo[4.3.0]-nonane·1.9HCl·1.4$H_2O$ | 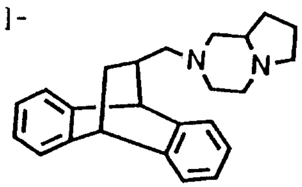 | V, VIII | C<br>H<br>N | 65.58<br>7.51<br>6.37 | 65.59<br>7.35<br>6.21 | 213-216°C |

Table I (Cont'd.)

| Compound Number | Name | Structure | Method of Preparation | Elemental Analysis Theor. | Elemental Analysis Found | Melting Point |
|---|---|---|---|---|---|---|
| 5 | 4-[(9,10-dihydro-9,10-ethanoanthracenyl)carbonyl]-1-methylpiperazine· 0.3EtOH (comparison compound disclosed in Netherland Patent application 6 412 205) | 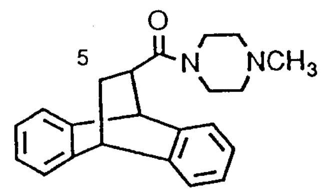 | V, VII | C 78.25<br>H 7.54<br>N 8.05 | 78.36<br>7.38<br>7.58 | 73-76°C |
| 6 | 4-[(9,10-dihydro-9,10-ethanoanthracenyl)methyl]-1-benzylpiperazine· $2HCl\cdot0.9H_2O$ | 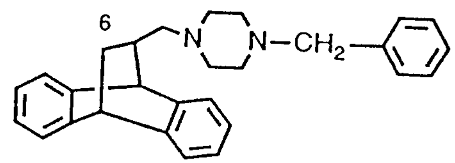 | IX | C 71.27<br>H 6.94<br>N 5.94 | 71.25<br>6.98<br>5.89 | 254-256°C |

## Biological Evaluation

### Radioreceptor Assay

Compounds 1-6 were compared against di-o-tolylguanidine (DTG) [E. Weber et al, Proc. Natl. Acad. Sci., 83, 8784- 8788, 1986] to determine the relative potency of the compounds interacting with the sigma receptor. To determine the effects of the compounds in a sigma receptor assay, crude membrane preparations were prepared as follows. Brains from male Sprague-Dawley rats were homogenized in 10 volumes (wt/vol) of 0.32 M sucrose, using a Polytron grinder. The homogenate was centrifuged at 900 X G for 10 minutes at 4°C. The supernatant was collected and centrifuged at 22,000 X g for 20 minutes at 4°C. The pellet was resuspended in 10 volumes of 50 mM Tris/HCl buffer (pH 7.4) and centrifuged at 22,000 X g for 20 minutes at 4°C. The pellet was resuspended in 5 mM Tris/HCl buffer (pH 7.4) to give a final concentration of 250 mg/ml of the crude material. Incubation tubes were prepared in triplicate and contained 0.1 ml of tissue suspension, 2 nM of [$^3$H]-(+)-1-propyl-3-(3-hydroxyphenyl)piperidine {[$^3$H]-3-(+)-PPP}, and varying concentrations of the displacing ligand (0.1-1000 nM) in a final volume of 0.5 ml. After a 1 hr incubation at room temperature, the contents of the test tubes were filtered through GS filter paper which had been presoaked for at least 2 hours in 0.05% polyethyleneimine. The test tubes were rinsed three times with Tris/HCl buffer. Radioactivity on the filters was determined using the method of Cheng and Prusoff [Biochem. Pharmacol., 22, 3099-3108, 1973].

Table II

| Test Compound | Ki apparent (nM) (units ± SEM) |
|---|---|
| DTG | 47 ± 5 |
| Compound No. 1 | 120 ± 20 |
| Compound No. 2 | 21 ± 6 |
| Compound No. 3 | >1000 |
| Compound No. 4 | 20 ± 3 |
| Compound No. 5 (comparison compound) | >1000 |
| Compound No. 6 | 98 ± 18 |

### Blockade of Agonist-induced Stereotyped Behavior and Ataxia

Compounds of the invention were evaluated for their ability to block the effects of N-allylnormetazocine on the induction of stereotyped behavior and ataxia. To test for antagonism, drugs are administered at varying times before i.p. administration of 15 mg/kg of N-allylnormetazocine. Behavioral and ataxia ratings are taken at 2.5 minutes, 5 minutes, and every 5 minutes thereafter until the animal returns to control behavior. The rating scale for stereotyped behavior is; (0) inactive or in-place non-repetitive activity; (1) sniffing, grooming, or rearing; (2) undirected head movements, reciprocal forepaw treading or a greater frequency of sniffing than in (1); (3) appearance of circling, weaving or backward walking; (4) gagging or continuous circling, weaving or backward walking; and (5) dyskinetic extension or flexation of head, neck and limbs, or rapid and continuous weaving greater than (4). The rating scale for ataxia is: (0) inactive or coordinated movements; (1) awkward or jerky movements or loss of balance while rearing; (2) stumbles or awkward position; (3) falling or leaning against cage; (4) supports weight on stomach or haunches; and (5) unable to move except for twitching movements. The lowest dose of the test compound which is capable of blocking the stereotyped behavior and ataxia induced by N-allylnormetazocine was determined. For example, at a dose of 1 mg/kg i.p., Compound No. 1 fully blocked N-allylnormetazocine-induced stereotyped behavior, while a dose of 5 mg/kg fully blocked N-allylnormetazocine-induced ataxia.

### Blockade of Apomorphine-induced Climbing

Compounds of the invention were evaluated for their ability to block apomorphine-induced climbing. The evaluation of the compounds followed the method outlined by Protais [Psychopharmacol., 50, 1-6, 1976]. Swiss-Webster mice, weighing 20-25 g, are pretreated with the compounds of the invention by i.p. or s.c. administration at various times before 2 mg/kg apomorphine is administered s.c in a volume of 1 ml/kg. All test compounds are administered in a volume of 10 ml/kg. Mice are rated at 10 and 20 minutes after

apomorphine administration using the following rating scale: (0) forepaws on the floor, (1) forefeet holding the bars, and (2) four paws holding bars. Dose-response curves are analyzed by a computerized Finney assay [Statistical Methods in Biological Assays, 2nd Edn., Hatner Pub. Co., New York (1964)].

Table III

| Test Compound | Blockade of Apomorphine-induced Climbing $ED_{50}$ (mg/kg) |
|---|---|
| Compound No. 1 | 1.0 |
| Compound No. 2 | 1.0 |
| Compound No. 6 | 10.0 |

Also embraced within this invention is a class of pharmaceutical compositions comprising one or more compounds of Formula I in association with one or more non-toxic, pharmaceutically acceptable carriers and/or diluents and/or adjuvants (collectively referred to herein as "carrier" materials) and, if desired, other active ingredients. The compounds of the present invention may be administered by any suitable route, preferably in the form of a pharmaceutical composition adapted to such a route, and in a dose effective for the treatment intended. Therapeutically effective doses of the compounds of the present invention required to prevent or arrest the progress of the medical condition are readily ascertained by one of ordinary skill in the art. The compounds and composition may, for example, be administered intravascularly, intraperitoneally, subcutaneously, intramuscularly or topically.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. These may with advantage contain an amount of active ingredient from about 1 to 250 mg, preferably from about 25 to 150 mg. A suitable daily dose for a mammal may vary widely depending on the condition of the patient and other factors. However, a dose of from about 0.1 to 3000 mg/kg body weight, particularly from about 1 to 100 mg/kg body weight, may be appropriate.

The active ingredient may also be administered by injection as a composition wherein, for example, saline, dextrose or water may be used as a suitable carrier. A suitable daily dose is from about 0.1 to 100 mg/kg body weight injected per day in multiple doses depending on the disease being treated. A preferred daily dose would be from about 1 to 30 mg/kg body weight. Compounds indicated for prophylactic therapy will preferably be administered in a daily dose generally in a range from about 0.1 mg to about 100 mg per kilogram of body weight per day. A more preferred dosage will be a range from about 1 mg to about 100 mg per kilogram of body weight. Most preferred is a dosage in a range from about 1 to about 50 mg per kilogram of body weight per day. A suitable dose can be administered, in multiple sub-doses per day. These sub-doses may be administered in unit dosage forms. Typically, a dose or sub-dose may contain from about 1 mg to about 100 mg of active compound per unit dosage form. A more preferred dosage will contain from about 2 mg to about 50 mg of active compound per unit dosage form. Most preferred is a dosage form containing from about 3 mg to about 25 mg of active compound per unit dose.

The dosage regimen for treating a disease condition with the compounds and/or compositions of this invention is selected in accordance with a variety of factors, including the type, age, weight, sex and medical condition of the patient, the severity of the disease, the route of administration, and the particular compound employed, and thus may vary widely.

For therapeutic purposes, the compounds of this invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

**Claims**

**Claims for the following Contracting States : AT, BE, CH, DE, DK, FR, GB, IT, LI, LU, NL, SE**

1. A compound of the formula

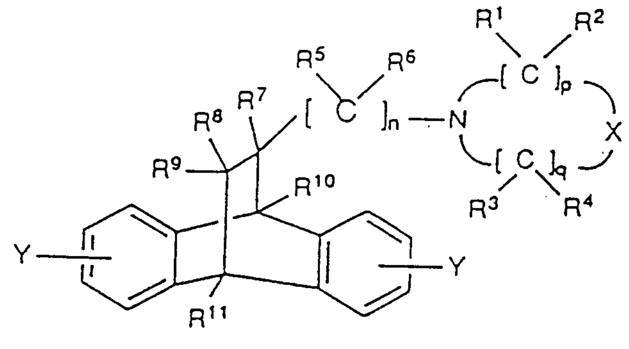

wherein each of $R^1$ through $R^6$ is independently selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phenyl-$C_1$-$C_{20}$-alkyl, phenyl, $C_1$-$C_{10}$-alkoxyalkyl, $C_1$-$C_{20}$-hydroxyalkyl and halo; wherein $R^1$ and $R^2$ may be taken together to form oxo; wherein $R^3$ and $R^4$ may be taken together to form oxo; wherein $R^5$ and $R^6$ may be taken together to form oxo; wherein each of $R^7$ through $R^{11}$ is independently selected from hydrido, $C_1$-$C_{20}$-alkyl, hydroxy, $C_3$-$C_{10}$-cycloalkyl, $C_3$-$C_{10}$-cycloalkyl-$C_1$-$C_{20}$-alkyl, phenyl-$C_1$-$C_{20}$-alkyl, phenyl, $C_1$-$C_{10}$-alkoxy, $C_1$-$C_{10}$-alkoxyalkyl, phenyloxy, phenyl-$C_1$-$C_{10}$-alkoxy, $C_1$-$C_{20}$-hydroxyalkyl, halo and $C_1$-$C_{20}$-haloalkyl; wherein $R^8$ and $R^9$ may be taken together to form oxo; wherein n is a number selected from zero to five, inclusive; wherein each of p and q is a number selected from one to four, inclusive; wherein X is selected from O, S, $>$N-$R^{12}$, $>$SO and $>SO_2$; wherein $R^{12}$ may be selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phenyl, phenyl-$C_1$-$C_{20}$-alkyl, an aromatic ring system containing one or two heteroatoms selected from oxygen, nitrogen, sulfur in a ring system having five or six ring members, $C_1$-$C_{10}$-alkoxyalkyl, $C_1$-$C_{20}$-hydroxyalkyl, alkanoyl, aralkanoyl, aroyl, amino-$C_1$-$C_{20}$-alkyl, mono-$C_1$-$C_{20}$-alkylamino-$C_1$-$C_{20}$-alkyl and di-$C_1$-$C_{20}$-alkylamino-$C_1$-$C_{20}$-alkyl; wherein $R^{12}$ together with one of $R^1$ through $R^4$ may form a fused heterocyclic ring containing five to eight ring members; wherein each Y is independently one or more groups selected from hydrido, hydroxy, $C_1$-$C_{20}$-alkyl, $C_3$-$C_{10}$-cycloalkyl, $C_3$-$C_{10}$-cycloalkyl-$C_1$-$C_{20}$-alkyl, phenyl-$C_1$-$C_{20}$-alkyl, phenyl, $C_1$-$C_{10}$-alkoxy, phenyl-$C_1$-$C_{10}$-alkoxy, phenyloxy, $C_1$-$C_{10}$-alkoxyalkyl, $C_1$-$C_{20}$-haloalkyl, $C_1$-$C_{20}$-hydroxyalkyl, halo, cyano, amino, mono-$C_1$-$C_{20}$-alkylamino, di-$C_1$-$C_{20}$-alkylamino, nitro, carboxy, carboxy-$C_1$-$C_{20}$-alkyl and alkanoyl; or a pharmaceutically-acceptable salt thereof; with the proviso that when X is $>NR^{12}$, and each of p and q is two and each of $R^1$ through $R^4$ is hydrido and each of $R^7$ through $R^{11}$ is hydrido, and $R^{12}$ is selected from hydrido, methyl, hydroxyethyl and acetoxy, then $R^5$ together with $R^6$ cannot be oxo and at least one of $R^5$ and $R^6$ must be other than hydrido; and with the further proviso that when X is oxygen atom and each of p and q is two, then each of the following selections cannot be made:

- $R^5$ and $R^6$ together being oxo when each of $R^1$ through $R^4$, $R^7$ through $R^{11}$ and Y is hydrido;
- each of $R^1$ through $R^{11}$ and Y being hydrido;
- $R^8$ and $R^9$ together being oxo when each of $R^1$ through $R^4$, $R^7$ through $R^{11}$ and Y is hydrido;
- $R^8$ and $R^9$ together being oxo with each of $R^{10}$ and $R^{11}$ being methyl when each of $R^1$ through $R^4$, $R^7$ through $R^9$ and Y is hydrido.

2. Compound of Claim 1 wherein each of $R^1$ through $R^6$ is independently selected from hydrido; $C_1$-$C_{20}$-alkyl, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxyalkyl, $C_1$-$C_{20}$-hydroxyalkyl and halo; wherein $R^5$ and $R^6$ may be taken together to form oxo; wherein each of $R^7$ through $R^{11}$ is independently selected from hydrido, $C_1$-$C_{20}$-alkyl, $C_1$-$C_{10}$-hydroxy, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxy, $C_1$-$C_{10}$-alkoxyalkyl, phenoxy, phenyl-$C_1$-$C_{10}$-alkoxy, $C_1$-$C_{20}$-hydroxyalkyl, halo and $C_1$-$C_{20}$-haloalkyl; wherein $R^8$ and $R^9$ may be taken together to form oxo; wherein n is a number selected from zero to five, inclusive; wherein each of p and q is a member selected from one to four, inclusive; wherein X is selected from O, S and $>$N-$R^{12}$; wherein $R^{12}$ may be selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phenyl, phen-$C_1$-$C_{20}$-alkyl, an aromatic ring system containing one or two hetero

atoms selected from oxygen, nitrogen, sulfur in a ring system having five or six ring members, $C_1$-$C_{10}$-alkoxyalkyl, $C_1$-$C_{20}$-hydroxyalkyl, alkanoyl, phenalkanoyl, aroyl, amino-$C_1$-$C_{20}$-alkyl, mono-$C_1$-$C_{20}$-alkylamino-$C_1$-$C_{20}$-alkyl and di-$C_1$-$C_{20}$-alkylamino-$C_1$-$C_{20}$-alkyl; wherein $R^{12}$ together with one of $R^1$ through $R^4$ form a fused heterocyclic ring containing five to eight ring members; wherein each Y is independently one or more groups selected from hydrido, hydroxy, $C_1$-$C_{20}$-alkyl, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxy, phen-$C_1$-$C_{10}$-alkoxy, phenoxy, $C_1$-$C_{10}$-alkoxyalkyl, $C_1$-$C_{20}$-haloalkyl, $C_1$-$C_{20}$-hydroxyalkyl, halo, cyano, amino, mono-$C_1$-$C_{20}$-alkylamino, di-$C_1$-$C_{20}$-alkylamino, carboxy, carboxy-$C_1$-$C_{20}$-alkyl and alkanoyl; or a pharmaceutically-acceptable salt thereof.

**3.** Compound of Claim 2 wherein each of $R^1$ through $R^4$ is independently selected from hydrido, $C_1$-$C_5$-alkyl, cycloalkyl of three to eight carbon atoms, cycloalkylalkyl of four to eight carbon atoms, phenyl-$C_1$-$C_5$-alkyl, phenyl, $C_1$-$C_5$-alkoxy-$C_1$-$C_5$-alkyl, hydroxy-$C_1$-$C_5$-alkyl and halo; wherein each of $R^5$ and $R^6$ is independently selected from hydrido, $C_1$-$C_5$-alkyl, phenyl-$C_1$-$C_5$-alkyl, phenyl and $C_1$-$C_5$-alkoxy-$C_1$-$C_5$-alkyl; wherein $R^5$ and $R^6$ may be taken together to form oxo; wherein each of $R^7$ through $R^{11}$ is independently selected from hydrido, $C_1$-$C_5$-alkyl, hydroxy, cycloalkyl of three to eight carbon atoms, cycloalkylalkyl of four to eight carbon atoms, phenyl-$C_1$-$C_5$-alkyl, phenyl, $C_1$-$C_5$-alkoxy, $C_1$-$C_5$-alkoxy-$C_1$-$C_5$-alkyl, phenoxy, phenyl-$C_1$-$C_{10}$-alkoxy, hydroxy-$C_1$-$C_5$-alkyl, halo and halo-$C_1$-$C_5$-alkyl; wherein $R^8$ and $R^9$ may be taken together to form oxo; wherein n is a number selected from zero to five, inclusive; wherein each of p and q is two or three; wherein X is selected from O, S, $>$N-$R^{12}$; wherein $R^{12}$ may be selected from hydrido, $C_1$-$C_5$-alkyl, cycloalkyl of three to eight carbon atoms, cycloalkylalkyl of four to eight carbon atoms, phenyl, phenyl-$C_1$-$C_5$-alkyl, $C_1$-$C_5$-alkoxy-$C_1$-$C_5$-alkyl, hydroxy-$C_1$-$C_5$-alkyl, lower-alkanoyl and heteroaryl selected from saturated, partially unsaturated and fully unsaturated heterocyclic rings containing five to seven ring members of which one or two ring members are selected from oxygen atom and nitrogen atom; wherein $R^{12}$ together with one of $R^1$ through $R^4$ may form a fused heterocyclic ring containing five or six ring members; wherein each Y is independently one or more groups selected from hydrido, hydroxy, $C_1$-$C_5$-alkyl, cycloalkyl of three to eight carbon atoms, cycloalkylalkyl of four to eight carbon atoms, phenyl-$C_1$-$C_5$-alkyl, phenyl, $C_1$-$C_5$-alkoxy, phenoxy, $C_1$-$C_5$-alkoxy-$C_1$-$C_5$-alkyl, halo-$C_1$-$C_5$-alkyl, hydroxy-$C_1$-$C_5$-alkyl, halo, carboxy, carboxy-$C_1$-$C_5$-alkyl and loweralkanoyl; or a pharmaceutically-acceptable salt thereof.

**4.** Compound of Claim 3 of the formula

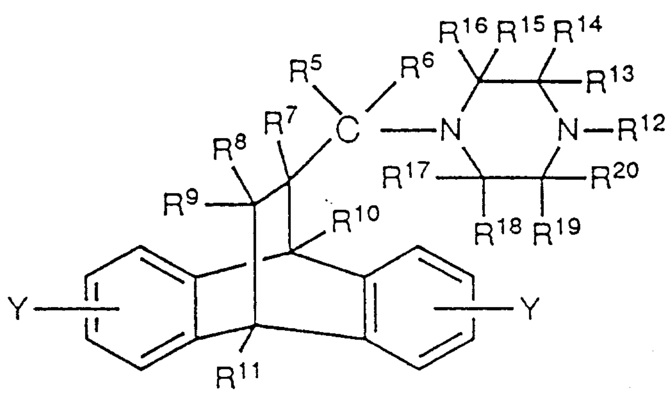

wherein each of $R^5$ and $R^6$ is independently selected from hydrido, $C_1$-$C_5$-alkyl, benzyl and phenyl; wherein each of $R^7$ through $R^{11}$ is independently selected from hydrido, $C_1$-$C_5$-alkyl, hydroxy, benzyl, phenyl, $C_1$-$C_5$-alkoxy, phenoxy, benzyloxy, halo and halo-$C_1$-$C_5$-alkyl; wherein $R^{12}$ may be selected from hydrido, $C_1$-$C_5$-alkyl, cycloalkyl of five or six carbon atoms, cycloalkylalkyl of six or seven carbon atoms, phenyl, benzyl, hydroxy-$C_1$-$C_5$-alkyl, and heteroaryl selected from saturated or fully unsaturated heterocyclic rings containing five to seven ring members of which one or two ring members are nitrogen atom; wherein each Y is independently one or more groups selected from hydrido, hydroxy, $C_1$-$C_5$-alkyl, benzyl, phenyl, $C_1$-$C_5$-alkoxy, phenoxy, halo-$C_1$-$C_5$-alkyl, halo, and loweralkanoyl; and wherein each of $R^{13}$ through $R^{20}$ is independently selected from hydrido, $C_1$-$C_5$-alkyl, benzyl, phenyl and halo; wherein $R^{12}$ together with one of $R^{13}$, $R^{14}$, $R^{19}$ or $R^{20}$ may form a fused heterocyclic ring containing five or six ring members; or a pharmaceutically-acceptable salt thereof.

**5.** Compound of Claim 4 wherein each of $R^5$ and $R^6$ is independently hydrido or methyl; wherein each of $R^7$ through $R^{11}$ is independently selected from hydrido, methyl, ethyl, hydroxy, methoxy, halo and trihalomethyl; wherein $R^{12}$ is selected from hydrido, methyl, ethyl, hydroxyethyl, benzyl, pyridyl and pyrimidyl; wherein each of $R^{13}$ through $R^{20}$ is independently selected from hydrido, methyl and ethyl; wherein $R^{12}$ together with one of $R^{13}$, $R^{14}$, $R^{19}$ or $R^{20}$ may form a fused heterocyclic ring containing five or six ring members; and wherein each Y is independently one or more groups selected from hydrido, methyl, ethyl, hydroxy, methoxy, trihalomethyl and halo.

**6.** Compound of Claim 5 selected from 4-[(9,10-dihydro-9,10-ethanoanthracenyl)methyl]-1-(2-pyrimidyl)-piperazine; 4-[(9,10-dihydro-9,10-ethanoanthracenyl)methyl]-1,4-diazabicyclo[4.3.0]nonane; and 4-[(9,10-dihydro-9,10-ethanoanthracenyl)methyl]-1-benzylpiperazine.

**7.** A pharmaceutical composition comprising a therapeutically-effective amount of an active compound and a pharmaceutically-acceptable carrier or diluent, said active compound selected from a family of compounds according to any of Claims 1, 2, 3, 4, 5 or 6.

**8.** Use of a therapeutically-effective amount of a compound according to any of Claims 1, 2, 4, 5 or 6 for preparing a medicament for treating a central nervous system disorder.

**9.** Use according to Claim 8 wherein said central nervous system disorder is selected from a psychotic disorder, a convulsive disorder and a dystonic disorder.

**10.** Use according to Claim 9 wherein said central nervous system disorder is a psychotic disorder.

**11.** A process for preparing a product compound of the formula

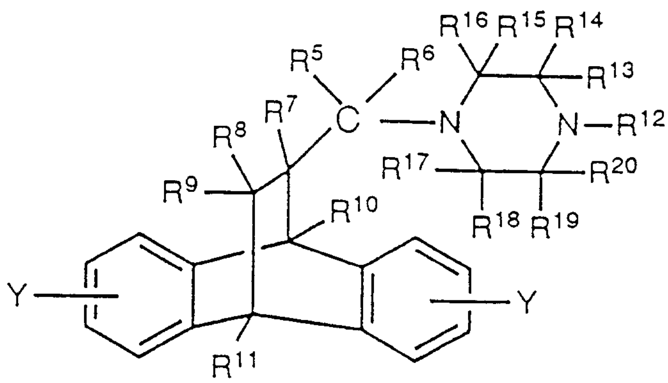

wherein each of $R^5$ and $R^6$ is independently selected from hydrido, $C_1$-$C_5$-alkyl, benzyl and phenyl; wherein each of $R^7$ through $R^{11}$ is independently selected from hydrido, $C_1$-$C_5$-alkyl, hydroxy, benzyl, phenyl, $C_1$-$C_5$-alkoxy, phenoxy, benzyloxy, halo and halo-$C_1$-$C_5$-alkyl; wherein $R^{12}$ may be selected from hydrido, $C_1$-$C_5$-alkyl, cycloalkyl of five or six carbon atoms, cycloalkylalkyl of six or seven carbon atoms, phenyl, benzyl, hydroxy-$C_1$-$C_5$-alkyl, and heteroaryl selected from saturated or fully unsaturated heterocyclic rings containing five to seven ring members of which one or two ring members are nitrogen atom; wherein each Y is independently one or more groups selected from hydrido, hydroxy, $C_1$-$C_5$-alkyl, benzyl, phenyl, $C_1$-$C_5$-alkoxy, phenoxy, halo-$C_1$-$C_5$-alkyl, halo, and loweralkanoyl; and wherein each of $R^{13}$ through $R^{20}$ is independently selected from hydrido, $C_1$-$C_5$-alkyl, benzyl, phenyl and halo; wherein $R^{12}$ together with one of $R^{13}$, $R^{14}$, $R^{19}$ and $R^{20}$ may form a fused heterocyclic ring containing five or six ring members; or a pharmaceutically-acceptable salt thereof;
said method comprising reacting a compound of the formula

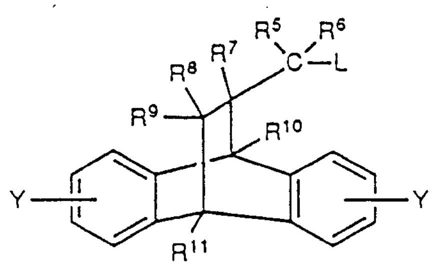

wherein each of $R^5$ through $R^{11}$ and Y is defined above; and wherein L is selected from halo, hydroxy, para-toluenesulfonyloxy, methylsulfonyloxy and para-bromotoluenesulfonyloxy; with an amine of the formula

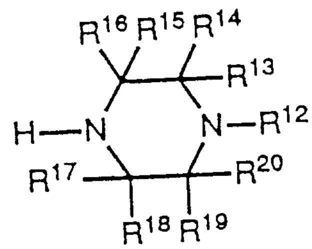

wherein each of $R^{12}$ through $R^{20}$ is as defined above.

**12.** The process of Claim 11 wherein each of $R^5$ and $R^6$ is independently hydrido or methyl; wherein each of $R^7$ through $R^{11}$ is independently selected from hydrido, methyl, ethyl, hydroxy, methoxy, halo and trihalomethyl; wherein $R^{12}$ is selected from hydrido, methyl, ethyl, hydroxyethyl, benzyl, pyridyl and pyrimidyl; wherein each of $R^{13}$ through $R^{20}$ is independently selected from hydrido, methyl, ethyl and trihalomethyl; wherein $R^{12}$ together with one of $R^{13}$, $R^{14}$, $R^{19}$ or $R^{20}$ may form a fused heterocyclic ring containing five or six ring members; wherein each Y isindependently one or more groups selected from hydrido, methyl, ethyl, hydroxy, methoxy, trihalomethyl and halo; and wherein L is selected from halo and hydroxy.

**13.** The process of Claim 12 wherein L is halo.

**14.** The process of Claim 13 wherein L is chloro or bromo.

**15.** The process of Claim 12 wherein L is hydroxy.

**16.** The process of Claim 11 wherein a compound of the formula

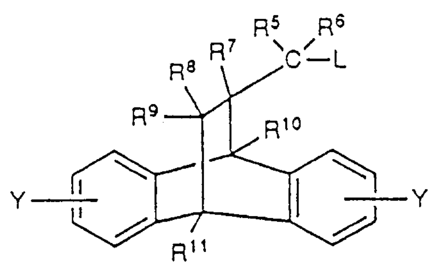

is prepared by mixing an alcohol of the formula

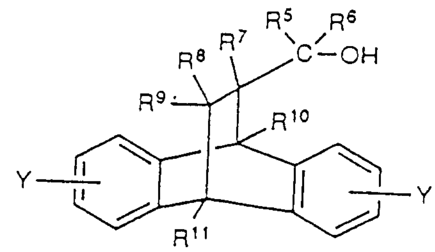

wherein each of $R^5$ through $R^{11}$ and L is as defined above, with a reagent selected from thionyl chloride, phosphorous oxychloride, triphenylphosphine dibromide, methanesulfonyl chloride and p-toluenesulfonyl chloride.

**17.** The process of Claim 11 wherein said product compound is selected from 4-[(9,10-dihydro-9,10-ethanoanthracenyl)methyl]-1-methylpiperazine; 4-[(9,10-dihydro-9,10-ethanoanthracenyl)methyl]-1-(2-hydroxyethyl)-piperazine; 4-[(9,10-dihydro-9,10-ethanoanthracenyl)methyl]-1-(2-pyrimidyl)piperzaine; 4-[(9,10-dihydro-9,10-ethanoanthracenyl)methyl]-1,4-diazabicyclo[4.3.0]nonane; and 4-[(9,10-dihydro-9,10-ethanoanthracenyl)methyl]-1-benzylpiperazine.

**18.** The process of Claim 17 wherein said product compound is selected from 4-[(9,10-dihydro-9,10-ethanoanthracenyl)methyl]-1-methylpiperaZine; 4-[(9,10-dihydro-9,10-ethanoanthracenyl)methyl]-1-(2-hydroxyethyl)-piperazine; and 4-[(9,10-dihydro-9,10-ethanoanthracenyl)methyl]-1-benzylpiperazlne.

## Claims for the following Contracting State : ES

**1.** A process for preparing a compound of the formula

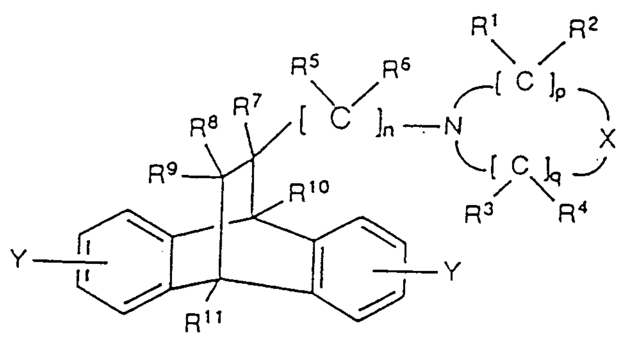

wherein each of $R^1$ through $R^6$ is independently selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phenyl-$C_1$-$C_{20}$-alkyl, phenyl, $C_1$-$C_{10}$-alkoxyalkyl, $C_1$-$C_{20}$-hydroxyalkyl and halo; wherein $R^1$ and $R^2$ may be taken together to form oxo; wherein $R^3$ and $R^4$ may be taken together to form oxo; wherein $R^5$ and $R^6$ may be taken together to form oxo; wherein each of $R^7$ through $R^{11}$ is independently selected from hydrido, $C_1$-$C_{20}$-alkyl, hydroxy, $C_3$-$C_{10}$-cycloalkyl, $C_3$-$C_{10}$-cycloalkyl-$C_1$-$C_{20}$-alkyl, phenyl-$C_1$-$C_{20}$-alkyl, phenyl, $C_1$-$C_{10}$-alkoxy, $C_1$-$C_{10}$-alkoxyalkyl, phenyloxy, phenyl-$C_1$-$C_{10}$-alkoxy, $C_1$-$C_{20}$-hydroxyalkyl, halo and $C_1$-$C_{20}$-haloalkyl; wherein $R^8$ and $R^9$ may be taken together to form oxo; wherein n is a number selected from zero to five, inclusive; wherein each of p and q is a number selected from one to four, inclusive; wherein X is selected from O, S, $>$N-$R^{12}$, $>$SO and $>SO_2$; wherein $R^{12}$ may be selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phenyl, phenyl-$C_1$-$C_{20}$-alkyl, an aromatic ring system containing one or two heteroatoms selected from oxygen, nitrogen, sulfur in a ring system having five or six ring members, $C_1$-$C_{10}$-alkoxyalkyl, $C_1$-$C_{20}$-hydroxyalkyl, alkanoyl, aralkanoyl, aroyl, amino-$C_1$-$C_{20}$-alkyl, mono-$C_1$-$C_{20}$-alkylamino-$C_1$-$C_{20}$-alkyl and di-$C_1$-$C_{20}$-alkylamino-$C_1$-$C_{20}$-alkyl; wherein $R^{12}$ together with one of $R^1$ through $R^4$ may form a fused heterocyclic ring containing five to eight ring members; wherein each Y is independently one or more groups selected from hydrido, hydroxy, $C_1$-$C_{20}$-alkyl, $C_3$-$C_{10}$-cycloalkyl, $C_3$-$C_{10}$-cycloalkyl-$C_1$-$C_{20}$-alkyl, phenyl-$C_1$-$C_{20}$-alkyl, phenyl, $C_1$-$C_{10}$-alkoxy, phenyl-$C_1$-$C_{10}$-alkoxy,

phenyloxy, $C_1$-$C_{10}$-alkoxyalkyl, $C_1$-$C_{20}$-haloalkyl, $C_1$-$C_{20}$-hydroxyalkyl, halo, cyano, amino, mono-$C_1$-$C_{20}$-alkylamino, di-$C_1$-$C_{20}$-alkylamino, nitro, carboxy, $C_3$-$C_{10}$-carboxyalkyl and alkanoyl; or a pharmaceutically-acceptable salt thereof; with the proviso that when X is $>NR^{12}$, and each of p and q is two and each of $R^1$ through $R^4$ is hydrido and each of $R^7$ through $R^{11}$ is hydrido, and $R^{12}$ is selected from hydrido, methyl, hydroxyethyl and acetoxy, then $R^5$ together with $R^6$ cannot be oxo and at least one of $R^5$ and $R^6$ must be other than hydrido; and with the further proviso that when X is oxygen atom and each of p and q is two, then each of the following selections cannot be made:

- $R^5$ and $R^6$ together being oxo when each of $R^1$ through $R^4$, $R^7$ through $R^{11}$ and Y is hydrido;
- each of $R^1$ through $R^{11}$ and Y being hydrido;
- $R^8$ and $R^9$ together being oxo when each of $R^1$ through $R^4$, $R^7$ through $R^{11}$ and Y is hydrido;
- $R^8$ and $R^9$ together being oxo with each of $R^{10}$ and $R^{11}$ being methyl when each of $R^1$ through $R^4$, $R^7$ through $R^9$ and Y is hydrido,

characterized in that a compound of the formula 3

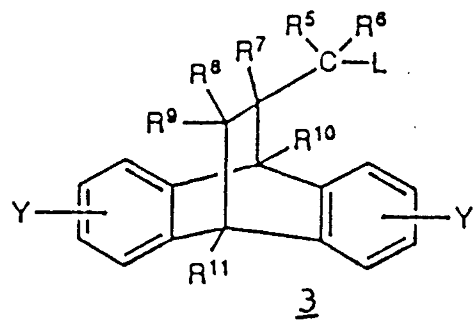

3

wherein each of $R^5$ through $R^{11}$ and Y is as defined above; and wherein L is selected from halo, hydroxy, para-toluenesulfonyloxy, methylsulfonyloxy and para-bromotoluenesulfonyloxy,
is reacted with a compound of the formula 5

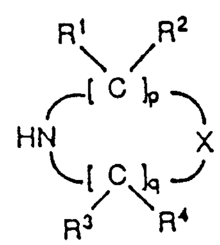

5

wherein p, q, X and $R^1$ through $R^4$ are as defined above.

**2.** Process according to Claim 1 wherein each of $R^1$ through $R^6$ of the compound prepared is independently selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxyalkyl, $C_1$-$C_{20}$-hydroxyalkyl and halo; wherein $R^5$ and $R^6$ may be taken together to form oxo; wherein each of $R^7$ through $R^{11}$ is independently selected from hydrido, $C_1$-$C_{20}$-alkyl, $C_1$-$C_{10}$-hydroxy, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxy, $C_1$-$C_{10}$-alkoxyalkyl, phenoxy, phenyl-$C_1$-$C_{10}$-alkoxy, $C_1$-$C_{20}$-hydroxyalkyl, halo and $C_1$-$C_{20}$-haloalkyl; wherein $R^8$ and $R^9$ may be taken together to form oxo; wherein n is a number selected from zero to five, inclusive; wherein each of p and q is a member selected from one to four, inclusive; wherein X is selected from O, S and $>N$-$R^{12}$; wherein $R^{12}$ may be selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phenyl, phen-$C_1$-$C_{20}$-alkyl, an aromatic ring system containing one or two hetero atoms selected from oxygen, nitrogen, sulfur in a ring system having five or six ring members, $C_1$-$C_{10}$-alkoxyalkyl, $C_1$-$C_{20}$-hydroxyalkyl, alkanoyl, phenalkanoyl, aroyl, amino-$C_1$-$C_{20}$-alkyl, mono-$C_1$-$C_{20}$-alkylamino-$C_1$-$C_{20}$-alkyl and di-$C_1$-$C_{20}$-alkylamino-$C_1$-$C_{20}$-alkyl; wherein $R^{12}$ together with one of $R^1$ through $R^4$ form a fused heterocyclic ring containing five to eight ring

members; wherein each Y is independently one or more groups selected from hydrido, hydroxy, $C_1$-$C_{20}$-alkyl, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxy, phen-$C_1$-$C_{10}$-alkoxy, phenoxy, $C_1$-$C_{10}$-alkoxyalkyl, $C_1$-$C_{20}$-haloalkyl, $C_1$-$C_{20}$-hydroxyalkyl, halo, cyano, amino, mono-$C_1$-$C_{20}$-alkylamino, di-$C_1$-$C_{20}$-alkylamino, carboxy, carboxy-$C_1$-$C_{20}$-alkyl and alkanoyl; or a pharmaceutically-acceptable salt thereof.

**3.** Process according to Claim 2 wherein each of $R^1$ through $R^4$ of the compound prepared is independently sekected from hydrido, $C_1$-$C_5$-alkyl, cycloalkyl of three to eight carbon atoms, cycloalkyl of four to eight carbon atoms, phenyl-$C_1$-$C_5$-alkyl, phenyl, $C_1$-$C_5$-alkoxy-$C_1$-$C_5$-alkyl, hydroxy-$C_1$-$C_5$-alkyl and halo; wherein each of $R^5$ and $R^6$ is independently selected from hydrido, $C_1$-$C_5$-alkyl, phenyl-$C_1$-$C_5$-alkyl, phenyl and $C_1$-$C_5$-alkoxy-$C_1$-$C_5$-alkyl; wherein $R^5$ and $R^6$ may be taken together to form oxo; wherein each of $R^7$ through $R^{11}$ is independently selected from hydrido, $C_1$-$C_5$-alkyl, hydroxy, cycloalkyl of three to eight carbon atoms, cycloalkylalkyl of four to eight carbon atoms, phenyl-$C_1$-$C_5$-alkyl, phenyl, $C_1$-$C_5$-alkoxy, $C_1$-$C_5$-alkoxy-$C_1$-$C_5$-alkyl, phenoxy, phenyl-$C_1$-$C_{10}$-alkoxy, hydroxy-$C_1$-$C_5$-alkyl, halo and halo-$C_1$-$C_5$-alkyl; wherein $R^8$ and $R^9$ may be taken together to form oxo; wherein n is a number selected from zero to five, inclusive; wherein each of p and q is two or three; wherein X is selected from O, S, $>$N-$R^{12}$; wherein $R^{12}$ may be selected from hydrido, $C_1$-$C_5$-alkyl, cycloalkyl of three to eight carbon atoms, cycloalkylalkyl of four to eight carbon atoms, phenyl, phenyl-$C_1$-$C_5$-alkyl, $C_1$-$C_5$-alkoxy-$C_1$-$C_5$-alkyl, hydroxy-$C_1$-$C_5$-alkyl, lower-alkanoyl and heteroaryl selected from saturated, partially unsaturated and fully unsaturated heterocyclic rings containing five to seven ring members of which one or two ring members are selected from oxygen atom and nitrogen atom; wherein $R^{12}$ together with one of $R^1$ through $R^4$ may form a fused heterocyclic ring containing five or six ring members; wherein each Y is independently one or more groups selected from hydrido, hydroxy, $C_1$-$C_5$-alkyl, cycloalkyl of three to eight carbon atoms, cycloalkylalkyl of four to eight carbon atoms, phenyl-$C_1$-$C_5$-alkyl, phenyl, $C_1$-$C_5$-alkoxy, phenoxy, $C_1$-$C_5$-alkoxy-$C_1$-$C_5$-alkyl, halo-$C_1$-$C_5$-alkyl, hydroxy-$C_1$-$C_5$-alkyl, halo, carboxy, carboxy-$C_1$-$C_5$-alkyl and loweralkanoyl; or a pharmaceutically-acceptable salt thereof.

**4.** Process according to Claim 3 of the formula

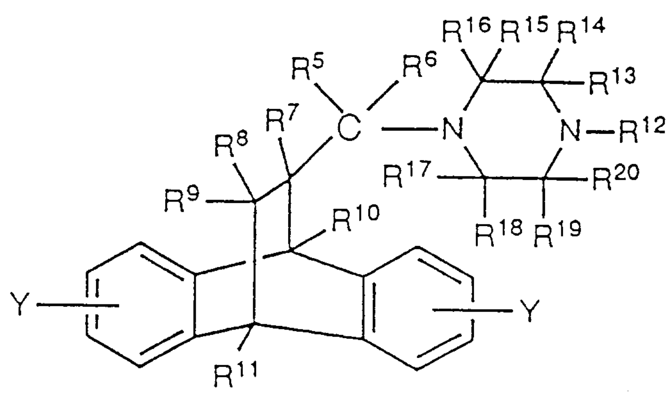

wherein each of $R^5$ and $R^6$ of the compound prepared is independently selected from hydrido, $C_1$-$C_5$-alkyl, benzyl and phenyl; wherein each of $R^7$ through $R^{11}$ is independently selected from hydrido, $C_1$-$C_5$-alkyl, hydroxy, benzyl, phenyl, $C_1$-$C_5$-alkoxy, phenoxy, benzyloxy, halo and halo-$C_1$-$C_5$-alkyl; wherein $R^{12}$ may be selected from hydrido, $C_1$-$C_5$-alkyl, cycloalkyl of five or six carbon atoms, cycloalkylalkyl of six or seven carbon atoms, phenyl, benzyl, hydroxy-$C_1$-$C_5$-alkyl, and heteroaryl selected from saturated or fully unsaturated heterocyclic rings containing five to seven ring members of which one or two ring members are nitrogen atom; wherein each Y is independently one or more groups selected from hydrido, hydroxy, $C_1$-$C_5$-alkyl, benzyl, phenyl, $C_1$-$C_5$-alkoxy, phenoxy, halo-$C_1$-$C_5$-alkyl, halo, and loweralkanoyl; and wherein each of $R^{13}$ through $R^{20}$ is independently selected from hydrido, $C_1$-$C_5$-alkyl, benzyl, phenyl and halo; wherein $R^{12}$ together with one of $R^{13}$, $R^{14}$, $R^{19}$ or $R^{20}$ may form a fused heterocyclic ring containing five or six ring members; or a pharmaceutically-acceptable salt thereof.

**5.** Process according to Claim 4 wherein each of $R^5$ and $R^6$ of the compound prepared is independently hydrido or methyl; wherein each of $R^7$ through $R^{11}$ is independently selected from hydrido, methyl, ethyl, hydroxy, methoxy, halo and trihalomethyl; wherein $R^{12}$ is selected from hydrido, methyl, ethyl, hydroxyethyl, benzyl, pyridyl and pyrimidyl; wherein each of $R^{13}$ through $R^{20}$ is independently selected from hydrido, methyl and ethyl ; wherein $R^{12}$ together with one of $R^{13}$, $R^{14}$, $R^{19}$ or $R^{20}$ may form a fused heterocyclic ring containing five or six ring members; and wherein each Y is independently one or more groups selected from hydrido, methyl, ethyl, hydroxy, methoxy, trihalomethyl and halo.

**6.** Process according to Claim 5 wherein the compound prepared is selected from 4-[(9,10-dihydro-9,10-ethanoanthracenyl)methyl]-1-(2-pyrimidyl)piperazine; 4-[(9,10-dihydro-9,10-ethanoanthracenyl)methyl]-1,4-diazabicyclo[4.3.0]nonane; and 4-[(9,10-dihydro-9,10-ethanoanthracenyl)methyl]-1-benzylpiperazine.

**7.** A process for preparing a pharmaceutical composition characterized in that a therapeutically-effective amount of an active compound is mixed with a pharmaceutically-acceptable carrier or diluent, said active compound selected from a family of compounds according to any of Claims 1, 2, 3, 4, 5 or 6.

**8.** Use of a therapeutically-effective amount of a compound as prepared according to any of Claims 1, 2, 4, 5 or 6 for preparing a medicament for treating a central nervous system disorder.

**9.** Use according to Claim 8 wherein said central nervous system disorder is selected from a psychotic disorder, a convulsive disorder and a dystonic disorder.

**10.** Use according to Claim 9 wherein said central nervous system disorder is a psychotic disorder.

**11.** A process for preparing a product compound of the formula

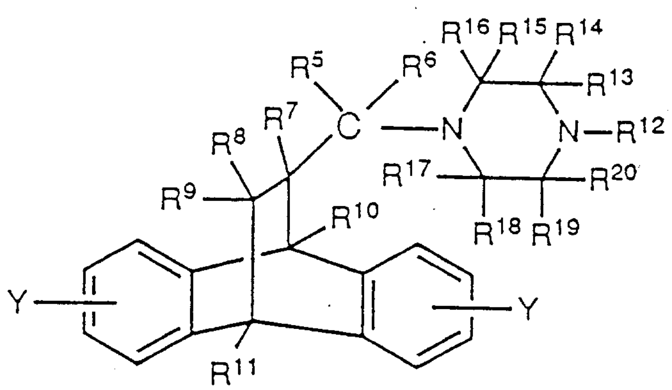

wherein each of $R^5$ and $R^6$ is independently selected from hydrido, $C_1$-$C_5$-alkyl, benzyl and phenyl; wherein each of $R^7$ through $R^{11}$ is independently selected from hydrido, $C_1$-$C_5$-alkyl, hydroxy, benzyl, phenyl, $C_1$-$C_5$-alkoxy, phenoxy, benzyloxy, halo and halo-$C_1$-$C_5$-alkyl; wherein $R^{12}$ may be selected from hydrido, $C_1$-$C_5$-alkyl, cycloalkyl of five or six carbon atoms, cycloalkylalkyl of six or seven carbon atoms, phenyl, benzyl, hydroxy-$C_1$-$C_5$-alkyl, and heteroaryl selected from saturated or fully unsaturated heterocyclic rings containing five to seven ring members of which one or two ring members are nitrogen atom; wherein each Y is independently one or more groups selected from hydrido, hydroxy, $C_1$-$C_5$-alkyl, benzyl, phenyl, $C_1$-$C_5$-alkoxy, phenoxy, halo-$C_1$-$C_5$-alkyl, halo, and loweralkanoyl; and wherein each of $R^{13}$ through $R^{20}$ is independently selected from hydrido, $C_1$-$C_5$-alkyl, benzyl, phenyl and halo; wherein $R^{12}$ together with one of $R^{13}$, $R^{14}$, $R^{19}$ and $R^{20}$ may form a fused heterocyclic ring containing five or six ring members; or a pharmaceutically-acceptable salt thereof;
said method comprising reacting a compound of the formula

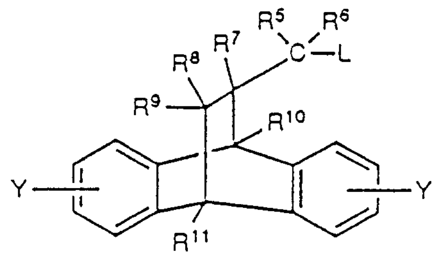

wherein each of $R^5$ through $R^{11}$ and Y is defined above; and wherein L is selected from halo, hydroxy, para-toluenesulfonyloxy, methylsulfonyloxy and para-bromotoluenesulfonyloxy; with an amine of the formula

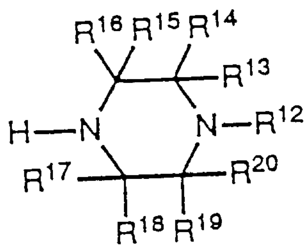

wherein each of $R^{12}$ through $R^{20}$ is as defined above.

**12.** The process of Claim 11 wherein each of $R^5$ and $R^6$ is independently hydrido or methyl; wherein each of $R^7$ through $R^{11}$ is independently selected from hydrido, methyl, ethyl, hydroxy, methoxy, halo and trihalomethyl; wherein $R^{12}$ is selected from hydrido, methyl, ethyl, hydroxyethyl, benzyl, pyridyl and pyrimidyl; wherein each of $R^{13}$ through $R^{20}$ is independently selected from hydrido, methyl, ethyl and trihalomethyl; wherein $R^{12}$ together with one of $R^{13}$, $R^{14}$, $R^{19}$ or $R^{20}$ may form a fused heterocyclic ring containing five or six ring members; wherein each Y isindependently one or more groups selected from hydrido, methyl, ethyl, hydroxy, methoxy, trihalomethyl and halo; and wherein L is selected from halo and hydroxy.

**13.** The process of Claim 12 wherein L is halo.

**14.** The process of Claim 13 wherein L is chloro or bromo.

**15.** The process of Claim 12 wherein L is hydroxy.

**16.** The process of Claim 11 wherein a compound of the formula

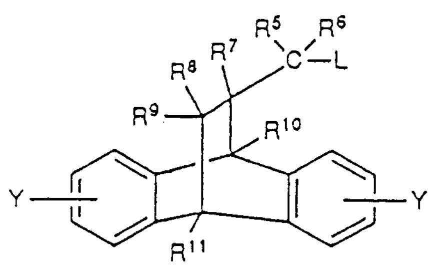

is prepared by mixing an alcohol of the formula

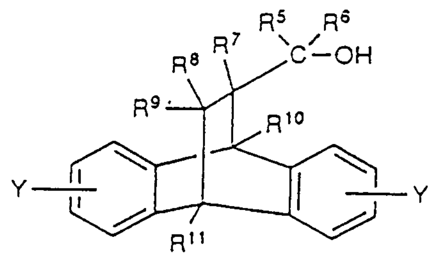

wherein each of $R^5$ through $R^{11}$ and L is as defined above, with a reagent selected from thionyl chloride, phosphorous oxychloride, triphenylphosphine dibromide, methanesulfonyl chloride and p-toluenesulfonyl chloride.

**17.** The process of Claim 11 wherein said product compound is selected from 4-[(9,10-dihydro-9,10-ethanoanthracenyl)methyl]-1-methylpiperazine; 4-[(9,10-dihydro-9,10-ethanoanthracenyl)methyl]-1-(2-hydroxyethyl)-piperazine; 4-[(9,10-dihydro-9,10-ethanoanthracenyl)methyl]-1-(2-pyrimidyl)piperzaine; 4-[(9,10-dihydro-9,10-ethanoanthracenyl)methyl]-1,4-diazabicyclo[4.3.0]-nonane; and 4-[(9,10-dihydro-9,10-ethanoanthracenyl)methyl]-1-benzylpiperazine.

**18.** The process of Claim 17 wherein said product compound is selected from 4-[(9,10-dihydro-9,10-ethanoanthracenyl)methyl]-1-methylpiperazine; 4-[(9,10-dihydro-9,10-ethanoanthracenyl)methyl]-1-(2-hydroxyethyl)-piperazine; and 4-[(9,10-dihydro-9,10-ethanoanthracenyl)methyl]-1-benzylpiperazine.

**Claims for the following Contracting State : GR**

**1.** A compound of the formula

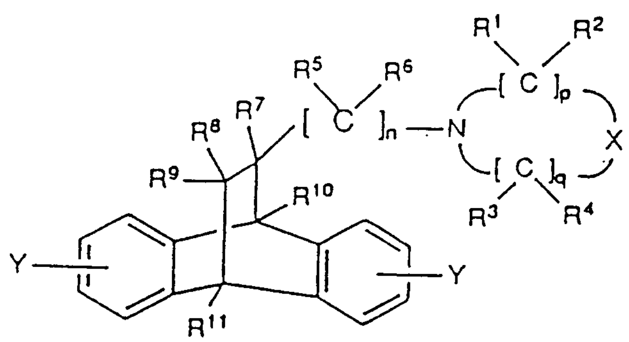

wherein each of $R^1$ through $R^6$ is independently selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phenyl-$C_1$-$C_{20}$-alkyl, phenyl, $C_1$-$C_{10}$-alkoxyalkyl, $C_1$-$C_{20}$-hydroxyalkyl and halo; wherein $R^1$ and $R^2$ may be taken together to form oxo; wherein $R^3$ and $R^4$ may be taken together to form oxo; wherein $R^5$ and $R^6$ may be taken together to form oxo; wherein each of $R^7$ through $R^{11}$ is independently selected from hydrido, $C_1$-$C_{20}$-alkyl, hydroxy, $C_3$-$C_{10}$-cycloalkyl, $C_3$-$C_{10}$-cycloalkyl-$C_1$-$C_{20}$-alkyl, phenyl-$C_1$-$C_{20}$-alkyl, phenyl, $C_1$-$C_{10}$-alkoxy, $C_1$-$C_{10}$-alkoxyalkyl, phenyloxy, phenyl-$C_1$-$C_{10}$-alkoxy, $C_1$-$C_{20}$-hydroxyalkyl, halo and $C_1$-$C_{20}$-haloalkyl; wherein $R^8$ and $R^9$ may be taken together to form oxo; wherein n is a number selected from zero to five, inclusive; wherein each of p and q is a number selected from one to four, inclusive; wherein X is selected from O, S, $>$N-$R^{12}$, $>$SO and $>SO_2$; wherein $R^{12}$ may be selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phenyl, phenyl-$C_1$-$C_{20}$-alkyl, an aromatic ring system containing one or two heteroatoms selected from oxygen, nitrogen, sulfur in a ring system having five or six ring members, $C_1$-$C_{10}$-alkoxyalkyl, $C_1$-$C_{20}$-hydroxyalkyl, alkanoyl, aralkanoyl, aroyl, amino-$C_1$-$C_{20}$-alkyl, mono-$C_1$-$C_{20}$-alkylamino-$C_1$-$C_{20}$-alkyl and di-$C_1$-$C_{20}$-alkylamino-$C_1$-$C_{20}$-alkyl; wherein $R^{12}$ together with one of $R^1$ through $R^4$ may form a fused heterocyclic ring containing five to eight ring members; wherein each Y is independently one or more groups selected from hydrido, hydroxy, $C_1$-$C_{20}$-alkyl, $C_3$-$C_{10}$-cycloalkyl, $C_3$-

$C_{10}$-cycloalkyl-$C_1$-$C_{20}$-alkyl, phenyl-$C_1$-$C_{20}$-alkyl, phenyl, $C_1$-$C_{10}$-alkoxy, phenyl-$C_1$-$C_{10}$-alkoxy, phenyloxy, $C_1$-$C_{10}$-alkoxyalkyl, $C_1$-$C_{20}$-haloalkyl, $C_1$-$C_{20}$-hydroxyalkyl, halo, cyano, amino, mono-$C_1$-$C_{20}$-alkylamino, di-$C_1$-$C_{20}$-alkylamino, nitro, carboxy, $C_3$-$C_{10}$-carboxyalkyl and alkanoyl; or a pharmaceutically-acceptable salt thereof; with the proviso that when X is $>NR^{12}$, and each of p and q is two and each of $R^1$ through $R^4$ is hydrido and each of $R^7$ through $R^{11}$ is hydrido, and $R^{12}$ is selected from hydrido, methyl, hydroxyethyl and acetoxy, then $R^5$ together with $R^6$ cannot be oxo and at least one of $R^5$ and $R^6$ must be other than hydrido; and with the further proviso that when X is oxygen atom and each of p and q is two, then each of the following selections cannot be made:

- $R^5$ and $R^6$ together being oxo when each of $R^1$ through $R^4$, $R^7$ through $R^{11}$ and Y is hydrido;
- each of $R^1$ through $R^{11}$ and Y being hydrido;
- $R^8$ and $R^9$ together being oxo when each of $R^1$ through $R^4$, $R^7$ through $R^{11}$ and Y is hydrido;
- $R^8$ and $R^9$ together being oxo with each of $R^{10}$ and $R^{11}$ being methyl when each of $R^1$ through $R^4$, $R^7$ through $R^9$ and Y is hydrido.

**2.** Compound of Claim 1 wherein each of $R^1$ through $R^6$ is independently selected from hydrido; $C_1$-$C_{20}$-alkyl, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxyalkyl, $C_1$-$C_{20}$-hydroxyalkyl and halo; wherein $R^5$ and $R^6$ may be taken together to form oxo; wherein each of $R^7$ through $R^{11}$ is independently selected from hydrido, $C_1$-$C_{20}$-alkyl, $C_1$-$C_{10}$-hydroxy, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxy, $C_1$-$C_{10}$-alkoxyalkyl, phenoxy, phenyl-$C_1$-$C_{10}$-alkoxy, $C_1$-$C_{20}$-hydroxyalkyl, halo and $C_1$-$C_{20}$-haloalkyl; wherein $R^8$ and $R^9$ may be taken together to form oxo; wherein n is a number selected from zero to five, inclusive; wherein each of p and q is a member selected from one to four, inclusive; wherein X is selected from O, S and $>N-R^{12}$; wherein $R^{12}$ may be selected from hydrido, $C_1$-$C_{20}$-alkyl, $C_3$-$C_{10}$-cycloalkyl, $C_3$-$C_{10}$-cycloalkyl-$C_1$-$C_{20}$-alkyl, phenyl, phen-$C_1$-$C_{20}$-alkyl, an aromatic ring system containing one or two hetero atoms selected from oxygen, nitrogen, sulfur in a ring system having five or six ring members, $C_1$-$C_{10}$-alkoxyalkyl, $C_1$-$C_{20}$-hydroxyalkyl, alkanoyl, phenalkanoyl, aroyl, amino-$C_1$-$C_{20}$-alkyl, mono-$C_1$-$C_{20}$-alkylamino-$C_1$-$C_{20}$-alkyl and di-$C_1$-$C_{20}$-alkylamino-$C_1$-$C_{20}$-alkyl; wherein $R^{12}$ together with one of $R^1$ through $R^4$ form a fused heterocyclic ring containing five to eight ring members; wherein each Y is independently one or more groups selected from hydrido, hydroxy, $C_1$-$C_{20}$-alkyl, $C_3$-$C_{10}$-cycloalkyl, $C_3$-$C_{10}$-cycloalkyl-$C_1$-$C_{20}$-alkyl, phen-$C_1$-$C_{20}$-alkyl, phenyl, $C_1$-$C_{10}$-alkoxy, phen-$C_1$-$C_{10}$-alkoxy, phenoxy, $C_1$-$C_{10}$-alkoxyalkyl, $C_1$-$C_{20}$-haloalkyl, $C_1$-$C_{20}$-hydroxyalkyl, halo, cyano, amino, mono-$C_1$-$C_{20}$-alkylamino, di-$C_1$-$C_{20}$-alkylamino, carboxy, carboxy-$C_1$-$C_{20}$-alkyl and alkanoyl; or a pharmaceutically-acceptable salt thereof.

**3.** Compound of Claim 2 wherein each of $R^1$ through $R^4$ is independently selected from hydrido, $C_1$-$C_5$-alkyl, cycloalkyl of three to eight carbon atoms, cycloalkyl of four to eight carbon atoms, phenyl-$C_1$-$C_5$-alkyl, phenyl, $C_1$-$C_5$-alkoxy-$C_1$-$C_5$-alkyl, hydroxy-$C_1$-$C_5$-alkyl and halo; wherein each of $R^5$ and $R^6$ is independently selected from hydrido, $C_1$-$C_5$-alkyl, phenyl-$C_1$-$C_5$-alkyl, phenyl and $C_1$-$C_5$-alkoxy-$C_1$-$C_5$-alkyl; wherein $R^5$ and $R^6$ may be taken together to form oxo; wherein each of $R^7$ through $R^{11}$ is independently selected from hydrido, $C_1$-$C_5$-alkyl, hydroxy, cycloalkyl of three to eight carbon atoms, cycloalkylalkyl of four to eight carbon atoms, phenyl-$C_1$-$C_5$-alkyl, phenyl, $C_1$-$C_5$-alkoxy, $C_1$-$C_5$-alkoxy-$C_1$-$C_5$-alkyl, phenoxy, phenyl-$C_1$-$C_{10}$-alkoxy, hydroxy-$C_1$-$C_5$-alkyl, halo and halo-$C_1$-$C_5$-alkyl; wherein $R^8$ and $R^9$ may be taken together to form oxo; wherein n is a number selected from zero to five, inclusive; wherein each of p and q is two or three; wherein X is selected from O, S, $>N-R^{12}$; wherein $R^{12}$ may be selected from hydrido, $C_1$-$C_5$-alkyl, cycloalkyl of three to eight carbon atoms, cycloalkylalkyl of four to eight carbon atoms, phenyl, phenyl-$C_1$-$C_5$-alkyl, $C_1$-$C_5$-alkoxy-$C_1$-$C_5$-alkyl, hydroxy-$C_1$-$C_5$-alkyl, lower-alkanoyl and heteroaryl selected from saturated, partially unsaturated and fully unsaturated heterocyclic rings containing five to seven ring members of which one or two ring members are selected from oxygen atom and nitrogen atom; wherein $R^{12}$ together with one of $R^1$ through $R^4$ may form a fused heterocyclic ring containing five or six ring members; wherein each Y is independently one or more groups selected from hydrido, hydroxy, $C_1$-$C_5$-alkyl, cycloalkyl of three to eight carbon atoms, cycloalkylalkyl of four to eight carbon atoms, phenyl-$C_1$-$C_5$-alkyl, phenyl, $C_1$-$C_5$-alkoxy, phenoxy, $C_1$-$C_5$-alkoxy-$C_1$-$C_5$-alkyl, halo-$C_1$-$C_5$-alkyl, hydroxy-$C_1$-$C_5$-alkyl, halo, carboxy, carboxy-$C_1$-$C_5$-alkyl and loweralkanoyl; or a pharmaceutically-acceptable salt thereof.

**4.** Compound of Claim 3 of the formula

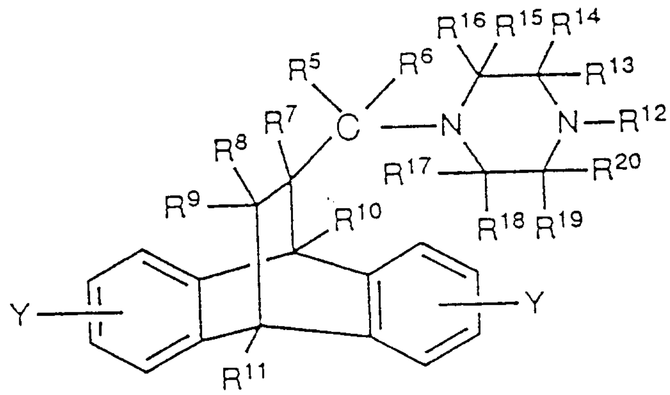

wherein each of $R^5$ and $R^6$ is independently selected from hydrido, $C_1$-$C_5$-alkyl, benzyl and phenyl; wherein each of $R^7$ through $R^{11}$ is independently selected from hydrido, $C_1$-$C_5$-alkyl, hydroxy, benzyl, phenyl, $C_1$-$C_5$-alkoxy, phenoxy, benzyloxy, halo and halo-$C_1$-$C_5$-alkyl; wherein $R^{12}$ may be selected from hydrido, $C_1$-$C_5$-alkyl, cycloalkyl of five or six carbon atoms, cycloalkylalkyl of six or seven carbon atoms, phenyl, benzyl, hydroxy-$C_1$-$C_5$-alkyl, and heteroaryl selected from saturated or fully unsaturated heterocyclic rings containing five to seven ring members of which one or two ring members are nitrogen atom; wherein each Y is independently one or more groups selected from hydrido, hydroxy, $C_1$-$C_5$-alkyl, benzyl, phenyl, $C_1$-$C_5$-alkoxy, phenoxy, halo-$C_1$-$C_5$-alkyl, halo, and loweralkanoyl; and wherein each of $R^{13}$ through $R^{20}$ is independently selected from hydrido, $C_1$-$C_5$-alkyl, benzyl, phenyl and halo; wherein $R^{12}$ together with one of $R^{13}$, $R^{14}$, $R^{19}$ or $R^{20}$ may form a fused heterocyclic ring containing five or six ring members; or a pharmaceutically-acceptable salt thereof.

**5.** Compound of Claim 4 wherein each of $R^5$ and $R^6$ is independently hydrido or methyl; wherein each of $R^7$ through $R^{11}$ is independently selected from hydrido, methyl, ethyl, hydroxy, methoxy, halo and trihalomethyl; wherein $R^{12}$ is selected from hydrido, methyl, ethyl, hydroxyethyl, benzyl, pyridyl and pyrimidyl; wherein each of $R^{13}$ through $R^{20}$ is independently selected from hydrido, methyl and ethyl; wherein $R^{12}$ together with one of $R^{13}$, $R^{14}$, $R^{19}$ or $R^{20}$ may form a fused heterocyclic ring containing five or six ring members; and wherein each Y is independently one or more groups selected from hydrido, methyl, ethyl, hydroxy, methoxy, trihalomethyl and halo.

**6.** Compound or Claim 5 selected from 4-[(9,10-dihydro-9,10-ethanoanthracenyl)methyl]-1-(2-pyrimidyl)-piperazine; 4-[(9,10-dihydro-9,10-ethanoanthracenyl)methyl]-1,4-diazabicyclo[4.3.0]nonane; and 4-[(9,10-dihydro-9,10-ethanoanthracenyl)methyl]-1-benzylpiperazine.

**7.** A process for preparing a pharmaceutical composition characterized in that a therapeutically-effective amount of an active compound is mixed with a pharmaceutically-acceptable carrier or diluent, said active compound being selected from a family of compounds as prepared according to any of Claims 1, 2, 3, 4, 5 or 6.

**8.** Use of a therapeutically-effective amount of a compound according to any of Claims 1, 2, 4, 5 or 6 for preparing a medicament for treating a central nervous system disorder.

**9.** Use according to Claim 8 wherein said central nervous system disorder is selected from a psychotic disorder, a convulsive disorder and a dystonic disorder.

**10.** Use according to Claim 9 wherein said central nervous system disorder is a psychotic disorder.

**11.** A process for preparing a product compound of the formula

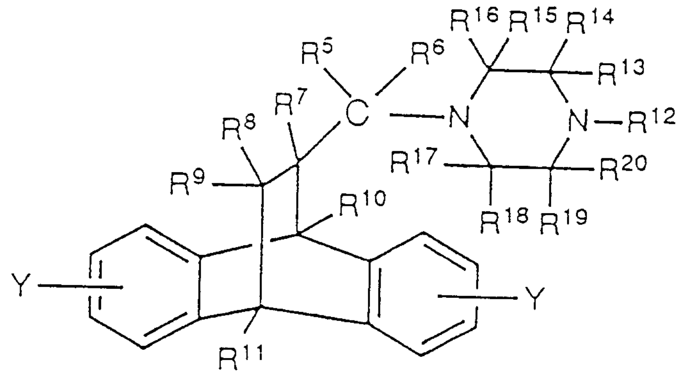

wherein each of $R^5$ and $R^6$ is independently selected from hydrido, $C_1$-$C_5$-alkyl, benzyl and phenyl; wherein each of $R^7$ through $R^{11}$ is independently selected from hydrido, $C_1$-$C_5$-alkyl, hydroxy, benzyl, phenyl, $C_1$-$C_5$-alkoxy, phenoxy, benzyloxy, halo and halo-$C_1$-$C_5$-alkyl; wherein $R^{12}$ may be selected from hydrido, $C_1$-$C_5$-alkyl, cycloalkyl of five or six carbon atoms, cycloalkylalkyl of six or seven carbon atoms, phenyl, benzyl, hydroxy-$C_1$-$C_5$-alkyl, and heteroaryl selected from saturated or fully unsaturated heterocyclic rings containing five to seven ring members of which one or two ring members are nitrogen atom; wherein each Y is independently one or more groups selected from hydrido, hydroxy, $C_1$-$C_5$-alkyl, benzyl, phenyl, $C_1$-$C_5$-alkoxy, phenoxy, halo-$C_1$-$C_5$-alkyl, halo, and loweralkanoyl; and wherein each of $R^{13}$ through $R^{20}$ is independently selected from hydrido, $C_1$-$C_5$-alkyl, benzyl, phenyl and halo; wherein $R^{12}$ together with one of $R^{13}$, $R^{14}$, $R^{19}$ and $R^{20}$ may form a fused heterocyclic ring containing five or six ring members; or a pharmaceutically-acceptable salt thereof;
said method comprising reacting a compound of the formula

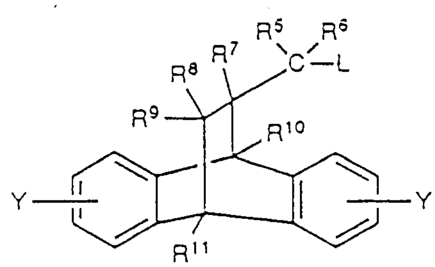

wherein each of $R^5$ through $R^{11}$ and Y is defined above; and wherein L is selected from halo, hydroxy, para-toluenesulfonyloxy, methylsulfonyloxy and para-bromotoluenesulfonyloxy; with an amine of the formula

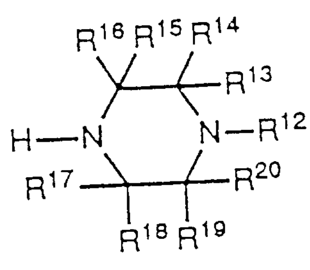

wherein each of $R^{12}$ through $R^{20}$ is as defined above.

**12.** The process of Claim 11 wherein each of $R^5$ and $R^6$ is independently hydrido or methyl; wherein each of $R^7$ through $R^{11}$ is independently selected from hydrido, methyl, ethyl, hydroxy, methoxy, halo and

trihalomethyl; wherein $R^{12}$ is selected from hydrido, methyl, ethyl, hydroxyethyl, benzyl, pyridyl and pyrimidyl; wherein each of $R^{13}$ through $R^{20}$ is independently selected from hydrido, methyl, ethyl and trihalomethyl; wherein $R^{12}$ together with one of $R^{13}$, $R^{14}$, $R^{19}$ or $R^{20}$ may form a fused heterocyclic ring containing five or six ring members; wherein each Y isindependently one or more groups selected from hydrido, methyl, ethyl, hydroxy, methoxy, trihalomethyl and halo; and wherein L is selected from halo and hydroxy.

**13.** The process of Claim 12 wherein L is halo.

**14.** The process of Claim 13 wherein L is chloro or bromo.

**15.** The process of Claim 12 wherein L is hydroxy.

**16.** The process of Claim 11 wherein a compound of the formula

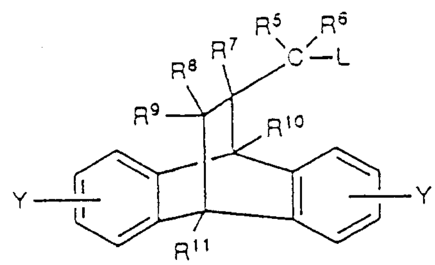

is prepared by mixing an alcohol of the formula

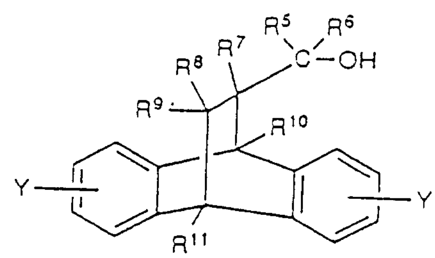

wherein each of $R^5$ through $R^{11}$ and L is as defined above, with a reagent selected from thionyl chloride, phosphorous oxychloride, triphenylphosphine dibromide, methanesulfonyl chloride and p-toluenesulfonyl chloride.

**17.** The process of Claim 11 wherein said product compound is selected from 4-[(9,10-dihydro-9,10-ethanoanthracenyl)methyl]-1-methylpiperazine; 4-[(9,10-dihydro-9,10-ethanoanthracenyl)methyl]-1-(2-hydroxyethyl)-piperazine; 4-[(9,10-dihydro-9,10-ethanoanthracenyl)methyl]-1-(2-pyrimidyl)piperzaine; 4-[(9,10-dihydro-9,10-ethanoanthracenyl)methyl]-1,4-diazabicyclo[4.3.0]nonane; and 4-[(9,10-dihydro-9,10-ethanoanthracenyl)methyl]-1-benzylpiperazine.

**18.** The process of Claim 17 wherein said product compound is selected from 4-[(9,10-dihydro-9,10-ethanoanthracenyl)methyl]-1-methylpiperazine; 4-[(9,10-dihydro-9,10-ethanoanthracenyl)methyl]-1-(2-hydroxyethyl)-piperazine; and 4-[(9,10-dihydro-9,10-ethanoanthracenyl)methyl]-1-benzylpiperazine.

**Patentansprüche**

**Patentansprüche für folgende Vertragsstaaten : AT, BE, CH, DE, DK, FR, GB, IT, LI, LU, NL, SE**

1. Eine Verbindung der Formel

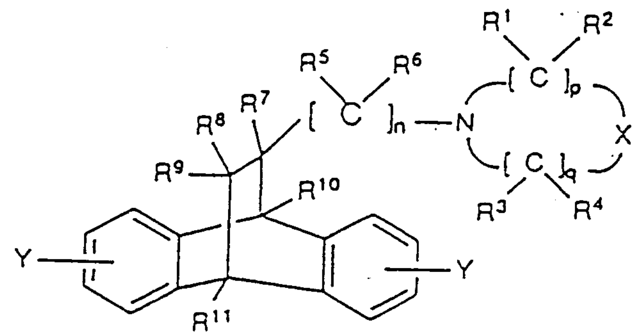

worin $R^1$ bis $R^6$ jeweils unabhängig voneinander ausgewählt sind aus Hydrido, $C_1$-$C_{20}$-Alkyl, $C_3$-$C_{10}$-Cycloalkyl, $C_3$-$C_{10}$-Cycloalkyl-$C_1$-$C_{20}$-alkyl, Phenyl-$C_1$-$C_{20}$-alkyl, Phenyl, $C_{1-10}$-Alkoxyalkyl, $C_1$-$C_{20}$-Hydroxyalkyl und Halogen; worin $R^1$ und $R^2$ zusammengenommen werden können unter Bildung von Oxo; worin $R^3$ und $R^4$ zusammenge nommen werden können unter Bildung von Oxo; worin $R^5$ und $R^6$ zusammengenommen werden können unter Bildung von Oxo; worin $R^7$ bis $R^{11}$ jeweils unabhängig voneinander ausgewählt sind aus Hydrido, $C_1$-$C_{20}$-Alkyl, Hydroxy, $C_3$-$C_{10}$-Cycloalkyl, $C_3$-$C_{10}$-Cycloalkyl-$C_1$-$C_{20}$-alkyl, Phenyl-$C_1$-$C_{20}$-alkyl, Phenyl, $C_1$-$C_{10}$-Alkoxy, $C_1$-$C_{10}$-Alkoxyalkyl, Phenyloxy, Phenyl-$C_1$-$C_{10}$-alkoxy, $C_1$-$C_{20}$-Hydroxyalkyl, Halogen und $C_1$-$C_{20}$-Haloalkyl; worin $R^8$ und $R^9$ zusammengenommen werden können unter Bildung von Oxo; worin n eine Zahl ist ausgewählt aus 0 bis 5 einschließlich; worin p und q jeweils eine Zahl ist ausgewählt aus 1 bis 4 einschließlich; worin X ausgewählt ist aus O, S, $>$N-$R^{12}$, $>$SO und $>SO_2$; worin $R^{12}$ ausgewählt sein kann aus Hydrido, $C_1$-$C_{20}$-Alkyl, $C_3$-$C_{10}$-Cycloalkyl, $C_3$-$C_{10}$-Cycloalkyl-$C_1$-$C_{20}$-alkyl, Phenyl, Phenyl-$C_1$-$C_{20}$-alkyl, einem aromatischen Ringsystem, enthaltend ein oder zwei Heteroatome ausgewählt aus Sauerstoff, Stickstoff, Schwefel in einem Ringsystem mit fünf oder sechs Ringgliedern, $C_1$-$C_{10}$-Alkoxyalkyl, $C_1$-$C_{20}$-Hydroxyalkyl, Alkanoyl, Aralkanoyl, Aroyl, Amino-$C_1$-$C_{20}$-alkyl, Mono-$C_1$-$C_{20}$-alkylamino-$C_1$-$C_{20}$-alkyl und Di-$C_1$-$C_{20}$-alkylamino-$C_1$-$C_{20}$-alkyl; worin $R^{12}$ zusammen mit einem von $R^1$ bis $R^4$ einen verschmolzenen heterocyclischen Ring bilden können, enthaltend fünf bis acht Ringglieder; worin jedes Y unabhängig voneinander eine oder mehrere Gruppen bedeutet, ausgewählt aus Hydrido, Hydroxy, $C_1$-$C_{20}$-Alkyl, $C_3$-$C_{10}$-Cycloalkyl, $C_3$-$C_{10}$-Cycloalkyl-$C_1$-$C_{20}$-alkyl, Phenyl-$C_1$-$C_{20}$-alkyl, Phenyl, $C_1$-$C_{10}$-Alkoxy, Phenyl-$C_1$-$C_{10}$-alkoxy, Phenyloxy, $C_1$-$C_{10}$-Alkoxyalkyl, $C_1$-$C_{20}$-Haloalkyl, $C_1$-$C_{20}$-Hydroxyalkyl, Halogen, Cyano, Amino, Mono-$C_1$-$C_{20}$-alkylamino, Di-$C_1$-$C_{20}$-alkylamino, Nitro, Carboxy, Carboxy-$C_1$-$C_{20}$-alkyl und Alkanoyl; oder ein pharmazeutisch verträgliches Salz davon; unter der Bedingung, daß, wenn X $>NR^{12}$ ist und p und q jeweils zwei sind und $R^1$ bis $R^4$ jeweils Hydrido sind und $R^7$ bis $R^{11}$ Hydrido sind und $R^{12}$ ausgewählt ist aus Hydrido, Methyl, Hydroxyethyl und Acetoxy, dann kann $R^5$ zusammen mit $R^6$ nicht Oxo sein und mindestens eines von $R^5$ und $R^6$ darf kein Hydrido sein; und unter der weiteren Bedingung, daß, wenn X ein Sauerstoffatom ist und p und q jeweils zwei sind, jede der nachstehenden Auswahlen nicht gemacht werden kann:

- $R^5$ und $R^6$ zusammen Oxo sind, wenn $R^1$ bis $R^4$, $R^7$ bis $R^{11}$ und Y Hydrido sind;
- jeweils $R^1$ bis $R^{11}$ und Y Hydrido sind;
- $R^8$ und $R^9$ zusammen Oxo sind, wenn $R^1$ bis $R^4$, $R^7$ bis $R^{11}$ und Y Hydrido sind;
- $R^8$ und $R^9$ zusammen Oxo sind wobei $R^{10}$ und $R^{11}$ jeweils Methyl sind, wenn $R^1$ bis $R^4$, $R^7$ bis $R^9$ und Y jeweils Hydrido sind.

2. Verbindung nach Anspruch 1, worin $R^1$ bis $R^6$ jeweils unabhängig voneinander ausgewählt sind aus Hydrido; $C_1$-$C_{20}$-Alkyl, $C_3$-$C_{10}$-Cycloalkyl, $C_3$-$C_{10}$-Cycloalkyl-$C_1$-$C_{20}$-alkyl, Phen-$C_1$-$C_{20}$-alkyl, Phenyl, $C_1$-$C_{10}$-Alkoxyalkyl, $C_1$-$C_{20}$-Hydroxyalkyl und Halogen; worin $R^5$ und $R^6$ zusammengenommen werden können unter Bildung von Oxo; worin $R^7$ bis $R^{11}$ jeweils unabhängig ausgewählt sind aus Hydrido, $C_1$-$C_{20}$-Alkyl, $C_1$-$C_{10}$-Hydroxy, $C_3$-$C_{10}$-Cycloalkyl, $C_3$-$C_{10}$-Cycloalkyl-$C_1$-$C_{20}$-alkyl, Phen-$C_1$-$C_{20}$-alkyl, Phenyl, $C_1$-$C_{10}$-Alkoxy, $C_1$-$C_{10}$-Alkoxyalkyl, Phenoxy, Phenyl-$C_1$-$C_{10}$-alkoxy, $C_1$-$C_{20}$-Hydroxyalkyl, Halogen

und $C_1$-$C_{20}$-Haloalkyl; worin $R^8$ und $R^9$ zusammengenommen werden können unter Bildung von Oxo; worin n eine Zahl ist ausgewählt aus 0 bis einschließlich 5; worin p und q jeweils eine Zahl sind ausgewählt aus 1 bis einschließlich 4; worin X ausgewählt ist aus O, S und $>$N-$R^{12}$; worin $R^{12}$ ausgewählt sein kann aus Hydrido, $C_1$-$C_{20}$-Alykl, $C_3$-$C_{10}$-Cycloalkyl, $C_3$-$C_{10}$-Cycloalkyl-$C_1$-$C_{20}$-alkyl, Phenyl, Phen-$C_1$-$C_{20}$-alkyl und einem aromatischen Ringsystem, enthaltend ein oder zwei Heteroatome ausgewählt aus Sauerstoff, Stickstoff, Schwefel in einem Ringsystem mit fünf oder sechs Ringgliedern, $C_1$-$C_{10}$-Alkoxyalkyl, $C_1$-$C_{12}$-Hydroxyalkyl, Alkanoyl, Phenalkanoyl, Aroyl, Amino-$C_1$-$C_{20}$-alkyl, Mono-$C_1$-$C_{20}$-alkylamino-$C_1$-$C_{20}$-alkyl und Di-$C_1$-$C_{20}$-alkylamino-$C_1$-$C_{20}$-alkyl; worin $R^{12}$ zusammen mit einem von $R^1$ bis $R^4$ ein verschmolzenes heterocyclisches Ringsystem bilden, enthaltend fünf bis acht Ringglieder; worin Y jeweils unabhängig voneinander eine oder mehrere Gruppen ist, ausgewählt aus Hydrido, Hydroxy, $C_1$-$C_{20}$-Alkyl, $C_3$-$C_{10}$-Cycloalkyl, $C_3$-$C_{10}$-Cycloalkyl-$C_1$-$C_{20}$-alkyl, Phen-$C_1$-$C_{20}$-alkyl, Phenyl, $C_1$-$C_{10}$-Alkoxy, Phen-$C_1$-$C_{10}$-alkoxy, Phenoxy, $C_1$-$C_{10}$-Alkoxyalkyl, $C_1$-$C_{20}$-Haloalkyl, $C_1$-$C_{20}$-Hydroxyalkyl, Halogen, Cyano, Amino, Mono-$C_1$-$C_{20}$-alkylamino,Di-$C_1$-$C_{20}$-alkylamino,Carboxy,Carobxy-$C_1$-$C_{20}$-alkylundAlkanoyl; oder ein pharmazeutisch verträgliches Salz davon.

3. Verbindung nach Anspruch 2, worin $R^1$ bis $R^4$ jeweils unabhängig voneinander ausgewählt sind aus Hydrido, $C_1$-$C_5$-Alkyl, Cycloalkyl mit 3 bis 8 Kohlenstoffatomen, Cycloalkylalkyl mit 4 bis 8 Kohlenstoffatomen, Phenyl-$C_1$-$C_5$-alkyl, Phenyl, $C_1$-$C_5$-Alkoxy-$C_1$-$C_5$-alkyl, Hydroxy-$C_1$-$C_5$-alkyl und Halogen; worin $R^5$ und $R^6$ jeweils unabhängig voneinander ausgewählt sind aus Hydrido, $C_1$-$C_5$-Alkyl, Phenyl-$C_1$-$C_5$-alkyl, Phenyl und $C_1$-$C_5$-Alkoxy-$C_1$-$C_5$-alkyl; worin $R^5$ und $R^6$ zusammengenommen werden können unter Bildung von Oxo; worin $R^7$ bis $R^{11}$ jeweils unabhängig voneinander ausgewählt sind aus Hydrido, $C_1$-$C_5$-Alkyl, Hydroxy, Cycloalkyl mit 3 bis 8 Kohlenstoffatomen, Cycloalkylalkyl mit 4 bis 8 Kohlenstoffatomen, Phenyl-$C_1$-$C_5$-alkyl, Phenyl, $C_1$-$C_5$-Alkoxy, $C_1$-$C_5$-Alkoxy-$C_1$-$C_5$-alkyl, Phenoxy, Phenyl-$C_1$-$C_{10}$-alkoxy, Hydroxy-$C_1$-$C_5$-alkyl, Halogen und Halo-$C_1$-$C_5$-alkyl; worin $R^8$ und $R^9$ zusammengenommen werden können unter Bildung von Oxo; worin n eine Zahl ist, ausgewählt aus 0 bis einschließlich 5; worin p und q jeweils 2 bis 3 sind; worin X ausgewählt ist aus O, S $>$N-$R^{12}$; worin $R^{12}$ ausgewählt sein kann aus Hydrido, $C_1$-$C_5$-Alkyl, Cycloalkyl mit 3 bis 8 Kohlenstoffatomen, Cycloalkylalkyl mit 4 bis 8 Kohlenstoffatomen, Phenyl, Phenyl-$C_1$-$C_5$-alkyl, $C_1$-$C_5$-Alkoxy-$C_1$-$C_5$-alkyl, Hydroxy-$C_1$-$C_5$-alkyl, Niederalkanoyl und Heteroaryl, ausgewählt aus gesättigten, teilweise ungesättigten und voll gesättigten heterocyclischen Ringen, enthaltend 5 bis 7 Ringglieder, wovon 1 oder 2 Ringglieder ausgewählt sind aus einem Sauerstoffatom und einem Stickstoffatom; worin $R^{12}$ zusammen mit einem von $R^1$ bis $R^4$ einen verschmolzenen heterocyclischen Ring bilden können, enthaltend 5 oder 6 Ringglieder; worin Y jeweils unabhängig voneinander eine oder mehrere Gruppen bedeutet, ausgewählt aus Hydrido, Hydroxy, $C_1$-$C_{20}$-Alkyl, Cycloalkyl mit 3 bis 8 Kohlenstoffatomen, Cycloalkylalkyl mit 4 bis 8 Kohlenstoffatomen, Phenyl-$C_1$-$C_5$-alkyl, Phenyl, $C_1$-$C_5$-Alkoxy, Phenoxy, $C_1$-$C_5$-Alkoxy-$C_1$-$C_5$-alkyl, Halo-$C_1$-$C_5$-alkyl, Hydroxy-$C_1$-$C_5$-alkyl, Halogen, Carboxy, Carboxy-$C_1$-$C_5$-alkyl und Niederalkanoyl; oder ein pharmazeutisch verträgliches Salz davon.

4. Verbindung nach Anspruch 3 der Formel

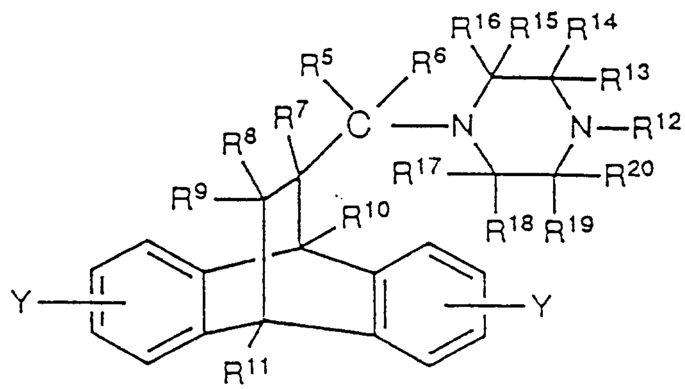

worin $R^5$ und $R^6$ jeweils unabhängig voneinander ausgewählt sind aus Hydrido, $C_1$-$C_5$-Alkyl, Benzyl und Phenyl; worin $R^7$ bis $R^{11}$ jeweils unabhängig voneinander ausgewählt sind aus Hydrido, $C_1$-$C_5$-Alkyl, Hydroxy, Benzyl, Phenyl, $C_1$-$C_5$-Alkoxy, Phenoxy, Benzyloxy, Halogen und Halo-$C_1$-$C_5$-alkyl; worin $R^{12}$ ausgewählt sein kann aus Hydrido, $C_1$-$C_5$-Alkyl, Cycloalkyl mit 5 oder 6 Kohlenstoffatomen,

Cycloalkylalkyl mit 6 oder 7 Kohlenstoffatomen, Phenyl, Benzyl, Hydroxy-$C_1$-$C_5$-alkyl und Heteroaryl, ausgewählt aus gesättigten oder voll ungesättigten heterocyclischen Ringen, enthaltend 5 bis 7 Ringglieder, wovon 1 bis 2 Ringglieder ein Stickstoffatom sind; worin Y jeweils unabhängig voneinander eine oder zwei Gruppen sind, ausgewählt aus Hydrido, Hydroxy, $C_1$-$C_5$-Alkyl, Benzyl, Phenyl, $C_1$-$C_5$-Alkoxy, Phenoxy, Halo-$C_1$-$C_5$-alkyl, Halogen und Niederalkanoyl; und worin $R^{13}$ bis $R^{20}$ jeweils unabhängig voneinander ausgewählt sind aus Hydrido, $C_1$-$C_5$-Alkyl, Benzyl, Phenyl und Halogen; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; oder ein pharmazeutisch veträgliches Salz davon.

**5.** Verbindung nach Anspruch 4, worin $R^5$ und $R^6$ jeweils unabhängig voneinander Hydrido oder Methyl sind; worin $R^7$ bis $R^{11}$ jeweils unabhängig voneinander ausgewählt sind aus Hydrido, Methyl, Ethyl, Hydroxy, Methoxy, Halogen und Trihalomethyl; worin $R^{12}$ ausgewählt ist aus Hydrido, Methyl, Ethyl, Hydroxyethyl, Benzyl, Pyridyl und Pyrimidyl; worin $R^{13}$ bis $R^{20}$ jeweils unabhängig voneinander ausgewählt sind aus Hydrido, Methyl und Ethyl; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; und worin Y jeweils unabhängig voneinander eine oder mehrere Gruppen bedeutet, ausgewählt aus Hydrido, Methyl, Ethyl, Hydroxy, Methoxy, Trihalomethyl und Halogen.

**6.** Verbindung nach Anspruch 5, ausgewählt aus 4-[(9,10-Dihydro-9,10-ethanoanthracenyl)methyl]-1-(2-pyrimidyl)piperazin; 4-[(9,10-Dihydro-9,10-ethanoanthracenyl)methyl]-1,4-diazabicyclo[4.3.0]-nonan und 4-[(9,10-Dihydro-9,10-ethanoanthracenyl)methyl]-1-benzylpiperazin.

**7.** Eine pharmazeutische Zusammensetzung, enthaltend eine therapeutisch wirksame Menge einer aktiven Verbindung und einen pharmazeutisch verträglichen Träger oder Verdünnungsmittel, wobei diese aktive Verbindung ausgewählt ist aus einer Verbindungsfamilie gemäß einem der Ansprüche 1, 2, 3, 4, 5 oder 6.

**8.** Verwendung einer therapeutisch wirksamen Menge einer Verbindung gemäß einem der Ansprüche 1, 2, 4, 5 oder 6 zur Herstellung eines Medikamentes zur Behandlung von Störungen des zentralen Nervensystems.

**9.** Verwendung nach Anspruch 8, worin diese Störung des zentralen Nervensystems ausgewählt ist aus einer psychotischen Störung, einer konvulsiven Störung und einer dystonischen Störung.

**10.** Verwendung nach Anspruch 9, worin diese Störung des zentralen Nervensystems eine psychotische Störung ist.

**11.** Ein Verfahren zur Herstellung einer Produktverbindung der Formel

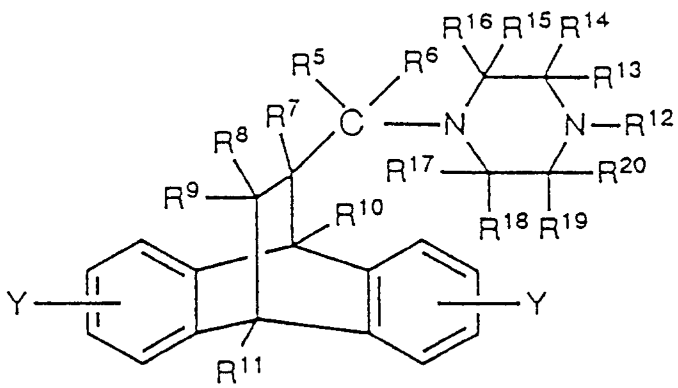

worin $R^5$ und $R^6$ jeweils unabhängig voneinander ausgewählt sind aus Hydrido, $C_1$-$C_5$-Alkyl, Benzyl und Phenyl; worin $R^7$ bis $R^{11}$ jeweils unabhängig voneinander ausgewählt sind aus Hydrido, $C_1$-$C_5$-Alkyl, Hydroxy, Benzyl, Phenyl, $C_1$-$C_5$-Alkoxy, Phenoxy, Benzyloxy, Halogen und Halo-$C_1$-$C_5$-alkyl; worin $R^{12}$ ausgewählt sein kann aus Hydrido, $C_1$-$C_5$-Alkyl, Cycloalkyl mit 5 oder 6 Kohlenstoffatomen, Cycloalkylalkyl mit 6 oder 7 Kohlenstoffatomen, Phenyl, Benzyl, Hydroxy-$C_1$-$C_5$-alkyl und Heteroaryl,

ausgewählt aus gesättigten oder voll ungesättigten heterocyclischen Ringen, enthaltend 5 bis 7 Ringglieder, wovon 1 bis 2 Ringglieder ein Stickstoffatom sind; worin Y jeweils unabhängig voneinander eine oder zwei Gruppen sind, ausgewählt aus Hydrido, Hydroxy, $C_1$-$C_5$-Alkyl, Benzyl, Phenyl, $C_1$-$C_5$-Alkoxy, Phenoxy, Halo-$C_1$-$C_5$-alkyl, Halogen und Niederalkanoyl; und worin $R^{13}$ bis $R^{20}$ jeweils unabhängig voneinander ausgewählt sind aus Hydrido, $C_1$-$C_5$-Alkyl, Benzyl, Phenyl und Halogen; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; oder eines pharmazeutisch veträglichen Salzes davon, umfassend die Umsetzung einer Verbindung der Formel

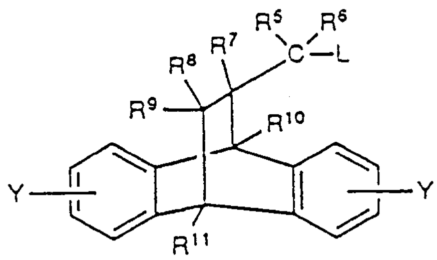

worin $R^5$ bis $R^{11}$ und Y jeweils vorstehende Bedeutung haben und worin L ausgewählt ist aus Halogen, Hydroxy, para-Toluolsulfonyloxy, Methylsulfonyloxy und para-Bromtoluolsulfonyloxy, mit einem Amin der Formel

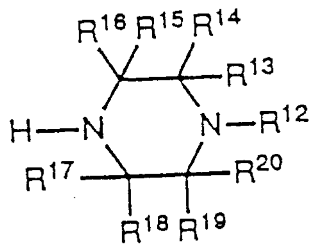

worin $R^{12}$ bis $R^{20}$ jeweils vorstehende Bedeutung haben.

**12.** Das Verfahren nach Anspruch 11, worin $R^5$ und $R^6$ jeweils unabhängig voneinander Hydrido oder Methyl sind; worin $R^7$ bis $R^{11}$ jeweils unabhängig voneinander ausgewählt sind aus Hydrido, Methyl, Ethyl, Hydroxy, Methoxy, Halogen und Trihalomethyl; worin $R^{12}$ ausgewählt ist aus Hydrido, Methyl, Ethyl, Hydroxyethyl, Benzyl, Pyridyl und Pyrimidyl; worin $R^{13}$ bis $R^{20}$ jeweils unabhängig voneinander ausgewählt sind aus Hydrido, Methyl, Ethyl und Trihalomethyl; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; worin Y jeweils unabhängig voneinander eine oder mehrere Gruppen bedeutet, ausgewählt aus Hydrido, Methyl, Ethyl, Hydroxy, Methoxy, Trihalomethyl und Halogen; und worin L ausgewählt ist aus Halogen und Hydroxy.

**13.** Das Verfahren nach Anspruch 12, worin L Halogen ist.

**14.** Das Verfahren nach Anspruch 13, worin L Chlor oder Brom ist.

**15.** Das Verfahren nach Anspruch 12, worin L Hydroxy ist.

**16.** Das Verfahren nach Anspruch 11, worin eine Verbindung der Formel

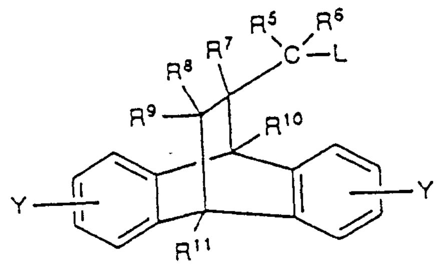

hergestellt wird durch Vermischen eines Alkohols der Formel

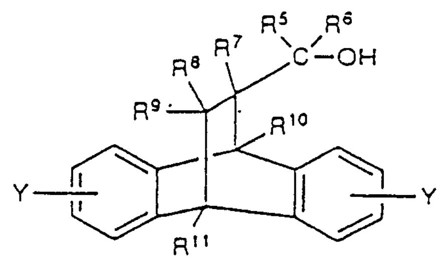

worin $R^5$ bis $R^{11}$ und L jeweils vorstehende Bedeutung haben, mit einem Reagens, ausgewählt aus Thionylchlorid, Phosphoroxychlorid, Triphenylphosphindibromid, Methansulfonylchlorid und p-Toluolsulfonylchlorid.

**17.** Das Verfahren nach Anspruch 11, worin diese Produktverbindung ausgewählt ist aus 4-[(9,10-Dihydro-9,10-ethanoanthracenyl)methyl]-1-methylpiperazin; 4-[(9,10-Dihydro-9,10-ethanoanthracenyl)methyl]-1-(2-hydroxyethyl)-piperazin; 4-[(9,10-Dihydro-9,10-ethanoanthracenyl)methyl]-1-(2-pyrimidyl)piperazin; 4-[(9,10-Dihydro-9,10-ethanoanthracenyl)methyl]-1,4-diazabicyclo[4.3.0]-nonan und 4-[(9,10-Dihydro-9.10-ethanoanthracenyl)methyl]-1-benzylpiperazin.

**18.** Das Verfahren nach Anspruch 17, worin diese Produktverbindung ausgewählt ist aus 4-[(9,10-Dihydro-9,10-ethanoanthracenyl)methyl]-1-methylpiperazin; 4-[(9,10-Dihydro-9,10-ethanoanthracenyl)methyl]-1-(2-hydroxyethyl)-piperazin und 4-[(9,10-Dihydro-9,10-ethanoanthracenyl)methyl]-1-benzylpiperazin.

**Patentansprüche für folgenden Vertragsstaat : ES**

**1.** Ein Verfahren zur Herstellung einer Verbindung der Formel

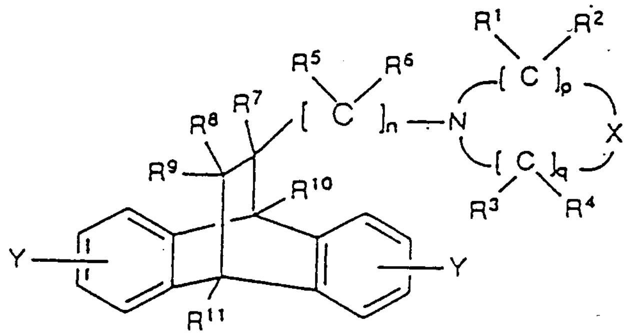

worin worin $R^1$ bis $R^6$ jeweils unabhängig voneinander ausgewählt sind aus Hydrido, $C_1$-$C_{20}$-Alkyl, $C_3$-$C_{10}$-Cycloalkyl, $C_3$-$C_{10}$-Cycloalkyl-$C_1$-$C_{20}$-alkyl, Phenyl-$C_1$-$C_{20}$-alkyl, Phenyl, $C_{1-10}$-Alkoxyalkyl, $C_1$-$C_{20}$-Hydroxyalkyl und Halogen; worin $R^1$ und $R^2$ zusammengenommen werden können unter Bildung

von Oxo; worin $R^3$ und $R^4$ zusammengenommen werden können unter Bildung von Oxo; worin $R^5$ und $R^6$ zusammengenommen werden können unter Bildung von Oxo; worin $R^7$ bis $R^{11}$ jeweils unabhängig voneinander ausgewählt sind aus Hydrido, $C_1$-$C_{20}$-Alkyl, Hydroxy, $C_3$-$C_{10}$-Cycloalkyl, $C_3$-$C_{10}$-Cycloalkyl-$C_1$-$C_{20}$-alkyl, Phenyl-$C_1$-$C_{20}$-alkyl, Phenyl, $C_1$-$C_{10}$-Alkoxy, $C_1$-$C_{10}$-Alkoxyalkyl, Phenyloxy, Phenyl-$C_1$-$C_{10}$-alkoxy, $C_1$-$C_{20}$-Hydroxyalkyl, Halogen und $C_1$-$C_{20}$-Haloalkyl; worin $R^8$ und $R^9$ zusammengenommen werden können unter Bildung von Oxo; worin n eine Zahl ist ausgewählt aus 0 bis 5 einschließlich; worin p und q jeweils eine Zahl ist ausgewählt aus 1 bis 4 einschließlich; worin X ausgewählt ist aus O, S, $>$N-$R^{12}$, $>$SO und $>SO_2$; worin $R^{12}$ ausgewählt sein kann aus Hydrido, $C_1$-$C_{20}$-Alkyl, $C_3$-$C_{10}$-Cycloalkyl, $C_3$-$C_{10}$-Cycloalkyl-$C_1$-$C_{20}$-alkyl, Phenyl, Phenyl-$C_1$-$C_{20}$-alkyl, einem aromatischen Ringsystem, enthaltend ein oder zwei Heteroatome ausgewählt aus Sauerstoff, Stickstoff, Schwefel in einem Ringsystem mit fünf oder sechs Ringgliedern, $C_1$-$C_{10}$-Alkoxyalkyl, $C_1$-$C_{20}$-Hydroxyalkyl, Alkanoyl, Aralkanoyl, Aroyl, Amino-$C_1$-$C_{20}$-alkyl, Mono-$C_1$-$C_{20}$-alkylamino-$C_1$-$C_{20}$-alkyl und Di-$C_1$-$C_{20}$-alkylamino-$C_1$-$C_{20}$-alkyl; worin $R^{12}$ zusammen mit einem von $R^1$ bis $R^4$ einen verschmolzenen heterocyclischen Ring bilden können, enthaltend fünf bis acht Ringglieder; worin jedes Y unabhängig voneinander eine oder mehrere Gruppen bedeutet, ausgewählt aus Hydrido, Hydroxy, $C_1$-$C_{20}$-Alkyl, $C_3$-$C_{10}$-Cycloalkyl, $C_3$-$C_{10}$-Cycloalkyl-$C_1$-$C_{20}$-alkyl, Phenyl-$C_1$-$C_{20}$-alkyl, Phenyl, $C_1$-$C_{10}$-Alkoxy, Phenyl-$C_1$-$C_{10}$-alkoxy, Phenyloxy, $C_1$-$C_{10}$-Alkoxyalkyl, $C_1$-$C_{20}$-Haloalkyl, $C_1$-$C_{20}$-Hydroxyalkyl, Halogen, Cyano, Amino, Mono-$C_1$-$C_{20}$-alkylamino, Di-$C_1$-$C_{20}$-alkylamino, Nitro, Carboxy, Carboxy-$C_1$-$C_{20}$-alkyl und Alkanoyl; oder ein pharmazeutisch verträgliches Salz davon; unter der Bedingung, daß, wenn X $>$N$R^{12}$ ist und p und q jeweils zwei sind und $R^1$ bis $R^4$ jeweils Hydrido sind und $R^7$ bis $R^{11}$ Hydrido sind und $R^{12}$ ausgewählt ist aus Hydrido, Methyl, Hydroxyethyl und Acetoxy, dann kann $R^5$ zusammen mit $R^6$ nicht Oxo sein und mindestens eines von $R^5$ und $R^6$ darf kein Hydrido sein; und unter der weiteren Bedingung, daß, wenn X ein Sauerstoffatom ist und p und q jeweils zwei sind, jede der nachstehenden Auswahlen nicht gemacht werden kann:

- $R^5$ und $R^6$ zusammen Oxo sind, wenn $R^1$ bis $R^4$, $R^7$ bis $R^{11}$ und Y Hydrido sind;
- jeweils $R^1$ bis $R^{11}$ und Y Hydrido sind;
- $R^8$ und $R^9$ zusammen Oxo sind, wenn $R^1$ bis $R^4$, $R^7$ bis $R^{11}$ und Y Hydrido sind;
- $R^8$ und $R^9$ zusammen Oxo sind wobei $R^{10}$ und $R^{11}$ jeweils Methyl sind, wenn $R^1$ bis $R^4$, $R^7$ bis $R^9$ und Y jeweils Hydrido sind, dadurch gekennzeichnet, daß eine Verbindung der Formel 3

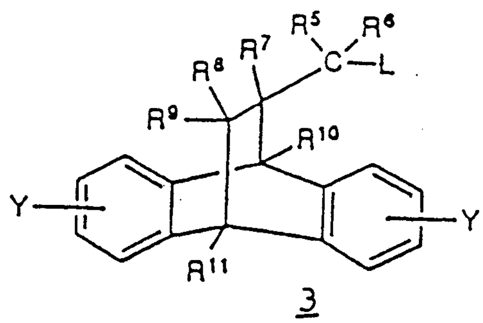

3

worin $R^5$ bis $R^{11}$ und Y jeweils vorstehende Bedeutung haben; und worin L ausgewählt ist aus Halogen, Hydroxy, para-Toluolsulfonyloxy, Methylsulfonyloxy und para-Bromtoluolsulfonyloxy, umgesetzt wird mit einer Verbindung der Formel 5

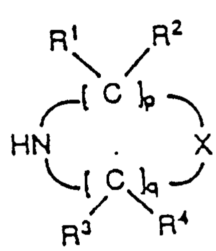

5

und Alkanoyl; oder ein pharmazeutisch verträgliches Salz davon; unter der Bedingung, daß, wenn X

> $NR^{12}$ ist und p und q jeweils zwei sind und $R^1$ bis $R^4$ jeweils Hydrido sind und $R^7$ bis $R^{11}$ Hydrido sind und $R^{12}$ ausgewählt ist aus Hydrido, Methyl, Hydroxyethyl und Acetoxy, dann kann $R^5$ zusammen mit $R^6$ nicht Oxo sein und mindestens eines von $R^5$ und $R^6$ darf kein Hydrido sein; und unter der weiteren Bedingung, daß, wenn X ein Sauerstoffatom ist und p und q jeweils zwei sind, jede der nachstehenden Auswahlen nicht gemacht werden kann:

- $R^5$ und $R^6$ zusammen Oxo sind, wenn $R^1$ bis $R^4$, $R^7$ bis $R^{11}$ und Y Hydrido sind;
- jeweils $R^1$ bis $R^{11}$ und Y Hydrido sind;
- $R^8$ und $R^9$ zusammen Oxo sind, wenn $R^1$ bis $R^4$, $R^7$ bis $R^{11}$ und Y Hydrido sind;
- $R^8$ und $R^9$ zusammen Oxo sind wobei $R^{10}$ und $R^{11}$ jeweils Methyl sind, wenn $R^1$ bis $R^4$, $R^7$ bis $R^9$ und Y jeweils Hydrido sind, dadurch gekennzeichnet, daß eine Verbindung der Formel $\underline{3}$

worin $R^5$ bis $R^{11}$ und Y jeweils vorstehende Bedeutung haben; und worin L ausgewählt ist aus Halogen, Hydroxy, para-Toluolsulfonyloxy, Methylsulfonyloxy und para-Bromtoluolsulfonyloxy,
umgesetzt wird mit einer Verbindung der Formel $\underline{5}$ worin p, q, X und $R^1$ bis $R^4$ vorstehende Bedeutung haben.

**2.** Verfahren nach Anspruch 1, worin $R^1$ bis $R^6$ der hergestellten Verbindung jeweils unabhängig voneinander ausgewählt sind aus Hydrido; $C_1$-$C_{20}$-Alkyl, $C_3$-$C_{10}$-Cycloalkyl, $C_3$-$C_{10}$-Cycloalkyl-$C_1$-$C_{20}$-alkyl, Phen-$C_1$-$C_{20}$-alkyl, Phenyl, $C_1$-$C_{10}$-Alkoxyalkyl, $C_1$-$C_{20}$-Hydroxyalkyl und Halogen; worin $R^5$ und $R^6$ zusammengenommen werden können unter Bildung von Oxo; worin $R^7$ bis $R^{11}$ jeweils unabhängig ausgewählt sind aus Hydrido, $C_1$-$C_{20}$-Alkyl, $C_1$-$C_{10}$-Hydroxy, $C_3$-$C_{10}$-Cycloalkyl, $C_3$-$C_{10}$-Cycloalkyl-$C_1$-$C_{20}$-alkyl, Phen-$C_1$-$C_{20}$-alkyl, Phenyl, $C_1$-$C_{10}$-Alkoxy, $C_1$-$C_{10}$-Alkoxyalkyl, Phenoxy, Phenyl-$C_1$-$C_{10}$-alkoxy, $C_1$-$C_{20}$-Hydroxyalkyl, Halogen und $C_2$-$C_{20}$-Haloalkyl; worin $R^8$ und $R^9$ zusammengenommen werden können unter Bildung von Oxo; worin n eine Zahl ist ausgewählt aus 0 bis einschließlich 5; worin p und q jeweils eine Zahl sind ausgewählt aus 1 bis einschließlich 4; worin X ausgewählt ist aus O, S und N-$R^{12}$; worin $R^{12}$ ausgewählt sein kann aus Hydrido, $C_1$-$C_{20}$-Alykl, $C_3$-$C_{10}$-Cycloalkyl, $C_3$-$C_{10}$-Cycloalkyl-$C_1$-$C_{20}$-alkyl, Phenyl, Phen-$C_1$-$C_{20}$-alkyl und einem aromatischen Ringsystem, enthaltend ein oder zwei Heteroatome ausgewählt aus Sauerstoff, Stickstoff, Schwefel in einem Ringsystem mit fünf oder sechs Ringgliedern, $C_1$-$C_{10}$-Alkoxyalkyl, $C_1$-$C_{12}$-Hydroxyalkyl, Alkanoyl, Phenalkanoyl, Aroyl, Amino-$C_1$-$C_{20}$-alkyl, Mono-$C_1$-$C_{20}$-alkylamino-$C_1$-$C_{20}$-alkyl und Di-$C_1$-$C_{20}$-alkylamino-$C_1$-$C_{20}$-alkyl; worin $R^{12}$ zusammen mit einem von $R^1$ bis $R^4$ ein verschmolzenes heterocyclisches Ringsystem bilden, enthaltend fünf bis acht Ringglieder; worin Y jeweils unabhängig voneinander eine oder mehrere Gruppen ist, ausgewählt aus Hydrido, Hydroxy, $C_1$-$C_{20}$-Alkyl, $C_3$-$C_{10}$-Cycloalkyl, $C_3$-$C_{10}$-Cycloalkyl-$C_1$-$C_{20}$-alkyl, Phen-$C_1$-$C_{20}$-alkyl, Phenyl, $C_1$-$C_{10}$-Alkoxy, Phen-$C_1$-$C_{10}$-alkoxy, Phenoxy, $C_1$-$C_{10}$-Alkoxyalkyl, $C_1$-$C_{20}$-Haloalkyl, $C_1$-$C_{20}$-Hydroxyalkyl, Halogen, Cyano, Amino, Mono-$C_1$-$C_{20}$-alkylamino, Di-$C_1$-$C_{20}$-alkylamino,Carboxy,Carobxy-$C_1$-$C_{20}$-alkylundAlkanoyl; oder ein pharmazeutisch verträgliches Salz davon.

**3.** Verfahren nach Anspruch 2, worin $R^1$ bis $R^4$ der hergestellten Verbindung jeweils unabhängig voneinander ausgewählt sind aus Hydrido, $C_1$-$C_5$-Alkyl, Cycloalkyl mit 3 bis 8 Kohlenstoffatomen, Cycloalkylalkyl mit 4 bis 8 Kohlenstoffatomen, Phenyl-$C_1$-$C_5$-alkyl, Phenyl, $C_1$-$C_5$-Alkoxy-$C_1$-$C_5$-alkyl, Hydroxy-$C_1$-$C_5$-alkyl und Halogen; worin $R^5$ und $R^6$ jeweils unabhängig voneinander ausgewählt sind aus Hydrido, $C_1$-$C_5$-Alkyl, Phenyl-$C_1$-$C_5$-alkyl, Phenyl und $C_1$-$C_5$-Alkoxy-$C_1$-$C_5$-alkyl; worin $R^5$ und $R^6$ zusammengenommen werden können unter Bildung von Oxo; worin $R^7$ bis $R^{11}$ jeweils unabhängig voneinander ausgewählt sind aus Hydrido, $C_1$-$C_5$-Alkyl, Hydroxy, Cycloalkyl mit 3 bis 8 Kohlenstoffatomen, Cycloalkylalkyl mit 4 bis 8 Kohlenstoffatomen, Phenyl-$C_1$-$C_5$-alkyl, Phenyl, $C_1$-$C_5$-Alkoxy, $C_1$-$C_5$-Alkoxy-$C_1$-$C_5$-alkyl, Phenoxy, Phenyl-$C_1$-$C_{10}$-alkoxy, Hydroxy-$C_1$-$C_5$-alkyl, Halogen und Halo-$C_1$-$C_5$-alkyl; worin $R^8$ und $R^9$ zusammengenommen werden können unter Bildung von Oxo; worin n eine Zahl ist, ausgewählt aus 0 bis einschließlich 5; worin p und q jeweils 2 bis 3 sind; worin X ausgewählt ist aus O, S > N-$R^{12}$; worin $R^{12}$ ausgewählt sein kann aus Hydrido, $C_1$-$C_5$-Alkyl, Cycloalkyl mit 3 bis 8 Kohlenstoffatomen, Cycloalkylalkyl mit 4 bis 8 Kohlenstoffatomen, Phenyl, Phenyl-$C_1$-$C_5$-alkyl, $C_1$-$C_5$-Alkoxy-$C_1$-$C_5$-alkyl, Hydroxy-$C_1$-$C_5$-alkyl, Niederalkanoyl und Heteroaryl, ausgewählt aus gesättigten, teilweise ungesättigten und voll gesättigten heterocyclischen Ringen, enthaltend 5 bis 7 Ringglieder, wovon 1 oder 2 Ringglieder ausgewählt sind aus einem Sauerstoffatom und einem Stickstoffatom; worin $R^{12}$ zusammen mit einem von $R^1$ bis $R^4$ einen verschmolzenen heterocyclischen Ring bilden können, enthaltend 5 oder 6 Ringglieder; worin Y jeweils unabhängig voneinander eine oder mehrere Gruppen bedeutet, ausgewählt aus Hydrido, Hydroxy, $C_1$-$C_{20}$-Alkyl, Cycloalkyl mit 3 bis 8 Kohlenstoffatomen, Cycloalkylalkyl mit 4 bis 8 Kohlenstoffatomen, Phenyl-$C_1$-$C_5$-alkyl, Phenyl, $C_1$-$C_5$-Alkoxy, Phenoxy, $C_1$-$C_5$-Alkoxy-$C_1$-$C_5$-alkyl, Halo-$C_1$-$C_5$-alkyl, Hydroxy-$C_1$-$C_5$-alkyl, Halogen, Carboxy, Carboxy-$C_1$-$C_5$-alkyl

und Niederalkanoyl; oder ein pharmazeutisch verträgliches Salz davon.

4. Verfahren nach Anspruch 3 der Formel

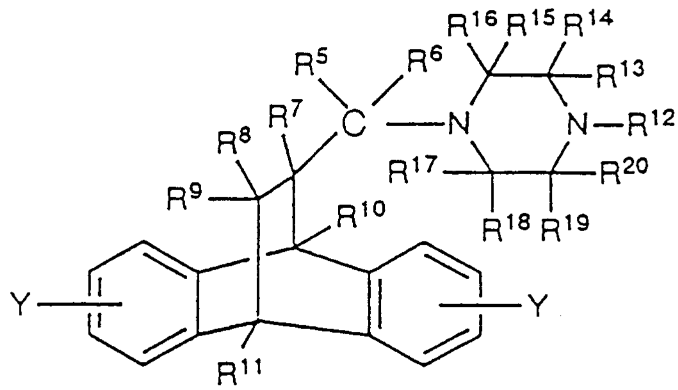

worin $R^5$ und $R^6$ der hergestellten Verbindung jeweils unabhängig voneinander ausgewählt sind aus Hydrido, $C_1$-$C_5$-Alkyl, Benzyl und Phenyl; worin $R^7$ bis $R^{11}$ jeweils unabhängig voneinander ausgewählt sind aus Hydrido, $C_1$-$C_5$-Alkyl, Hydroxy, Benzyl, Phenyl, $C_1$-$C_5$-Alkoxy, Phenoxy, Benzyloxy, Halogen und Halo-$C_1$-$C_5$-alkyl; worin $R^{12}$ ausgewählt sein kann aus Hydrido, $C_1$-$C_5$-Alkyl, Cycloalkyl mit 5 oder 6 Kohlenstoffatomen, Cycloalkylalkyl mit 6 oder 7 Kohlenstoffatomen, Phenyl, Benzyl, Hydroxy-$C_1$-$C_5$-alkyl und Heteroaryl, ausgewählt aus gesättigten oder voll ungesättigten heterocyclischen Ringen, enthaltend 5 bis 7 Ringglieder, wovon 1 bis 2 Ringglieder ein Stickstoffatom sind; worin Y jeweils unabhängig voneinander eine oder zwei Gruppen sind, ausgewählt aus Hydrido, Hydroxy, $C_1$-$C_5$-Alkyl, Benzyl, Phenyl, $C_1$-$C_5$-Alkoxy, Phenoxy, Halo-$C_1$-$C_5$-alkyl, Halogen und Niederalkanoyl; und worin $R^{13}$ bis $R^{20}$ jeweils unabhängig voneinander ausgewählt sind aus Hydrido, $C_1$-$C_5$-Alkyl, Benzyl, Phenyl und Halogen; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; oder ein pharmazeutisch veträgliches Salz davon.

5. Verfahren nach Anspruch 4, worin $R^5$ und $R^6$ der hergestellten Verbindung jeweils unabhängig voneinander Hydrido oder Methyl sind; worin $R^7$ bis $R^{11}$ jeweils unabhängig voneinander ausgewählt sind aus Hydrido, Methyl, Ethyl, Hydroxy, Methoxy, Halogen und Trihalomethyl; worin $R^{12}$ ausgewählt ist aus Hydrido, Methyl, Ethyl, Hydroxyethyl, Benzyl, Pyridyl und Pyrimidyl; worin $R^{13}$ bis $R^{20}$ jeweils unabhängig voneinander ausgewählt sind aus Hydrido, Methyl und Ethyl; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; und worin Y jeweils unabhängig voneinander eine oder mehrere Gruppen bedeutet, ausgewählt aus Hydrido, Methyl, Ethyl, Hydroxy, Methoxy, Trihalomethyl und Halogen.

6. Verfahren nach Anspruch 5, worin die hergestellte Verbindung ausgewählt ist aus 4-[(9,10-Dihydro-9,10-ethanoanthracenyl)methyl]-1-(2-pyrimidyl)piperazin; 4-[(9,10-Dihydro-9,10-ethanoanthracenyl)methyl]-1,4-diazabicyclo[4.3.0]-nonan und 4-[(9,10-Dihydro-9,10-ethanoanthracenyl)methyl]-1-benzylpiperazin.

7. Ein Verfahren zur Herstellung einer pharmazeutischen Zusammensetzung, dadurch gekennzeichnet, daß eine therapeutisch wirksame Menge einer aktiven Verbindung mit einem pharmazeutisch verträglichen Träger oder Verdünnungsmittel vermischt wird, wobei diese aktive Verbindung ausgewählt ist aus einer Familie von Verbindungen gemäß einem der Ansprüche 1, 2, 3, 4, 5 oder 6.

8. Verwendung einer therapeutisch wirksamen Menge einer nach einem der Ansprüche 1, 2, 4, 5 oder 6 hergestellten Verbindung zur Herstellung eines Medikamentes zur Behandlung von Störungen des zentralen Nervensystems.

9. Verwendung nach Anspruch 8, worin diese Störung des zentralen Nervensystems ausgewählt ist aus einer psychotischen Störung, einer konvulsiven Störung und einer dystonischen Störung.

**10.** Verwendung nach Anspruch 9, worin diese Störung des zentralen Nervensystems eine psychotische Störung ist.

**11.** Ein Verfahren zur Herstellung einer Produktverbindung der Formel

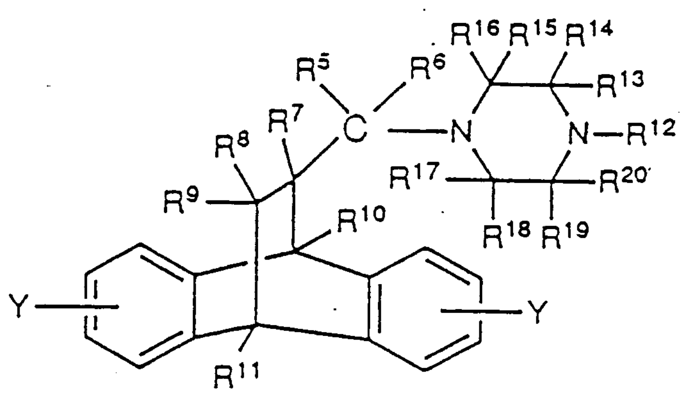

worin $R^5$ und $R^6$ jeweils unabhängig voneinander ausgewählt sind aus Hydrido, $C_1$-$C_5$-Alkyl, Benzyl und Phenyl; worin $R^7$ bis $R^{11}$ jeweils unabhängig voneinander ausgewählt sind aus Hydrido, $C_1$-$C_5$-Alkyl, Hydroxy, Benzyl, Phenyl, $C_1$-$C_5$-Alkoxy, Phenoxy, Benzyloxy, Halogen und Halo-$C_1$-$C_5$-alkyl; worin $R^{12}$ ausgewählt sein kann aus Hydrido, $C_1$-$C_5$-Alkyl, Cycloalkyl mit 5 oder 6 Kohlenstoffatomen, Cycloalkylalkyl mit 6 oder 7 Kohlenstoffatomen, Phenyl, Benzyl, Hydroxy-$C_1$-$C_5$-alkyl und Heteroaryl, ausgewählt aus gesättigten oder voll ungesättigten heterocyclischen Ringen, enthaltend 5 bis 7 Ringglieder, wovon 1 bis 2 Ringglieder ein Stickstoffatom sind; worin Y jeweils unabhängig voneinander eine oder zwei Gruppen sind, ausgewählt aus Hydrido, Hydroxy, $C_1$-$C_5$-Alkyl, Benzyl, Phenyl, $C_1$-$C_5$-Alkoxy, Phenoxy, Halo-$C_1$-$C_5$-alkyl, Halogen und Niederalkanoyl; und worin $R^{13}$ bis $R^{20}$ jeweils unabhängig voneinander ausgewählt sind aus Hydrido, $C_1$-$C_5$-Alkyl, Benzyl, Phenyl und Halogen; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; oder eines pharmazeutisch veträglichen Salzes davon, umfassend die Umsetzung einer Verbindung der Formel

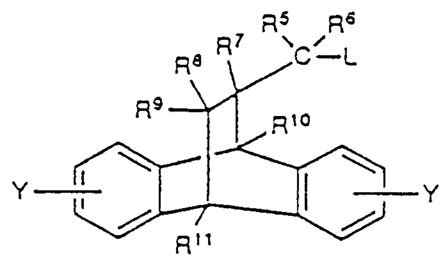

worin $R^5$ bis $R^{11}$ und Y jeweils vorstehende Bedeutung haben und worin L ausgewählt ist aus Halogen, Hydroxy, para-Toluolsulfonyloxy, Methylsulfonyloxy und para-Bromtoluolsulfonyloxy, mit einem Amin der Formel

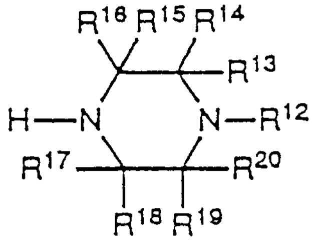

worin $R^{12}$ bis $R^{20}$ jeweils vorstehende Bedeutung haben.

**12.** Das Verfahren nach Anspruch 11, worin $R^5$ und $R^6$ jeweils unabhängig voneinander Hydrido oder Methyl sind; worin $R^7$ bis $R^{11}$ jeweils unabhängig voneinander ausgewählt sind aus Hydrido, Methyl, Ethyl, Hydroxy, Methoxy, Halogen und Trihalomethyl; worin $R^{12}$ ausgewählt ist aus Hydrido, Methyl, Ethyl, Hydroxyethyl, Benzyl, Pyridyl und Pyrimidyl; worin $R^{13}$ bis $R^{20}$ jeweils unabhängig voneinander ausgewählt sind aus Hydrido, Methyl, Ethyl und Trihalomethyl; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; worin Y jeweils unabhängig voneinander eine oder mehrere Gruppen bedeutet, ausgewählt aus Hydrido, Methyl, Ethyl, Hydroxy, Methoxy, Trihalomethyl und Halogen; und worin L ausgewählt ist aus Halogen und Hydroxy.

**13.** Das Verfahren nach Anspruch 12, worin L Halogen ist.

**14.** Das Verfahren nach Anspruch 13, worin L Chlor oder Brom ist.

**15.** Das Verfahren nach Anspruch 12, worin L Hydroxy ist.

**16.** Das Verfahren nach Anspruch 11, worin eine Verbindung der Formel

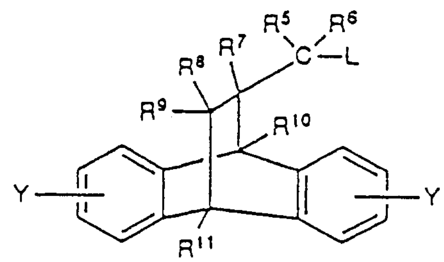

hergestellt wird durch Vermischen eines Alkohols der Formel

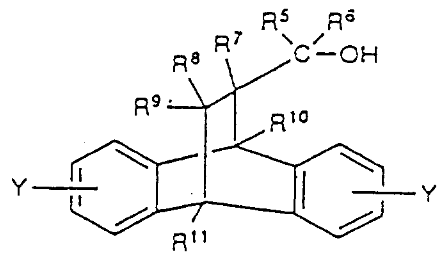

worin $R^5$ bis $R^{11}$ und L jeweils vorstehende Bedeutung haben, mit einem Reagens, ausgewählt aus Thionylchlorid, Phosphoroxychlorid, Triphenylphosphindibromid, Methansulfonylchlorid und p-Toluolsulfonylchlorid.

**17.** Das Verfahren nach Anspruch 11, worin diese Produktverbindung ausgewählt ist aus 4-[(9,10-Dihydro-9,10-ethanoanthracenyl)methyl]-1-methylpiperazin; 4-[(9,10-Dihydro-9,10-ethanoanthracenyl)methyl]-1-(2-hydroxyethyl)-piperazin; 4-[(9,10-Dihydro-9,10-ethanoanthracenyl)methyl]-1-(2-pyrimidyl)piperazin; 4-[(9,10-Dihydro-9,10-ethanoanthracenyl)methyl]-1,4-diazabicyclo[4.3.0]-nonan und 4-[(9,10-Dihydro-9,10-ethanoanthracenyl)methyl]-1-benzylpiperazin.

**18.** Das Verfahren nach Anspruch 17, worin diese Produktverbindung ausgewählt ist aus 4-[(9,10-Dihydro-9,10-ethanoanthracenyl)methyl]-1-methylpiperazin; 4-[(9,10-Dihydro-9,10-ethanoanthracenyl)methyl]-1-(2-hydroxyethyl)-piperazin und 4-[(9,10-Dihydro-9,10-ethanoanthracenyl)methyl]-1-benzylpiperazin.

**Patentansprüche für folgenden Vertragsstaat : GR**

1. Eine Verbindung der Formel

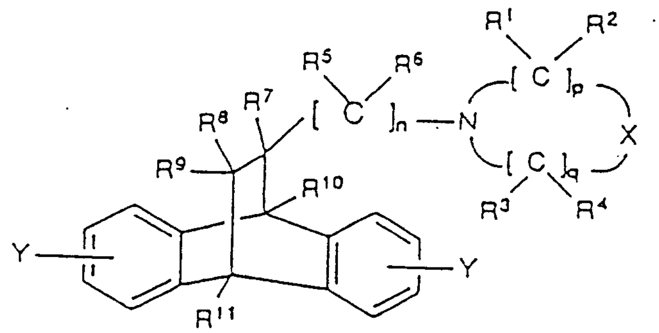

worin $R^1$ bis $R^6$ jeweils unabhängig voneinander ausgewählt sind aus Hydrido, $C_1$-$C_{20}$-Alkyl, $C_3$-$C_{10}$-Cycloalkyl, $C_3$-$C_{10}$-Cycloalkyl-$C_1$-$C_{20}$-alkyl, Phenyl-$C_1$-$C_{20}$-alkyl, Phenyl, $C_{1-10}$-Alkoxyalkyl, $C_1$-$C_{20}$-Hydroxyalkyl und Halogen; worin $R^1$ und $R^2$ zusammengenommen werden können unter Bildung von Oxo; worin $R^3$ und $R^4$ zusammenge nommen werden können unter Bildung von Oxo; worin $R^5$ und $R^6$ zusammengenommen werden können unter Bildung von Oxo; worin $R^7$ bis $R^{11}$ jeweils unabhängig voneinander ausgewählt sind aus Hydrido, $C_1$-$C_{20}$-Alkyl, Hydroxy, $C_3$-$C_{10}$-Cycloalkyl, $C_3$-$C_{10}$-Cycloalkyl-$C_1$-$C_{20}$-alkyl, Phenyl-$C_1$-$C_{20}$-alkyl, Phenyl, $C_1$-$C_{10}$-Alkoxy, $C_1$-$C_{10}$-Alkoxyalkyl, Phenyloxy, Phenyl-$C_1$-$C_{10}$-alkoxy, $C_1$-$C_{20}$-Hydroxyalkyl, Halogen und $C_1$-$C_{20}$-Haloalkyl; worin $R^8$ und $R^9$ zusammengenommen werden können unter Bildung von Oxo; worin n eine Zahl ist ausgewählt aus 0 bis 5 einschließlich; worin p und q jeweils eine Zahl ist ausgewählt aus 1 bis 4 einschließlich; worin X ausgewählt ist aus O, S, $>$N-$R^{12}$, $>$SO und $>SO_2$; worin $R^{12}$ ausgewählt sein kann aus Hydrido, $C_1$-$C_{20}$-Alkyl, $C_3$-$C_{10}$-Cycloalkyl, $C_3$-$C_{10}$-Cycloalkyl-$C_1$-$C_{20}$-alkyl, Phenyl, Phenyl-$C_1$-$C_{20}$-alkyl, einem aromatischen Ringsystem, enthaltend ein oder zwei Heteroatome ausgewählt aus Sauerstoff, Stickstoff, Schwefel in einem Ringsystem mit fünf oder sechs Ringgliedern, $C_1$-$C_{10}$-Alkoxyalkyl, $C_1$-$C_{20}$Hydroxyalkyl, Alkanoyl, Aralkanoyl, Aroyl, Amino-$C_1$-$C_{20}$-alkyl, Mono-$C_1$-$C_{20}$-alkylamino-$C_1$-$C_{20}$-alkyl und Di-$C_1$-$C_{20}$-alkylamino-$C_1$-$C_{20}$-alkyl; worin $R^{12}$ zusammen mit einem von $R^1$ bis $R^4$ einen verschmolzenen heterocyclischen Ring bilden können, enthaltend fünf bis acht Ringglieder; worin jedes Y unabhängig voneinander eine oder mehrere Gruppen bedeutet, ausgewählt aus Hydrido, Hydroxy, $C_1$-$C_{20}$-Alkyl, $C_3$-$C_{10}$-Cycloalkyl, $C_3$-$C_{10}$-Cycloalkyl-$C_1$-$C_{20}$-alkyl, Phenyl-$C_1$-$C_{20}$-alkyl, Phenyl, $C_1$-$C_{10}$-Alkoxy, Phenyl-$C_1$-$C_{10}$-alkoxy, Phenyloxy, $C_1$-$C_{10}$-Alkoxyalkyl, $C_1$-$C_{20}$-Haloalkyl, $C_1$-$C_{20}$-Hydroxyalkyl, Halogen, Cyano, Amino, Mono-$C_1$-$C_{20}$-alkylamino, Di-$C_1$-$C_{20}$-alkylamino, Nitro, Carboxy, Carboxy-$C_1$-$C_{20}$-alkyl und Alkanoyl; oder ein pharmazeutisch verträgliches Salz davon; unter der Bedingung, daß, wenn X $>NR^{12}$ ist und p und q jeweils zwei sind und $R^1$ bis $R^4$ jeweils Hydrido sind und $R^7$ bis $R^{11}$ Hydrido sind und $R^{12}$ ausgewählt ist aus Hydrido, Methyl, Hydroxyethyl und Acetoxy, dann kann $R^5$ zusammen mit $R^6$ nicht Oxo sein und mindestens eines von $R^5$ und $R^6$ darf kein Hydrido sein; und unter der weiteren Bedingung, daß, wenn X ein Sauerstoffatom ist und p und q jeweils zwei sind, jede der nachstehenden Auswahlen nicht gemacht werden kann:

- $R^5$ und $R^6$ zusammen Oxo sind, wenn $R^1$ bis $R^4$, $R^7$ bis $R^{11}$ und Y Hydrido sind;
- jeweils $R^1$ bis $R^{11}$ und Y Hydrido sind;
- $R^8$ und $R^9$ zusammen Oxo sind, wenn $R^1$ bis $R^4$, $R^7$ bis $R^{11}$ und Y Hydrido sind;
- $R^8$ und $R^9$ zusammen Oxo sind wobei $R^{10}$ und $R^{11}$ jeweils Methyl sind, wenn $R^1$ bis $R^4$, $R^7$ bis $R^9$ und Y jeweils Hydrido sind.

2. Verbindung nach Anspruch 1, worin $R^1$ bis $R^6$ jeweils unabhängig voneinander ausgewählt sind aus Hydrido; $C_1$-$C_{20}$-Alkyl, $C_3$-$C_{10}$-Cycloalkyl, $C_3$-$C_{10}$-Cycloalkyl-$C_1$-$C_{20}$-alkyl, Phen-$C_1$-$C_{20}$-alkyl, Phenyl, $C_1$-$C_{10}$-Alkoxyalkyl, $C_1$-$C_{20}$-Hydroxyalkyl und Halogen; worin $R^5$ und $R^6$ zusammengenommen werden können unter Bildung von Oxo; worin $R^7$ bis $R^{11}$ jeweils unabhängig ausgewählt sind aus Hydrido, $C_1$-$C_{20}$-Alkyl, $C_1$-$C_{10}$-Hydroxy, $C_3$-$C_{10}$-Cycloalkyl, $C_3$-$C_{10}$-Cycloalkyl-$C_1$-$C_{20}$-alkyl, Phen-$C_1$-$C_{20}$-alkyl, Phenyl, $C_1$-$C_{10}$-Alkoxy, $C_1$-$C_{10}$-Alkoxyalkyl, Phenoxy, Phenyl-$C_1$-$C_{10}$-alkoxy, $C_1$-$C_{20}$-Hydroxyalkyl, Halogen und $C_1$-$C_{20}$-Haloalkyl; worin $R^8$ und $R^9$ zusammengenommen werden können unter Bildung von Oxo; worin n eine Zahl ist ausgewählt aus 0 bis einschließlich 5; worin p und q jeweils eine Zahl sind ausgewählt aus 1 bis einschließlich 4; worin X ausgewählt ist aus O, S und $>$N-$R^{12}$; worin $R^{12}$

ausgewählt sein kann aus Hydrido, $C_1$-$C_{20}$-Alykl, $C_3$-$C_{10}$-Cycloalkyl, $C_3$-$C_{10}$-Cycloalkyl-$C_1$-$C_{20}$-alkyl, Phenyl, Phen-$C_1$-$C_{20}$-alkyl und einem aromatischen Ringsystem, enthaltend ein oder zwei Heteroatome ausgewählt aus Sauerstoff, Stickstoff, Schwefel in einem Ringsystem mit fünf oder sechs Ringgliedern, $C_1$-$C_{10}$-Alkoxyalkyl, $C_1$-$C_{12}$-Hydroxyalkyl, Alkanoyl, Phenalkanoyl, Aroyl, Amino-$C_1$-$C_{20}$-alkyl, Mono-$C_1$-$C_{20}$-alkylamino-$C_1$-$C_{20}$-alkyl und Di-$C_1$-$C_{20}$-alkylamino-$C_1$-$C_{20}$-alkyl; worin $R^{12}$ zusammen mit einem von $R^1$ bis $R^4$ ein verschmolzenes heterocyclisches Ringsystem bilden, enthaltend fünf bis acht Ringglieder; worin Y jeweils unabhängig voneinander eine oder mehrere Gruppen ist, ausgewählt aus Hydrido, Hydroxy, $C_1$-$C_{20}$-Alkyl, $C_3$-$C_{10}$-Cycloalkyl, $C_3$-$C_{10}$-Cycloalkyl-$C_1$-$C_{20}$-alkyl, Phen-$C_1$-$C_{20}$-alkyl, Phenyl, $C_1$-$C_{10}$-Alkoxy, Phen-$C_1$-$C_{10}$-alkoxy, Phenoxy, $C_1$-$C_{10}$-Alkoxyalkyl, $C_1$-$C_{20}$-Haloalkyl, $C_1$-$C_{20}$-Hydroxyalkyl, Halogen, Cyano, Amino, Mono-$C_1$-$C_{20}$-alkylamino, Di-$C_1$-$C_{20}$-alkylamino,Carboxy,Carobxy-$C_1$-$C_{20}$-alkylundAlkanoyl; oder ein pharmazeutisch verträgliches Salz davon.

**3.** Verbindung nach Anspruch 2, worin $R^1$ bis $R^4$ jeweils unabhängig voneinander ausgewählt sind aus Hydrido, $C_1$-$C_5$-Alkyl, Cycloalkyl mit 3 bis 8 Kohlenstoffatomen, Cycloalkylalkyl mit 4 bis 8 Kohlenstoffatomen, Phenyl-$C_1$-$C_5$-alkyl, Phenyl, $C_1$-$C_5$-Alkoxy-$C_1$-$C_5$-alkyl, Hydroxy-$C_1$-$C_5$-alkyl und Halogen; worin $R^5$ und $R^6$ jeweils unabhängig voneinander ausgewählt sind aus Hydrido, $C_1$-$C_5$-Alkyl, Phenyl-$C_1$-$C_5$-alkyl, Phenyl und $C_1$-$C_5$-Alkoxy-$C_1$-$C_5$-alkyl; worin $R^5$ und $R^6$ zusammengenommen werden können unter Bildung von Oxo; worin $R^7$ bis $R^{11}$ jeweils unabhängig voneinander ausgewählt sind aus Hydrido, $C_1$-$C_5$-Alkyl, Hydroxy, Cycloalkyl mit 3 bis 8 Kohlenstoffatomen, Cycloalkylalkyl mit 4 bis 8 Kohlenstoffatomen, Phenyl-$C_1$-$C_5$-alkyl, Phenyl, $C_1$-$C_5$-Alkoxy, $C_1$-$C_5$-Alkoxy-$C_1$-$C_5$-alkyl, Phenoxy, Phenyl-$C_1$-$C_{10}$-alkoxy, Hydroxy-$C_1$-$C_5$-alkyl, Halogen und Halo-$C_1$-$C_5$-alkyl; worin $R^8$ und $R^9$ zusammengenommen werden können unter Bildung von Oxo; worin n eine Zahl ist, ausgewählt aus 0 bis einschließlich 5; worin p und q jeweils 2 bis 3 sind; worin X ausgewählt ist aus O, S $>$N-$R^{12}$; worin $R^{12}$ ausgewählt sein kann aus Hydrido, $C_1$-$C_5$-Alkyl, Cycloalkyl mit 3 bis 8 Kohlenstoffatomen, Cycloalkylalkyl mit 4 bis 8 Kohlenstoffatomen, Phenyl, Phenyl-$C_1$-$C_5$-alkyl, $C_1$-$C_5$-Alkoxy-$C_1$-$C_5$-alkyl, Hydroxy-$C_1$-$C_5$-alkyl, Niederalkanoyl und Heteroaryl, ausgewählt aus gesättigten, teilweise ungesättigten und voll gesättigten heterocyclischen Ringen, enthaltend 5 bis 7 Ringglieder, wovon 1 oder 2 Ringglieder ausgewählt sind aus einem Sauerstoffatom und einem Stickstoffatom; worin $R^{12}$ zusammen mit einem von $R^1$ bis $R^4$ einen verschmolzenen heterocyclischen Ring bilden können, enthaltend 5 oder 6 Ringglieder; worin Y jeweils unabhängig voneinander eine oder mehrere Gruppen bedeutet, ausgewählt aus Hydrido, Hydroxy, $C_1$-$C_{20}$-Alkyl, Cycloalkyl mit 3 bis 8 Kohlenstoffatomen, Cycloalkylalkyl mit 4 bis 8 Kohlenstoffatomen, Phenyl-$C_1$-$C_5$-alkyl, Phenyl, $C_1$-$C_5$-Alkoxy, Phenoxy, $C_1$-$C_5$-Alkoxy-$C_1$-$C_5$-alkyl, Halo-$C_1$-$C_5$-alkyl, Hydroxy-$C_1$-$C_5$-alkyl, Halogen, Carboxy, Carboxy-$C_1$-$C_5$-alkyl und Niederalkanoyl; oder ein pharmazeutisch verträgliches Salz davon.

**4.** Verbindung nach Anspruch 3 der Formel

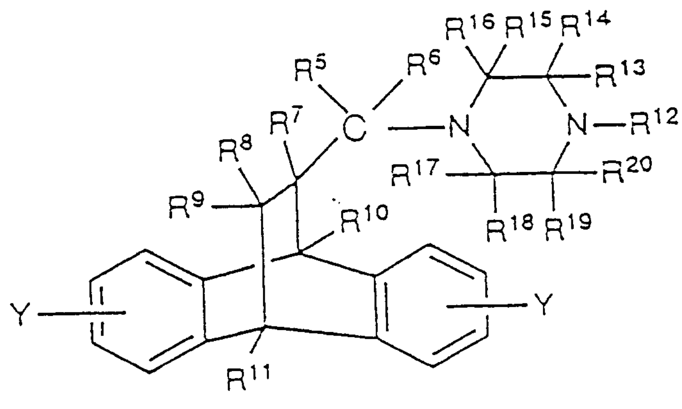

worin $R^5$ und $R^6$ jeweils unabhängig voneinander ausgewählt sind aus Hydrido, $C_1$-$C_5$-Alkyl, Benzyl und Phenyl; worin $R^7$ bis $R^{11}$ jeweils unabhängig voneinander ausgewählt sind aus Hydrido, $C_1$-$C_5$-Alkyl, Hydroxy, Benzyl, Phenyl, $C_1$-$C_5$-Alkoxy, Phenoxy, Benzyloxy, Halogen und Halo-$C_1$-$C_5$-alkyl; worin $R^{12}$ ausgewählt sein kann aus Hydrido, $C_1$-$C_5$-Alkyl, Cycloalkyl mit 5 oder 6 Kohlenstoffatomen, Cycloalkylalkyl mit 6 oder 7 Kohlenstoffatomen, Phenyl, Benzyl, Hydroxy-$C_1$-$C_5$-alkyl und Heteroaryl, ausgewählt aus gesättigten oder voll ungesättigten heterocyclischen Ringen, enthaltend 5 bis 7 Ringglieder, wovon 1 bis 2 Ringglieder ein Stickstoffatom sind; worin Y jeweils unabhängig voneinander

eine oder zwei Gruppen sind, ausgewählt aus Hydrido, Hydroxy, $C_1$-$C_5$-Alkyl, Benzyl, Phenyl, $C_1$-$C_5$-Alkoxy, Phenoxy, Halo-$C_1$-$C_5$-alkyl, Halogen und Niederalkanoyl; und worin $R^{13}$ bis $R^{20}$ jeweils unabhängig voneinander ausgewählt sind aus Hydrido, $C_1$-$C_5$-Alkyl, Benzyl, Phenyl und Halogen; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; oder ein pharmazeutisch veträgliches Salz davon.

**5.** Verbindung nach Anspruch 4, worin $R^5$ und $R^6$ jeweils unabhängig voneinander Hydrido oder Methyl sind; worin $R^7$ bis $R^{11}$ jeweils unabhängig voneinander ausgewählt sind aus Hydrido, Methyl, Ethyl, Hydroxy, Methoxy, Halogen und Trihalomethyl; worin $R^{12}$ ausgewählt ist aus Hydrido, Methyl, Ethyl, Hydroxyethyl, Benzyl, Ryridyl und Pyrimidyl; worin $R^{13}$ bis $R^{20}$ jeweils unabhängig voneinander ausgewählt sind aus Hydrido, Methyl und Ethyl; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; und worin Y jeweils unabhängig voneinander eine oder mehrere Gruppen bedeutet, ausgewählt aus Hydrido, Methyl, Ethyl, Hydroxy, Methoxy, Trihalomethyl und Halogen.

**6.** Verbindung nach Anspruch 5, ausgewählt aus 4-[(9,10-Dihydro-9,10-ethanoanthracenyl)methyl]-1-(2-pyrimidyl)piperazin; 4-[(9,10-Dihydro-9,10-ethanoanthracenyl)methyl]-1,4-diazabicyclo[4.3.0]-nonan und 4-[(9,10-Dihydro-9,10-ethanoanthracenyl)methyl]-1-benzylpiperazin.

**7.** Ein Verfahren zur Herstellung einer pharmazeutischen Zusammensetzung, dadurch gekennzeichnet. daß eine therapeutisch wirksame Menge einer aktiven Verbindung mit einem pharmazeutisch verträglichen Träger oder Verdünnungsmittel vermischt wird, wobei diese aktive Verbindung ausgewählt ist aus einer Familie von Verbindungen gemäß einem der Ansprüche 1, 2, 3, 4, 5 oder 6.

**8.** Verwendung einer therapeutisch wirksamen Menge einer Verbindung gemäß einem der Ansprüche 1, 2, 4, 5 oder 6 zur Herstellung eines Medikamentes zur Behandlung von Störungen des zentralen Nervensystems.

**9.** Verwendung nach Anspruch 8, worin diese Störung des zentralen Nervensystems ausgewählt ist aus einer psychotischen Störung, einer konvulsiven Störung und einer dystonischen Störung.

**10.** Verwendung nach Anspruch 9, worin diese Störung des zentralen Nervensystems eine psychotische Störung ist.

**11.** Ein Verfahren zur Herstellung einer Produktverbindung der Formel

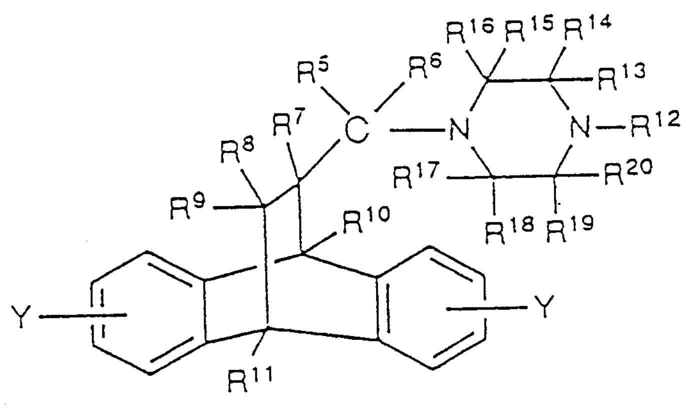

worin $R^5$ und $R^6$ jeweils unabhängig voneinander ausgewählt sind aus Hydrido, $C_1$-$C_5$-Alkyl, Benzyl und Phenyl; worin $R^7$ bis $R^{11}$ jeweils unabhängig voneinander ausgewählt sind aus Hydrido, $C_1$-$C_5$-Alkyl, Hydroxy, Benzyl, Phenyl, $C_1$-$C_5$-Alkoxy, Phenoxy, Benzyloxy, Halogen und Halo-$C_1$-$C_5$-alkyl; worin $R^{12}$ ausgewählt sein kann aus Hydrido, $C_1$-$C_5$-Alkyl, Cycloalkyl mit 5 oder 6 Kohlenstoffatomen, Cycloalkylalkyl mit 6 oder 7 Kohlenstoffatomen, Phenyl, Benzyl, Hydroxy-$C_1$-$C_5$-alkyl und Heteroaryl, ausgewählt aus gesättigten oder voll ungesättigten heterocyclischen Ringen, enthaltend 5 bis 7 Ringglieder, wovon 1 bis 2 Ringglieder ein Stickstoffatom sind; worin Y jeweils unabhängig voneinander eine oder zwei Gruppen sind, ausgewählt aus Hydrido, Hydroxy, $C_1$-$C_5$-Alkyl, Benzyl, Phenyl, $C_1$-$C_5$-

Alkoxy, Phenoxy, Halo-$C_1$-$C_5$-alkyl, Halogen und Niederalkanoyl; und worin $R^{13}$ bis $R^{20}$ jeweils unabhängig voneinander ausgewählt sind aus Hydrido, $C_1$-$C_5$-Alkyl, Benzyl, Phenyl und Halogen; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; oder eines pharmazeutisch veträglichen Salzes davon, umfassend die Umsetzung einer Verbindung der Formel

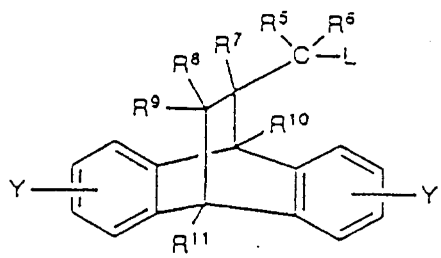

worin $R^5$ bis $R^{11}$ und Y jeweils vorstehende Bedeutung haben und worin L ausgewählt ist aus Halogen, Hydroxy, para-Toluolsulfonyloxy, Methylsulfonyloxy und para-Bromtoluolsulfonyloxy, mit einem Amin der Formel

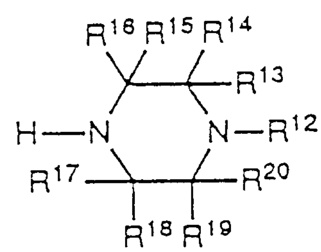

worin $R^{12}$ bis $R^{20}$ jeweils vorstehende Bedeutung haben.

**12.** Das Verfahren nach Anspruch 11, worin $R^5$ und $R^6$ jeweils unabhängig voneinander Hydrido oder Methyl sind; worin $R^7$ bis $R^{11}$ jeweils unabhängig voneinander ausgewählt sind aus Hydrido, Methyl, Ethyl, Hydroxy, Methoxy, Halogen und Trihalomethyl; worin $R^{12}$ ausgewählt ist aus Hydrido, Methyl, Ethyl, Hydroxyethyl, Benzyl, Pyridyl und Pyrimidyl; worin $R^{13}$ bis $R^{20}$ jeweils unabhängig voneinander ausgewählt sind aus Hydrido, Methyl, Ethyl und Trihalomethyl; worin $R^{12}$ zusammen mit einem von $R^{13}$, $R^{14}$, $R^{19}$ oder $R^{20}$ einen verschmolzenen heterocyclischen Ring bilden kann, enthaltend 5 oder 6 Ringglieder; worin Y jeweils unabhängig voneinander eine oder mehrere Gruppen bedeutet, ausgewählt aus Hydrido, Methyl, Ethyl, Hydroxy, Methoxy, Trihalomethyl und Halogen; und worin L ausgewählt ist aus Halogen und Hydroxy.

**13.** Das Verfahren nach Anspruch 12, worin L Halogen ist.

**14.** Das Verfahren nach Anspruch 13, worin L Chlor oder Brom ist.

**15.** Das Verfahren nach Anspruch 12, worin L Hydroxy ist.

**16.** Das Verfahren nach Anspruch 11, worin eine Verbindung der Formel

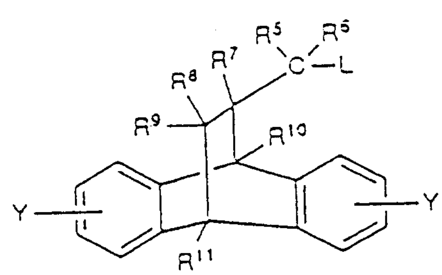

hergestellt wird durch Vermischen eines Alkohols der Formel

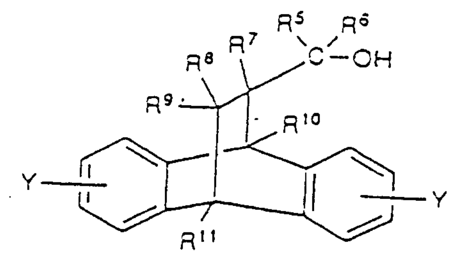

worin $R^5$ bis $R^{11}$ und L jeweils vorstehende Bedeutung haben, mit einem Reagens, ausgewählt aus Thionylchlorid, Phosphoroxychlorid, Triphenylphosphindibromid, Methansulfonylchlorid und p-Toluolsulfonylchlorid.

**17.** Das Verfahren nach Anspruch 11, worin diese Produktverbindung ausgewählt ist aus 4-[(9,10-Dihydro-9,10-ethanoanthracenyl)methyl]-1-methylpiperazin; 4-[(9,10-Dihydro-9,10-ethanoanthracenyl)methyl]-1-(2-hydroxyethyl)-piperazin; 4-[(9,10-Dihydro-9,10-ethanoanthracenyl)methyl]-1-(2-pyrimidyl)piperazin; 4-[(9,10-Dihydro-9,10-ethanoanthracenyl)methyl]-1,4-diazabicyclo[4.3.0]-nonan und 4-[(9,10-Dihydro-9,10-ethanoanthracenyl)methyl]-1-benzylpiperazin.

**18.** Das Verfahren nach Anspruch 17, worin diese Produktverbindung ausgewählt ist aus 4-[(9,10-Dihydro-9,10-ethanoanthracenyl)methyl]-1-methylpiperazin; 4-[(9,10-Dihydro-9,10-ethanoanthracenyl)methyl]-1-(2-hydroxyethyl)-piperazin und 4-[(9,10-Dihydro-9,10-ethanoanthracenyl)methyl]-1-benzylpiperazin.

**Revendications**

**Revendications pour les Etats contractants suivants : AT, BE, CH, DE, DK, FR, GB, IT, LI, LU, NL, SE**

**1.** Un compose de formule

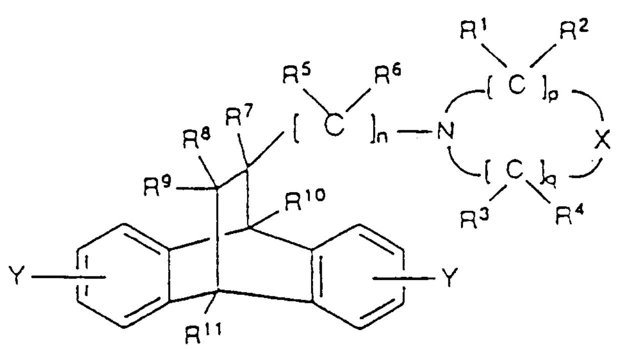

dans laquelle chacun des $R^1$ à $R^6$ est choisi indépendamment parmi les suivants : hydrido, $C_1$-$C_{20}$-alkyle, $C_3$-$C_{10}$-cycloalkyle, $C_3$-$C_{10}$-cycloalkyl-$C_1$-$C_{20}$-alkyle, phényl-$C_1$-$C_{20}$-alkyle, phényle, $C_1$-$C_{10}$-alcoxyalkyle, $C_1$-$C_{20}$-hydroxyalkyle et halo ; dans laquelle $R^1$ et $R^2$ peuvent être pris ensemble pour former un coupe oxo ; dans laquelle $R^3$ et $R^4$ peuvent être pris ensemble pour former un groupe oxo ; dans laquelle $R^5$ et $R^6$ peuvent être pris ensemble pour former un groupe oxo ; dans laquelle chacun des $R^7$ à $R^{11}$ est choisi indépendamment parmi les suivants : hydrido, $C_1$-$C_{20}$-alkyle, hydroxy, $C_3$-$C_{10}$-cycloalkyle, $C_3$-$C_{10}$-cycloalkyl-$C_1$-$C_{20}$-alkyle, phenyl-$C_1$-$C_{20}$-alkyle, phényle, $C_1$-$C_{10}$-alcoxy, $C_1$-$C_{10}$-alcoxyalkyle, phényloxy, phényl-$C_1$-$C_{10}$-alcoxy, $C_1$-$C_{20}$-hydroxyalkyle, halo et $C_1$-$C_{20}$-haloalkyle ; dans laquelle $R^8$ et $R^9$ peuvent être pris ensemble pour former un groupe oxo ; dans laquelle n est un nombre choisi de 0 à 5 inclus ; dans laquelle chacun des p et q est un nombre choisi de 1 à 4, inclus ; dans laquelle X est choisi parmi O, S, $>$N-$R^{12}$, $>$SO et $>SO_2$ ; dans laquelle $R^{12}$ peut être choisi parmi les suivants : hydrido, $C_1$-$C_{20}$-alkyle, $C_3$-$C_{10}$-cycloalkyle, $C_3$-$C_{10}$-cycloalkyl-$C_1$-$C_{20}$-alkyle, phényle, phényl-$C_1$-$C_{20}$-alkyle, un système de noyau aromatique contenant un ou deux hétéroatomes choisis parmi l'oxygène, l'azote, le soufre dans un système de noyau ayant 5 ou 6 chaînons, $C_1$-$C_{10}$-

alcoxyalkyle, $C_1$-$C_{20}$-hydroxyalkyle, alcanoyle, aralcanoyle, aroyle, amino-$C_1$-$C_{20}$-alkyle, mono-$C_1$-$C_{20}$-alkylamino-$C_1$-$C_{20}$-alkyle et di-$C_1$-$C_{20}$-alkylamino-$C_1$-$C_{20}$-alkyle ; dans laquelle $R^{12}$ ensemble avec l'un des $R^1$ à $R^4$ peut former un noyau hétérocyclique condensé contenant 5 à 8 chaînons ; dans laquelle chaque Y désire indépendamment un ou plusieurs groupes choisis parmi les suivants : hydrido, hydroxy, $C_1$-$C_{20}$-alkyle, $C_3$-$C_{10}$-cycloalkyle, $C_3$-$C_{10}$-cycloalkyl$C_1$-$C_{20}$-alkyle, phényl-$C_1$-$C_{20}$-alkyle, phényle, $C_1$-$C_{10}$-alcoxy, phényl-$C_1$-$C_{10}$-alcoxy, phényloxy, $C_1$-$C_{10}$-alcoxyalkyle, $C_1$-$C_{20}$-haloalkyle, $C_1$-$C_{20}$-hydroxyalkyle, halo, cyano, amino, mono-$C_1$-$C_{20}$-alkylamino, di-$C_1$-$C_{20}$-alkylamino, nitro, carboxy, carboxy-$C_1$-$C_{20}$-alkyle et alcanoyle ; ou un sel pharmaceutiquement acceptable dudit composé ; à la condition que lorsque X est $>NR^{12}$ et que chacun des p et q est égal à 2 et que chacun des $R^1$ à $R^4$ est un groupe hydrido et que chacun des $R^7$ à $R^{11}$ est un groupe hydrido, et que $R^{12}$ est choisi dans le groupe suivant : hydrido, méthyle, hydroxyéthyle et acétoxy, alors $R^5$ ensemble avec $R^6$ ne peut pas être un groupe oxo et au moins l'un des $R^5$ et $R^6$ doit être autre que le groupe hydrido ; et à la condition encore que lorsque X est un atome d'oxygène et que chacun des p et q est égal à 2, alors les sélections suivantes ne peuvent pas être faites :

- $R^5$ et $R^6$ ensemble étant un groupe oxo lorsque chacun des $R^1$ à $R^4$, $R^7$ à $R^{11}$ et Y est un groupe hydrido ;
- chacun des $R^1$ à $R^{11}$ et Y étant un groupe hydrido ;
- $R^8$ et $R^9$ ensemble étant un groupe oxo lorsque chacun des $R^1$ à $R^4$, $R^7$ à $R^{11}$ et Y est un groupe hydrido;
- $R^8$ et $R^9$ ensemble étant un groupe oxo avec chacun des $R^{10}$ et $R^{11}$ est un méthyle lorsque chacun des $R^1$ à $R^4$, $R^7$ à $R^9$ et Y est un groupe hydrido.

**2.** Composé selon la revendication 1, selon laquelle chacun des $R^1$ à $R^6$ est choisi indépendamment parmi les suivants : hydrido, $C_1$-$C_{20}$-alkyle, $C_3$-$C_{10}$-cycloalkyle, $C_3$-$C_{10}$-cycloalkyl-$C_1$-$C_{20}$-alkyle, phén-$C_1$-$C_{20}$-alkyle, phényle, $C_1$-$C_{10}$-alcoxyalkyle, $C_1$-$C_{20}$-hydroxyalkyle et halo ; dans laquelle $R^5$ et $R^6$ peuvent être pris ensemble pour former un groupe oxo ; dans laquelle chacun de $R^7$ à $R^{11}$ est choisi indépendamment parmi les suivants : hydrido, $C_1$-$C_{20}$-alkyle, $C_1$-$C_{20}$-hydroxy, $C_3$-$C_{10}$-cycloalkyle, $C_3$-$C_{10}$-cycloalkyl-$C_1$-$C_{20}$-alkyle, phén-$C_1$-$C_{20}$-alkyle, phényle, $C_1$-$C_{10}$-alcoxy, $C_1$-$C_{10}$-alcoxyalkyle, phénoxy, phényl-$C_1$-$C_{10}$-alcoxy, $C_1$-$C_{20}$-hydroxyalkyle, halo et $C_1$-$C_{20}$-haloalkyle ; dans laquelle $R^8$ et $R^9$ peuvent être pris ensemble pour former un groupe oxo ; dans laquelle n est un nombre choisi de 0 à 5, inclus ; dans laquelle chacun des p et q est un élément choisi de 1 à 4 inclus ; dans laquelle X est choisi parmi O, S et N-$R^{12}$; dans laquelle $R^{12}$ peut être choisi parmi les suivants : hydrido, $C_1$-$C_{20}$-alkyle, $C_3$-$C_{10}$-cycloalkyle, $C_3$-$C_{10}$-cycloalkyl-$C_1$-$C_{20}$-alkyle, phényle, phén-$C_1$-$C_{20}$-alkyle, un système de noyau aromatique contenant un ou deux hétéroatomes choisis parmi l'oxygène, l'azote, le soufre dans un système à noyau ayant 5 ou 6 chaînons, $C_1$-$C_{10}$-alcoxyalkyle, $C_1$-$C_{20}$-hydroxyalkyle, alcanoyle, phénalcanoyle, aroyle, amino-$C_1$-$C_{20}$-alkyle, mono-$C_1$-$C_{20}$-alkylamino-$C_1$-$C_{20}$-alkyle et di-$C_1$-$C_{20}$-alkylamino-$C_1$-$C_{20}$-alkyle ; dans laquelle $R^{12}$ ensemble avec l'un des $R^1$ à $R^4$ forme un noyau hétérocyclique condensé contenant 5 à 8 chaînons ; dans laquelle chaque Y est indépendamment un ou plusieurs groupes choisis parmi les suivants : hydrido, hydroxy, $C_1$-$C_{20}$-alkyle, $C_3$-$C_{10}$-cycloalkyle, $C_3$-$C_{10}$-cycloalkyl-$C_1$-$C_{20}$-alkyle, phén-$C_1$-$C_{20}$-alkyle, phényle, $C_1$-$C_{10}$-alcoxy, phén-$C_1$-$C_{10}$-alcoxy, phénoxy, $C_1$-$C_{10}$-alcoxyalkyle, $C_1$-$C_{20}$-haloalkyle, $C_1$-$C_{20}$-hydroxyalkyle, halo, cyano, amino, mono-$C_1$-$C_{20}$-alkylamino, di-$C_1$-$C_{20}$-alkylamino, carboxy, carboxy-$C_1$-$C_{20}$-alkyle et alcanoyle ; ou un sel pharmaceutiquement acceptable dudit composé.

**3.** Composé selon la revendication 2, selon laquelle chacun des $R^1$ à $R^4$ est indépendamment choisi parmi les suivants : hydrido, $C_1$-$C_5$-alkyle, cycloalkyle de 3 à 8 atomes de carbone, cycloalkylalkyle de 4 à 8 atomes de carbone, phényl-$C_1$-$C_5$-alkyle, phényle, $C_1$-$C_5$-alcoxy-$C_1$-$C_5$-alkyle, hydroxy-$C_1$-$C_5$-alkyle et halo ; dans laquelle chacun des $R^5$ et $R^6$ est choisi indépendamment parmi les suivants : hydrido, $C_1$-$C_5$-alkyle, phényl-$C_1$-$C_5$-alkyle, phényle et $C_1$-$C_5$-alcoxy-$C_1$-$C_5$-alkyle ; dans laquelle $R^5$ et $R^6$ peuvent être pris ensemble pour former un groupe oxo ; dans laquelle chacun des $R^7$ à $R^{11}$ est choisi indépendamment parmi les suivants : hydrido : $C_1$-$C_5$-alkyle, hydroxy, cycloalkyle de 3 à 8 atomes de carbone, cycloalkylalkyle de 4 à 8 atomes de carbone, phényl-$C_1$-$C_5$-alkyle, phényle, $C_1$-$C_5$-alcoxy, $C_1$-$C_5$-alcoxy-$C_1$-$C_5$-alkyle, phénoxy, phényl-$C_1$-$C_{10}$-alcoxy, hydroxy-$C_1$-$C_5$-alkyle, halo et halo-$C_1$-$C_5$-alkyle ; dans laquelle $R^8$ et $R^9$ peuvent être pris ensemble pour former un groupe oxo; dans laquelle n est un nombre choisi de 0 à 5 inclus ; dans laquelle chacun des p et q est égal à 2 ou 3 ; dans laquelle X est choisi parmi O, S, $>N$-$R^{12}$ ; dans laquelle $R^{12}$ peut être choisi parmi les suivants : hydrido, $C_1$-$C_5$-alkyle, cycloalkyle de 3 à 8 atomes de carbone, cycloalkylalkyle de 4 à 8 atomes de carbone, phényle, phényl-$C_1$-$C_5$-alkyle, $C_1$-$C_5$-alcoxy-$C_1$-$C_5$-alkyle, hydroxy-$C_1$-$C_5$-alkyle, alcanoyle in-

férieur et hétéroaryle, choisi parmi les noyaux hétérocycliques saturés, partiellement insaturés et totalement insaturés contenant 5 à 7 chaînons dont 1 ou 2 chaînons sont choisis parmi l'atome d'oxygène et l'atome d'azote ; dans laquelle $R^{12}$ ensemble avec l'un des $R^1$ à $R^4$ peut former un noyau héterocyclique condensé contenant 5 ou 6 chaînons ; dans laquelle chaque Y désigne indépendamment un ou plusieurs groupes choisis parmi les suivants : hydrido, hydroxy, $C_1$-$C_5$-alkyle, cycloalkyle de 3 à 8 atomes de carbone, cycloalkylalkyle de 4 à 8 atomes de carbone, phényl-$C_1$-$C_5$-alkyle, phényle, $C_1$-$C_5$-alcoxy, phénoxy, $C_1$-$C_5$-alcoxy-$C_1$-$C_5$-alkyle, halo-$C_1$-$C_5$-alkyle, hydroxy-$C_1$-$C_5$-alkyle, halo, carboxy, carboxy-$C_1$-$C_5$-alkyle et alcanoyle inférieur; ou un sel pharmaceutiquement acceptable dudit composé.

**4.** Composé selon la revendication 3 de formule

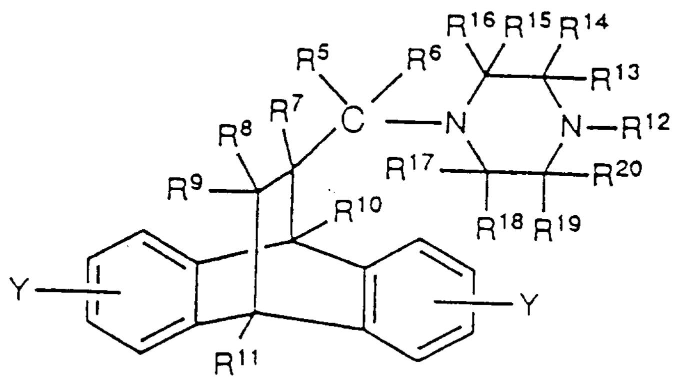

dans laquelle chacun des $R^5$ et $R^6$ est choisi indépendamment parmi les suivants : hydrido, $C_1$-$C_5$-alkyle, benzyle et phényle; dans laquelle chacun des $R^7$ à $R^{11}$ est choisi indépendamment parmi les suivnats : hydrido, $C_1$-$C_5$-alkyle, hydroxy, benzyle, phényle, $C_1$-$C_5$-alcoxy, phénoxy, benzyloxy, halo et halo-$C_1$-$C_5$-alkyle ; dans laquelle $R^{12}$ peut être choisi parmi les suivants : hydrido, $C_1$-$C_5$-alkyle, cycloalkyle de 5 à 6 atomes de carbone, cycloalkylalkyle de 6 à 7 atomes de carbone, phényle, benzyle, hydroxy-$C_1$-$C_5$-alkyle et hétéroalkyle choisi parmi les noyaux hétérocycliques saturés ou totalement insaturés contenant 5 à 7 chaînons dont 1 ou 2 chaînons sont des atomes d'azote ; dans laquelle chaque Y est indépendamment un ou plusieurs groupes choisis parmi les suivants : hydrido, hydroxy, $C_1$-$C_5$-alkyle, benzyle, phényle, $C_1$-$C_5$-alcoxy, phénoxy, halo-$C_1$-$C_5$-alkyle, halo et alcanoyle inférieur; et dans laquelle chacun des $R^{13}$ à $R^{20}$ est choisi indépendamment parmi les suivants : hydrido, $C_1$-$C_5$-alkyle, benzyle, phényle et halo; dans laquelle $R^{12}$ ensemble avec l'un des $R^{13}$, $R^{14}$, $R^{19}$ ou $R^{20}$peut former un noyau hétérocyclique condensé contenant 5 ou 6 chaînons ; ou un sel pharmaceutiquement acceptable dudit compose.

**5.** Composé selon la revendication 4, selon laquelle chacun des $R^5$ et $R^6$ est indépendamment un groupe hydrido ou méthyle ; dans laquelle chacun des $R^7$ à $R^{11}$ est choisi indépendamment parmi les suivants : hydrido, méthyle, éthyle, hydroxy, méthoxy, halo et trihalométhyle ; dans laquelle $R^{12}$ est choisi parmi les suivants : hydrido, méthyle, éthyle, hydroxyéthyle, benzyle, pyridyle et pyrimidyle; dans laquelle chacun des $R^{13}$ à $R^{20}$ est choisi indépendamment parmi les suivants : hydrido, méthyle et éthyle ; dans laquelle $R^{12}$ ensemble avec l'un des $R^{13}$, $R^{14}$, $R^{19}$ ou $R^{20}$ peut former un noyau hétérocyclique condensé contenant 5 ou 6 chaînons ; et dans laquelle chaque Y désigne un ou plusieurs groupes choisis parmi les suivants : hydrido, méthyle, éthyle, hydroxy, méthoxy, trihalométhyle et halo.

**6.** Composé selon la revendication 5 choisi parmi les suivants : 4-[(9,10-dihydro-9,10-éthanoanthracényl)-méthyl]-1-(2-pyrimidyl)pipérazine ; 4-[(9,10-dihydro-9,10-éthanoanthracényl)méthyl]-1,4-diazabicyclo-[4.3.0]-nonane ; et 4-[(9,10-dihydro-9,10-éthanoanthracényl)-méthyl]-1-benzylpipérazine.

**7.** Une composition pharmaceutique comprenant une quantité thérapeutique efficace d'un composé actif et un support ou diluant pharmaceutiquement acceptable, ledit composé actif étant choisi dans une famille de composés selon l'une quelconque des revendications 1, 2, 3, 4, 5 ou 6.

8. Utilisation d'une quantité thérapeutique efficace d'un composé selon l'une quelconque des revendications 1, 2, 4, 5 ou 6 pour la préparation d'un médicament pour le traitement d'un désordre du système nerveux central.

9. Utilisation selon la revendication 8, selon laquelle ledit désordre du système nerveux central est choisi parmi un désordre psychotique, un désordre convulsif et un désordre dystonique.

10. Utilisation selon la revendication 9, selon laquelle ledit désordre du système nerveux central est un désordre psychotique.

11. Un procédé de préparation d'un composé de formule :

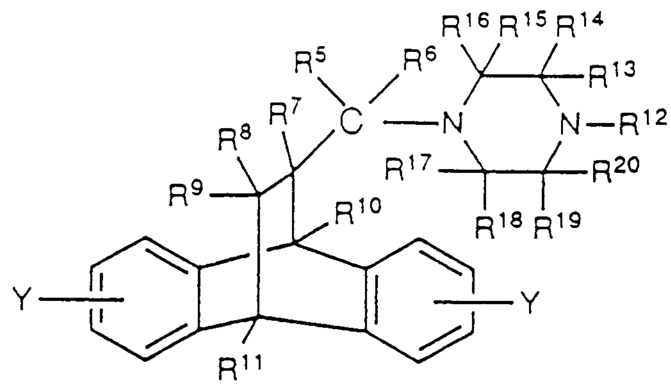

dans laquelle chacun des $R^5$ et $R^6$ est choisi indépendamment parmi les suivants : hydrido, $C_1$-$C_5$-alkyle, benzyle et phényle ; dans laquelle chacun des $R^7$ à $R^{11}$ est choisi indépendamment parmi les suivants : hydrido, $C_1$-$C_5$-alkyle, hydroxy, benzyle, phényle, $C_1$-$C_5$-alcoxy, phénoxy, benzyloxy, halo et halo-$C_1$-$C_5$-alkyle; dans laquelle $R^{12}$ peut être choisi parmi les suivants : hydrido, $C_1$-$C_5$-alkyle, cycloalkyle de 5 ou 6 atomes de carbone, cycloalkylalkyle de 6 ou 7 atomes de carbone, phényle, benzyle, hydroxy-$C_1$-$C_5$-alkyle et hétéroaryle choisi parmi les noyaux hétérocycliques saturés ou totalement insaturés contenant 5 à 7 chaînons dont 1 ou 2 chaînons sont des atomes d'azote ; dans laquelle chaque Y est indépendamment un ou plusieurs groupes choisis parmi les suivants : hydrido, hydroxy, $C_1$-$C_5$-alkyle, benzyle, phényle, $C_1$-$C_5$-alcoxy, phénoxy, halo-$C_1$-$C_5$-alkyle, halo et alcanoyle inférieur; et dans laquelle chacun des $R^{13}$ à $R^{20}$ est choisi indépendamment parmi les suivants : hydrido, $C_1$-$C_5$-alkyle, benzyle, phényle et halo ; dans laquelle $R^{12}$ ensemble avec l'un des $R^{13}$, $R^{14}$, $R^{19}$ et $R^{20}$ peut former un noyau hétérocyclique condensé contenant 5 ou 6 chaînons ; ou un sel pharmaceutiquement acceptable dudit composé;
ladite méthode comprenant la réaction d'un composé de formule :

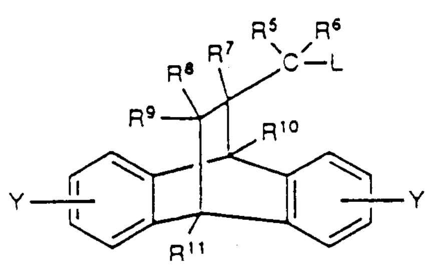

dans laquelle chacun des $R^5$ à $R^{11}$ et Y est défini comme précédemment ; et dans laquelle L est choisi parmi les suivants : halo, hydroxy, para-toluènesulfonyloxy,méthylsulfonyloxy et para-bromotoluènesulfonyloxy ; avec une amine de formule :

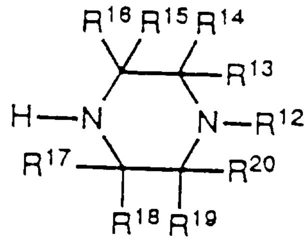

dans laquelle chacun des $R^{12}$ à $R^{20}$ est comme défini précédemment.

**12.** Le procédé selon la revendication 11, selon laquelle chacun des $R^5$ et $R^6$ désignent indépendamment un groupe hydrido ou méthyle ; dans laquelle chacun des $R^7$ à $R^{11}$ est choisi indépendamment parmi les suivants : hydrido, méthyle, éthyle, hydroxy, méthoxy, halo et trihalométhyle ; dans laquelle $R^{12}$ est choisi parmi les suivants : hydrido, méthyle, éthyle, hydroxyéthyle, benzyle, pyridyle et pyrimidyle ; dans laquelle chacun des $R^{13}$ à $R^{20}$ est choisi indépendamment parmi les suivants : hydrido, méthyle, éthyle et trihalométhyle ; dans laquelle $R^{12}$ ensemble avec l'un des $R^{13}$, $R^{14}$, $R^{19}$ ou $R^{20}$ peut former un noyau hétérocyclique condensé contenant 5 ou 6 chaînons ; dans laquelle chaque Y est indépendamment un ou plusieurs groupes choisis parmi les suivants : hydrido, méthyle, éthyle, hydroxy, méthoxy, trihalométhyle et halo ; et dans laquelle L est choisi parmi les groupes halo et hydroxy.

**13.** Le procédé selon la revendication 12, selon laquelle L est un groupe halo.

**14.** Le procédé selon la revendication 13, selon laquelle L est un groupe chloro ou bromo.

**15.** Le procédé selon la revendication 12, selon laquelle L est un groupe hydroxy.

**16.** Le procédé selon la revendication 11, selon laquelle un composé de formule :

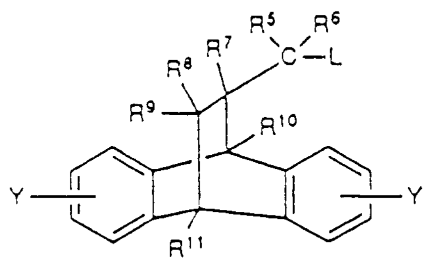

est préparé en mélangeant un alcool de formule :

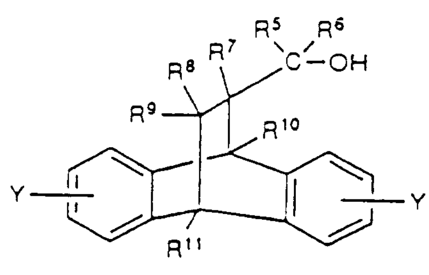

dans laquelle chacun des $R^5$ à $R^{11}$ et L est comme défini précédemment, avec un réactif choisi parmi le chlorure de thionyle, l'oxychlorure de phosphore, le dibromure de triphénylphosphine, le chlorure de méthanesulfonyle et le chlorure de p-toluènesulfonyle.

**17.** Le procédé selon la revendication 11, selon laquelle le produit est choisi parmi les suivants : 4-[(9,10-dihydro-9,10-éthanoanthracényl)méthyl]-1-méthylpipérazine ; 4-[(9,10-dihydro-9,10-éthanoanthracényl)-

méthyl]-1-(2-hydroxyéthyl)-pipérazine ; 4-[(9,10-dihydro-9,10-éthanoanthracényl)méthyl]-1-(2-pyrimidyl)-pipérazine ; 4-[(9,10-dihydro-9,10-éthanoanthracényl)méthyl]-1,4-diazabicyclo[4.3.0]-nonane ; et 4-[-(9,10-dihydro-9,10-éthanoanthracényl)méthyl]-1-benzylpipérazine.

**18.** Le procédé selon la revendication 17, selon laquelle ledit produit est choisi parmi les suivants : 4-[-(9,10-dihydro-9,10-éthanoanthracényl)méthyl]-1-méthylpipérazine ; 4-[(9,10-dihydro-9,10-éthanoanthracényl)méthyl]-1-(2-hydroxyéthyl)-pipérazine ; et 4-[(9,10-dihydro-9,10-éthanoanthracényl)méthyl]-1-benzylpipérazine.

**Revendications pour l'Etat contractant suivant : ES**

**1.** Un procédé de préparation d'un composé de formule

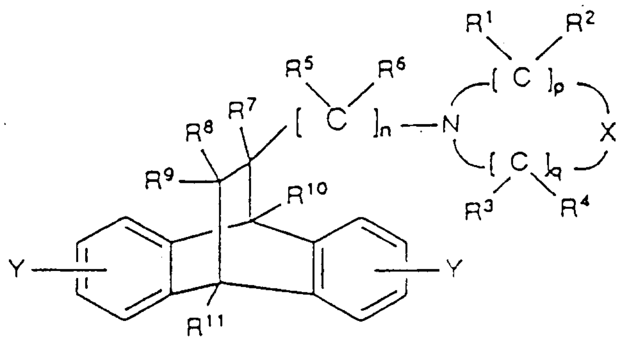

dans laquelle chacun des $R^1$ à $R^6$ est choisi indépendamment parmi les suivants : hydrido, $C_1$-$C_{20}$-alkyle, $C_3$-$C_{10}$-cycloalkyle, $C_3$-$C_{10}$-cycloalkyle-$C_1$-$C_{20}$-alkyle, phényl-$C_1$-$C_{20}$-alkyle, phényle, $C_1$-$C_{10}$-alcoxyalkyle, $C_1$-$C_{20}$-hydroxyalkyle et halo ; dans laquelle $R^1$ et $R^2$ peuvent être pris ensemble pour former un groupe oxo ; dans laquelle $R^3$ et $R^4$ peuvent être pris ensemble pour former un groupe oxo ; dans laquelle $R^5$ et $R^6$ peuvent être pris ensemble pour former un groupe oxo ; dans laquelle chacun des $R^7$ à $R^{11}$ est choisi indépendamment parmi les suivants : hydrido, $C_1$-$C_{20}$-alkyle, hydroxy, $C_3$-$C_{10}$-cycloalkyle, $C_3$-$C_{10}$-cycloalkyl-$C_1$-$C_{20}$-alkyle, phényl-$C_1$-$C_{20}$-alkyle, phényle, $C_1$-$C_{10}$-alcoxy, $C_1$-$C_{10}$-alcoxyalkyle, phényloxy, phényl-$C_1$-$C_{10}$-alcoxy, $C_1$-$C_{20}$-hydroxyalkyle, halo et $C_1$-$C_{20}$-haloalkyle ; dans laquelle $R^8$ et $R^9$ peuvent être pris ensemble pour former un groupe oxo ; dans laquelle n est un nombre choisi de 0 à 5 inclus ; dans laquelle chacun des p et q est un nombre choisi de 1 à 4, inclus ; dans laquelle X est choisi parmi O, S, $>$N-$R^{12}$, $>$SO et $>SO_2$ ; dans laquelle $R^{12}$ peut être choisi parmi les suivants : hydrido, $C_1$-$C_{20}$-alkyle, $C_3$-$C_{10}$-cycloalkyle, $C_3$-$C_{10}$-cycloalkyl-$C_1$-$C_{20}$-alkyle, phényle, phényl-$C_1$-$C_{20}$-alkyle, un système de noyau aromatique contenant un ou deux hétéroatomes choisis parmi l'oxygène, l'azote, le soufre dans un système de noyau ayant 5 ou 6 chaînons, $C_1$-$C_{10}$-alcoxyalkyle, $C_1$-$C_{20}$-hydroxyalkyle, alcanoyle, aralcanoyle, aroyle, amino-$C_1$-$C_{20}$-alkyle, mono-$C_1$-$C_{20}$-alkylamino-$C_1$-$C_{20}$-alkyle et di-$C_1$-$C_{20}$-alkylamino-$C_1$-$C_{20}$-alkyle ; dans laquelle $R^{12}$ ensemble avec l'un des $R^1$ à $R^4$ peut former un noyau hétérocyclique condensé contenant 5 à 8 chaînons ; dans laquelle chaque Y désigne indépendamment un ou plusieurs groupes choisis parmi les suivants : hydrido, hydroxy, $C_1$-$C_{20}$-alkyle, $C_3$-$C_{10}$-cycloalkyle, $C_3$-$C_{10}$-cycloalkyl-$C_1$-$C_{20}$-alkyle, phényl-$C_1$-$C_{20}$-alkyle, phényle, $C_1$-$C_{10}$-alcoxy, phényl-$C_1$-$C_{10}$-alcoxy, phényloxy, $C_1$-$C_{10}$-alcoxyalkyle, $C_1$-$C_{20}$-haloalkyle, $C_1$-$C_{20}$-hydroxyalkyle, halo, cyano, amino, mono-$C_1$-$C_{20}$-alkylamino, di-$C_1$-$C_{20}$-alkylamino, nitro, carboxy, carboxy-$C_1$-$C_{20}$-alkyle et alcanoyle ; ou un sel pharmaceutiquement acceptable dudit composé ; à la condition que lorsque X est $>NR^{12}$ et que chacun des p et q est égal à 2 et que chacun des $R^1$ à $R^4$ est un groupe hydrido et que chacun des $R^7$ à $R^{11}$ est un groupe hydrido, et que $R^{12}$ est choisi dans le groupe suivant : hydrido, méthyle, hydroxyéthyle et acétoxy, alors $R^5$ ensemble avec $R^6$ ne peut pas être un groupe oxo et au moins l'un des $R^5$ et $R^6$ doit être autre que le groupe hydrido; et à la condition encore que lorsque X est un atome d'oxygène et que chacun des p et q est égal à 2, alors les sélections suivantes ne peuvent pas être faites :

- $R^5$ et $R^6$ ensemble étant un groupe oxo lorsque chacun des $R^1$ à $R^4$, $R^7$ à $R^{11}$ et Y est un groupe hydrido ;
- chacun des $R^1$ à $R^{11}$ et Y étant un groupe hydrido ;

- $R^8$ et $R^9$ ensemble étant un groupe oxo lorsque chacun des $R^1$ à $R^4$, $R^7$ à $R^{11}$ et Y est un groupe hydrido;
- $R^8$ et $R^9$ ensemble étant un groupe oxo avec chacun des $R^{10}$ et $R^{11}$ est un méthyle lorsque chacun des $R^1$ à $R^4$, $R^7$ à $R^9$ et Y est un groupe hydrido,

caractérisé en ce qu'un composé de formule 3

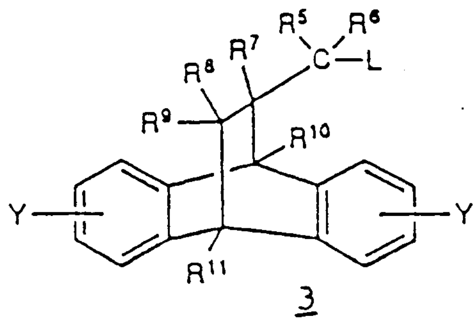

3

dans laquelle chacun des $R^5$ à $R^{11}$ et Y est comme défini ci-dessus ; et dans laquelle R est choisi parmi les suivants : halo, hydroxy, para-toluènesulfonyloxy,méthylsulfonyloxy et para-bromotoluènesulfonyloxy,

est mis à réagir avec un composé de formule 5

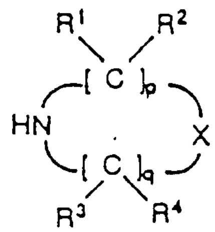

5

dans laquelle p, q, X et $R^1$ à $R^4$ sont comme définis précédemment.

**2.** Un procédé selon la revendication 1, selon laquelle chacun des $R^1$ à $R^6$ du composé préparé est choisi indépendamment parmi les suivants : hydrido, $C_1$-$C_{20}$-alkyle, $C_3$-$C_{10}$-cycloalkyle, $C_3$-$C_{10}$-cycloalkyl-$C_1$-$C_{20}$-alkyle, phén-$C_1$-$C_{20}$-alkyle, phényle, $C_1$-$C_{10}$-alcoxyalkyle, $C_1$-$C_{20}$-hydroxyalkyle et halo; dans laquelle $R^5$ et $R^6$ peuvent être pris ensemble pour former un groupe oxo ; dans laquelle chacun de $R^7$ à $R^{11}$ est choisi indépendamment parmi les suivants : hydrido, $C_1$-$C_{20}$-alkyle, $C_1$-$C_{10}$-hydroxy, $C_3$-$C_{10}$-cycloalkyle, $C_3$-$C_{10}$-cycloalkyl-$C_1$-$C_{20}$-alkyle, phén-$C_1$-$C_{20}$-alkyle, phényle, $C_1$-$C_{10}$-alcoxy, $C_1$-$C_{10}$-alcoxyalkyle, phénoxy, phényl-$C_1$-$C_{10}$-alcoxy, $C_1$-$C_{20}$-hydroxyalkyle, halo et $C_1$-$C_{20}$-haloalkyle ; dans laquelle $R^8$ et $R^9$ peuvent être pris ensemble pour former un groupe oxo ; dans laquelle n est un nombre choisi de 0 à 5, inclus ; dans laquelle chacun des p et q est un élément choisi de 1 à 4 inclus ; dans laquelle X est chois parmi O, S et N-$R^{12}$ ; dans laquelle $R^{12}$ peut être choisi parmi les suivants : hydrido, $C_1$-$C_{20}$-alkyle, $C_3$-$C_{10}$-cycloalkyle, $C_3$-$C_{10}$-cycloalkyl-$C_1$-$C_{20}$-alkyle, phényle, phén-$C_1$-$C_{20}$-alkyle, un système de noyau aromatique contenant un ou deux hétéroatomes choisis parmi l'oxygène, l'azote, le soufre dans un système à noyau ayant 5 ou 6 chaînons, $C_1$-$C_{10}$-alcoxyalkyle, $C_1$-$C_{20}$-hydroxyalkyle, alcanoyle, phénalcanoyle, aroyle, amino-$C_1$-$C_{20}$-alkyle, mono-$C_1$-$C_{20}$-alkylamino-$C_1$-$C_{20}$-alkyle et di-$C_1$-$C_{20}$-alkylamino-$C_1$-$C_{20}$-alkyle ; dans laquelle $R^{12}$ ensemble avec l'un des $R^1$ à $R^4$ forme un noyau hétérocyclique condensé contenant 5 à 8 chaînons ; dans laquelle chaque Y est indépendamment un ou plusieurs groupes choisis parmi les suivants : hydrido, hydroxy, $C_1$-$C_{20}$-alkyle, $C_3$-$C_{10}$-cycloalkyle, $C_3$-$C_{10}$-cycloalkyl-$C_1$-$C_{20}$-alkyle, phén-$C_1$-$C_{20}$-alkyle, phényle, $C_1$-$C_{10}$-alcoxy, phén-$C_1$-$C_{10}$-alcoxy, phénoxy, $C_1$-$C_{10}$-alcoxyalkyle, $C_1$-$C_{20}$-haloalkyle, $C_1$-$C_{20}$-hydroxyalkyle, halo, cyano, amino, mono-$C_1$-$C_{20}$-alkylamino, di-$C_1$-$C_{20}$-alkylamino, carboxy, carboxy-$C_1$-$C_{20}$-alkyle et alcanoyle ; ou un sel pharmaceutiquement acceptable dudit composé.

**3.** Procédé selon la revendication 2, selon laquelle chacun des $R^1$ à $R^4$ du composé préparé est choisi indépendamment parmi les suivants : hydrido, $C_1$-$C_5$-alkyle, cycloalkyle de 3 à 8 atomes de carbone, cycloalkylalkyle de 4 à 8 atomes de carbone, phényl-$C_1$-$C_5$-alkyle, phényle, $C_1$-$C_5$-alcoxy-$C_1$-$C_5$-alkyle, hydroxy-$C_1$-$C_5$-alkyle et halo ; dans laquelle chacun des $R^5$ et $R^6$ est choisi indépendamment parmi les suivants : hydrido, $C_1$-$C_5$-alkyle, phényl-$C_1$-$C_5$-alkyle, phényle et $C_1$-$C_5$-alcoxy-$C_1$-$C_5$-alkyle ; dans laquelle $R^5$ et $R^6$ peuvent être pris ensemble pour former un groupe oxo ; dans laquelle chacun des $R^7$ à $R^{11}$ est choisi indépendamment parmi les suivants : hydrido : $C_1$-$C_5$-alkyle, hydroxy, cycloalkyle de 3 à 8 atomes de carbone, cycloalkylalkyle de 4 à 8 atomes de carbone, phényl-$C_1$-$C_5$-alkyle, phényle, $C_1$-$C_5$-alcoxy, $C_1$-$C_5$-alcoxy-$C_1$-$C_5$-alkyle, phénoxy, phényl-$C_1$-$C_{10}$-alcoxy, hydroxy-$C_1$-$C_5$-alkyle, halo et halo-$C_1$-$C_5$-alkyle ; dans laquelle $R^8$ et $R^9$ peuvent être pris ensemble pour former un groupe oxo ; dans laquelle n est un nombre choisi de 0 à 5 inclu ; dans laquelle chacun des p et q est égal à 2 ou 3 ; dans laquelle X est choisi parmi O, S, $>$N-$R^{12}$ ; dans laquelle $R^{12}$ peut être choisi parmi les suivants : hydrido, $C_1$-$C_5$-alkyle, cycloalkyle de 3 à 8 atomes de carbone, cycloalkylalkyle de 4 à 8 atomes de carbone, phényle, phényl-$C_1$-$C_5$-alkyle, $C_1$-$C_5$-alcoxy-$C_1$-$C_5$-alkyle, hydroxy-$C_1$-$C_5$-alkyle, alcanoyle inférieur et hétéroaryle, choisis parmi les noyaux hétérocycliques saturés, partiellement insaturés et totalement insaturés contenant 5 à 7 chaînons dont 1 ou 2 chaînons sont choisis parmi l'atome d'oxygène et l'atome d'azote ; dans laquelle $R^{12}$ ensemble avec l'un des $R^1$ à $R^4$ peut former un noyau hétérocyclique condensé contenant 5 ou 6 chaînons ; dans laquelle chaque Y désigne indépendamment un ou plusieurs groupes choisis parmi les suivants : hydrido, hydroxy, $C_1$-$C_5$-alkyle, cycloalkyle de 3 à 8 atomes de carbone, cycloalkylalkyle de 4 à 8 atomes de carbone, phényl-$C_1$-$C_5$-alkyle, phényle, $C_1$-$C_5$-alcoxy, phénoxy, $C_1$-$C_5$-alcoxy-$C_1$-$C_5$-alkyle, halo-$C_1$-$C_5$-alkyle, hydroxy-$C_1$-$C_5$-alkyle, halo, carboxy, carboxy-$C_1$-$C_5$-alkyle et alcanoyle inférieur; ou un sel pharmaceutiquement acceptable dudit composé.

**4.** Procédé selon la revendication 3 de la formule

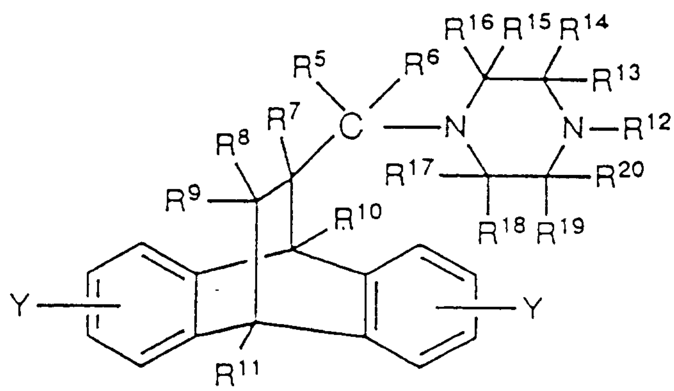

dans laquelle chacun des $R^5$ et $R^6$ est choisi indépendamment parmi les suivants : hydrido, $C_1$-$C_5$-alkyle, benzyle et phényle ; dans laquelle chacun des $R^7$ à $R^{11}$ est choisi indépendamment parmi les suivnats : hydrido, $C_1$-$C_5$-alkyle, hydroxy, benzyle, phényle, $C_1$-$C_5$-alcoxy, phénoxy, benzyloxy, halo et halo-$C_1$-$C_5$-alkyle; dans laquelle $R^{12}$ peut être choisi parmi les suivants : hydrido, $C_1$-$C_5$-alkyle, cycloalkyle de 5 à 6 atomes de carbone, cycloalkylalkyle de 6 à 7 atomes de carbone, phényle, benzyle, hydroxy-$C_1$-$C_5$-alkyle et hétéroalkyle choisis parmi les noyaux hétérocycliques saturés ou totalement insaturés contenant 5 à 7 chaînons dont 1 ou 2 chaînons sont des atomes d'azote ; dans laquelle chaque Y est indépendamment un ou plusieurs groupes choisis parmi les suivants : hydrido, hydroxy, $C_1$-$C_5$-alkyle, benzyle, phényle, $C_1$-$C_5$-alcoxy, phénoxy, halo-$C_1$-$C_5$-alkyle, halo et alcanoyle inférieur; et dans laquelle chacun des $R^{13}$ à $R^{20}$ est choisi indépendamment parmi les suivants : hydrido, $C_1$-$C_5$-alkyle, benzle, phényle et halo ; dans laquelle $R^{12}$ ensemble avec l'un des $R^{13}$, $R^{14}$, $R^{19}$ ou $R^{20}$ peut former un noyau hétérocyclique condensé contenant 5 ou 6 chaînons ; ou un sel pharmaceutiquement acceptable dudit composé.

**5.** Procédé selon la revendication 4, selon laquelle chacun des $R^5$ et $R^6$ du composé préparé est choisi indépendamment parmi le groupe hydrido ou méthyle ; dans laquelle chacun des $R^7$ à $R^{11}$ est choisi indépendamment parmi les suivants : hydrido, méthyle, éthyle, hydroxy, méthoxy, halo et trihalométhyle ; dans laquelle $R^{12}$ est choisi parmi les suivants : hydrido, méthyle, éthyle, hydroxyéthyle, benzyle,

pyridyle et pyrimidyle; dans laquelle chacun des $R^{13}$ à $R^{20}$ est choisi indépendamment parmi les suivants : hydrido, méthyle et éthyle ; dans laquelle $R^{12}$ ensemble avec l'un des $R^{13}$, $R^{14}$, $R^{19}$ ou $R^{20}$ peut former un noyau hétérocyclique condensé contenant 5 ou 6 chaînons ; et dans laquelle chaque Y désigne un ou plusieurs groupes choisis parmi les suivants : hydrido, méthyle, éthyle, hydroxy, méthoxy, trihalométhyle et halo.

**6.** Un procédé selon la revendication 5, selon laquelle le composé préparé est choisi parmi les suivants : 4-[(9,10-dihydro-9,10-éthanoanthracényl)méthyl]-1-(2-pyrimidyl)pipérazine ; 4-[(9,10-dihydro-9,10-éthanoanthracényl)méthyl]-1,4-diazabicyclo[4.3.0]-nonane ; et 4-[(9,10-dihydro-9,10-éthanoanthracényl)-méthyl]-1-benzylpipérazine.

**7.** Un procédé pour la préparation d'une composition pharmaceutique, caractérisé en ce qu'une quantité thérapeutique efficace d'un composé actif est mélangé à un support ou diluant pharmaceutique acceptable, ledit composé actif est choisi dans une famille de composés selon l'une quelconque des revendications 1, 2, 3, 4, 5 ou 6.

**8.** Utilisation d'une quantité thérapeutique efficace d'un composé selon l'une quelconque des revendications 1, 2, 4, 5 ou 6 pour la préparation d'un médicament pour le traitement d'un désordre du système nerveux central.

**9.** Utilisation selon la revendication 8, selon laquelle ledit désordre du système nerveux central est choisi parmi un désordre psychotique, un désordre convulsif et un désordre dystonique.

**10.** Utilisation selon la revendication 9, selon laquelle ledit désordre du système nerveux central est un désordre psychotique.

**11.** Un procédé de préparation d'un composé de formule :

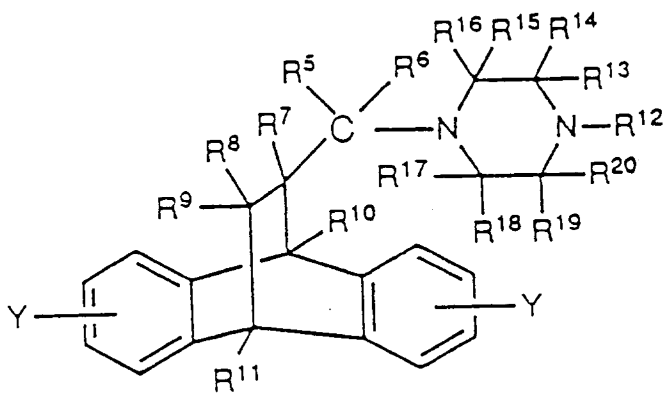

dans laquelle chacun des $R^5$ et $R^6$ est choisi indépendamment parmi les suivants : hydrido, $C_1$-$C_5$-alkyle, benzyle et phényle; dans laquelle chacun des $R^7$ à $R^{11}$ est choisi indépendamment parmi les suivants : hydrido, $C_1$-$C_5$-alkyle, hydroxy, benzyle, phényle, $C_1$-$C_5$-alcoxy, phénoxy, benzyloxy, halo et halo-$C_1$-$C_5$-alkyle; dans laquelle $R^{12}$ peut être choisi parmi les suivants : hydrido, $C_1$-$C_5$-alkyle, cycloalkyle de 5 ou 6 atomes de carbone, cycloalkylalkyle de 6 ou 7 atomes de carbone, phényle, benzyle, hydroxy-$C_1$-$C_5$-alkyle et hétéroaryle choisis parmi les noyaux hétérocycliques saturés ou totalement insaturés contenant 5 à 7 chaînons dont 1 ou 2 chaînons sont des atomes d'azote ; dans laquelle chaque Y est indépendamment un ou plusieurs groupes choisis parmi les suivants : hydrido, hydroxy, $C_1$-$C_5$-alkyle, benzyle, phényle, $C_1$-$C_5$-alcoxy, phénoxy, halo-$C_1$-$C_5$-alkyle, halo et alcanoyle inférieur; et dans laquelle chacun des $R^{13}$ à $R^{20}$ est choisi indépendamment parmi les suivants : hydrido, $C_1$-$C_5$-alkyle, benzyle, phényle et halo ; dans laquelle $R^{12}$ ensemble avec l'un des $R^{13}$, $R^{14}$, $R^{19}$ et $R^{20}$ peut former un noyau hétérocyclique condensé contenant 5 ou 6 chaînons ; ou un sel pharmaceutiquement acceptable dudit composé;
ladite méthode comprenant la réaction d'un composé de formule :

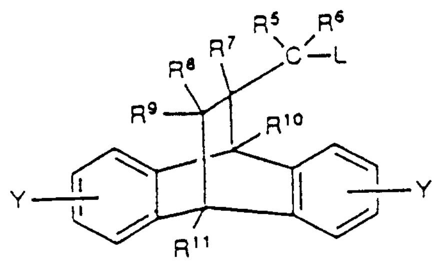

dans laquelle chacun des $R^5$ à $R^{11}$ et Y est défini comme précédemment ; et dans laquelle L est choisi parmi les suivants : halo, hydroxy, para-toluènesulfonyloxy,méthylsulfonyloxy et para-bromotoluènesulfonyloxy ; avec une amine de formule :

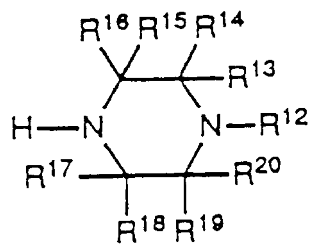

dans laquelle chacun des $R^{12}$ à $R^{20}$ est comme défini précédemment.

**12.** Le procédé selon la revendication 11, selon laquelle chacun des $R^5$ et $R^6$ désignent indépendamment un groupe hydrido ou méthyle ; dans laquelle chacun des $R^7$ à $R^{11}$ est choisi indépendamment parmi les suivants : hydrido, méthyle, éthyle, hydroxy, méthoxy, halo et trihalométhyle ; dans laquelle $R^{12}$ est choisi parmi les suivants : hydrido, méthyle, éthyle, hydroxyéthyle, benzyle, pyridyle et pyrimidyle ; dans laquelle chacun des $R^{13}$ à $R^{20}$ est choisi indépendamment parmi les suivants : hydrido, méthyle, éthyle et trihalométhyle ; dans laquelle $R^{12}$ ensemble avec l'un des $R^{13}$, $R^{14}$, $R^{19}$ ou $R^{20}$ peut former un noyau hétérocyclique condensé contenant 5 ou 6 chaînons ; dans laquelle chaque Y est indépendamment un ou plusieurs groupes choisis parmi les suivants : hydrido, méthyle, éthyle, hydroxy, méthoxy, trihalométhyle et halo; et dans laquelle L est choisi parmi les groupes halo et hydroxy.

**13.** Le procédé selon la revendication 12, selon laquelle L est un groupe halo.

**14.** Le procédé selon la revendication 13, selon laquelle L est un groupe chloro ou bromo.

**15.** Le procédé selon la revendication 12, selon laquelle L est un groupe hydroxy.

**16.** Le procédé selon la revendication 11, selon laquelle un composé de formule :

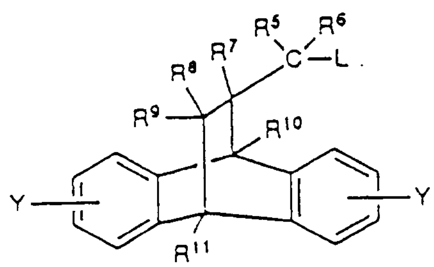

est préparé en mélangeant un alcool de formule :

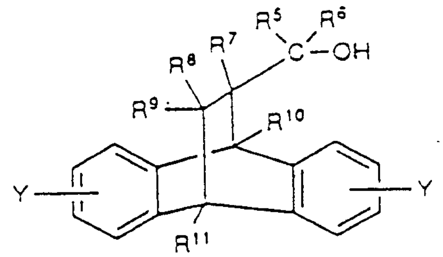

dans laquelle chacun des $R^5$ à $R^{11}$ et L est comme défini précédemment, avec un réactif choisi parmi le chlorure de thionyle, l'oxychlorure de phosphore, le dibromure de triphénylphosphine, le chlorure de méthanesulfonyle et le chlorure de p-toluènesulfonyle.

**17.** Le procédé selon la revendication 11, selon laquelle le produit est choisi parmi les suivants : 4-[(9,10-dihydro-9,10-éthanoanthracényl)méthyl]-1-méthylpipérazine ; 4-[(9,10-dihydro-9,10-éthanoanthracényl)-méthyl]-1-(2-hydroxyéthyl)-pipérazine ; 4-[(9,10-dihydro-9,10-éthanoanthracényl)méthyl]-1-(2-pyrimidyl)-pipérazine ; 4-[(9,10-dihydro-9,10-éthanoanthracényl)méthyl]-1,4-diazabicyclo[4.3.0]-nonane ; et 4-[-(9,10-dihydro-9,10-éthanoanthracényl)méthyl]-1-benzylpipérazine.

**18.** Le procédé selon la revendication 17, selon laquelle ledit produit est choisi parmi les suivants : 4-[-(9,10-dihydro-9,10-éthanoanthracényl)méthyl]-1-méthylpipérazine ; 4-[(9,10-dihydro-9,10-éthanoanthracényl)méthyl]-1-(2-hydroxyéthyl)-pipérazine ; et 4-[(9,10-dihydro-9,10-éthanoanthracényl)méthyl]-1-benzylpipérazine.

**Revendications pour l'Etat contractant suivant : GR**

**1.** Un composé de formule

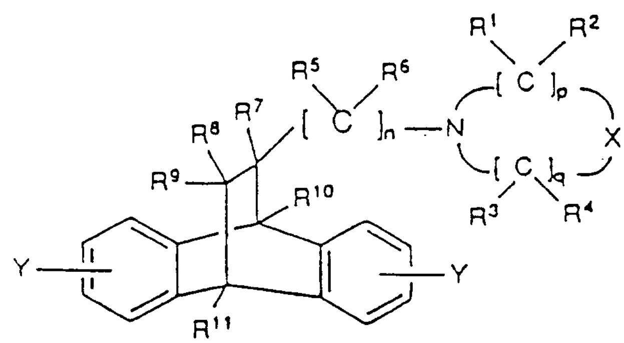

dans laquelle chacun des $R^1$ à $R^6$ est choisi indépendamment parmi les suivants : hydrido, $C_1$-$C_{20}$-alkyle, $C_3$-$C_{10}$-cycloalkyle, $C_3$-$C_{10}$-cycloalkyl-$C_1$-$C_{20}$-alkyle, phényl-$C_1$-$C_{20}$-alkyle, phényle, $C_1$-$C_{10}$-alcoxyalkyle, $C_1$-$C_{20}$-hydroxyalkyle et halo ; dans laquelle $R^1$ et $R^2$ peuvent être pris ensemble pour former un groupe oxo ; dans laquelle $R^3$ et $R^4$ peuvent être pris ensemble pour former un groupe oxo ; dans laquelle $R^5$ et $R^6$ peuvent être pris ensemble pour former un groupe oxo ; dans laquelle chacun des $R^7$ à $R^{11}$ est choisi indépendamment parmi les suivants : hydrido, $C_1$-$C_{20}$-alkyle, hydroxy, $C_3$-$C_{10}$-cycloalkyle, $C_3$-$C_{10}$-cycloalkyl-$C_1$-$C_{20}$-alkyle, phényl-$C_1$-$C_{20}$-alkyle, phényle, $C_1$-$C_{10}$-alcoxy, $C_1$-$C_{10}$-alcoxyalkyle, phényloxy, phényl-$C_1$-$C_{10}$-alcoxy, $C_1$-$C_{20}$-hydroxyalkyle, halo et $C_1$-$C_{20}$-haloalkyle ; dans laquelle $R^8$ et $R^9$ peuvent être pris ensemble pour former un groupe oxo ; dans laquelle n est un nombre choisi de 0 à 5 inclus ; dans laquelle chacun des p et q est un nombre choisi de 1 à 4, inclus ; dans laquelle X est choisi parmi O, S, $>$N-$R^{12}$, $>$SO et $>SO_2$ ; dans laquelle $R^{12}$ peut être choisi parmi les suivants : hydrido, $C_1$-$C_{20}$-alkyle, $C_3$-$C_{10}$-cycloalkyle, $C_3$-$C_{10}$-cycloalkyl-$C_1$-$C_{20}$-alkyle, phényle, phényl-$C_1$-$C_{20}$-alkyle, un système de noyau aromatique contenant un ou deux hétéroatomes choisis parmi l'oxygène, l'azote, le soufre dans un système de noyau ayant 5 ou 6 chaînons, $C_1$-$C_{10}$-alcoxyalkyle, $C_1$-$C_{20}$-hydroxyalkyle, alcanoyle, aralcanoyle, aroyle, amino-$C_1$-$C_{20}$-alkyle, mono-$C_1$-$C_{20}$-alkylamino-$C_1$-$C_{20}$-alkyle et di-$C_1$-$C_{20}$-alkylamino-$C_1$-$C_{20}$-alkyle ; dans laquelle $R^{12}$ ensemble avec l'un

des $R^1$ à $R^4$ peut former un noyau hétérocyclique condensé contenant 5 à 8 chaînons ; dans laquelle chaque Y désigne indépendamment un ou plusieurs groupes choisis parmi les suivants : hydrido, hydroxy, $C_1$-$C_{20}$-alkyle, $C_3$-$C_{10}$-cycloalkyle, $C_3$-$C_{10}$-cycloalkyl-$C_1$-$C_{20}$-alkyle, phényl-$C_1$-$C_{20}$-alkyle, phényle, $C_1$-$C_{10}$-alcoxy, phényl-$C_1$-$C_{10}$-alcoxy, phényloxy, $C_1$-$C_{10}$-alcoxyalkyle, $C_1$-$C_{20}$-haloalkyle, $C_1$-$C_{20}$-hydroxyalkyle, halo, cyano, amino, mono-$C_1$-$C_{20}$-alkylamino, di-$C_1$-$C_{20}$-alkylamino, nitro, carboxy, carboxy-$C_1$-$C_{20}$-alkyle et alcanoyle ; ou un sel pharmaceutiquement acceptable dudit composé ; à la condition que lorsque X est $\rangle NR^{12}$ et que chacun des p et q est égal à 2 et que chacun des $R^1$ à $R^4$ est un groupe hydrido et que chacun des $R^7$ à $R^{11}$ est un groupe hydrido, et que $R^{12}$ est choisi dans le groupe suivant : hydrido, méthyle, hydroxyéthyle et acétoxy, alors $R^5$ ensemble avec $R^6$ ne peut pas être un groupe oxo et au moins l'un des $R^5$ et $R^6$ doit être autre que le groupe hydrido ; et à la condition encore que lorsque X est un atome d'oxygène et que chacun des p et q est égal à 2, alors les sélections suivantes ne peuvent pas être faites :

- $R^5$ et $R^6$ ensemble étant un groupe oxo lorsque chacun des $R^1$ à $R^4$, $R^7$ à $R^{11}$ et Y est un groupe hydrido;
- chacun des $R^1$ à $R^{11}$ et Y étant un groupe hydrido;
- $R^8$ et $R^9$ ensemble étant un groupe oxo lorsque chacun des $R^1$ à $R^4$, $R^7$ à $R^{11}$ et Y est un groupe hydrido;
- $R^8$ et $R^9$ ensemble étant un groupe oxo avec chacun des $R^{10}$ et $R^{11}$ est un méthyle lorsque chacun des $R^1$ à $R^4$, $R^7$ à $R^9$ et Y est un groupe hydrido.

**2.** Composé selon la revendication 1, selon laquelle chacun des $R^1$ à $R^6$ est choisi indépendamment parmi les suivants : hydrido, $C_1$-$C_{20}$-alkyle, $C_3$-$C_{10}$-cycloalkyle, $C_3$-$C_{10}$-cycloalkyle, $C_1$-$C_{20}$-alkyle, phén-$C_1$-$C_{20}$-alkyle, phényle, $C_1$-$C_{10}$-alcoxyalkyl-$C_1$-$C_{20}$-hydroxyalkyle et halo ; dans laquelle $R^5$ et $R^6$ peuvent être pris ensemble pour former un groupe oxo ; dans laquelle chacun de $R^7$ à $R^{11}$ est choisi indépendamment parmi les suivants : hydrido, $C_1$-$C_{20}$-alkyle, $C_1$-$C_{10}$-hydroxy, $C_3$-$C_{10}$-cycloalkyle, $C_3$-$C_{10}$-cycloalkyl-$C_1$-$C_{20}$-alkyle, phén-$C_1$-$C_{20}$-alkylc, phényle, $C_1$-$C_{10}$-alcoxy, $C_1$-$C_{10}$-alcoxyalkyle, phénoxy, phényl-$C_1$-$C_{10}$-alcoxy, $C_1$-$C_{20}$-hydroxy-alkyle, halo et $C_1$-$C_{20}$-haloalkyle ; dans laquelle $R^8$ et $R^9$ peuvent être pris ensemble pour former un groupe oxo ; dans laquelle n est un nombre choisi de 0 à 5, inclus ; dans laquelle chacun des p et q est un élément choisi de 1 à 4 inclus ; dans laquelle X est choisi parmi O, S et N-$R^{12}$ ; dans laquelle $R^{12}$ peut être choisi parmi les suivants : hydrido, $C_1$-$C_{20}$-alkyle, $C_3$-$C_{10}$-cycloalkyle, $C_3$-$C_{10}$-cycloalkyl-$C_1$-$C_{20}$-alkyle, phényle, phén-$C_1$-$C_{20}$-alkyle, un système de noyau aromatique contenant un ou deux hétéroatomes choisis parmi l'oxygène, l'azote, le soufre dans un système à noyau ayant 5 ou 6 chaînons, $C_1$-$C_{10}$-alcoxyalkyle, $C_1$-$C_{20}$-hydroxyalkyle, alcanoyle, phénalcanoyle, aroyle, amino-$C_1$-$C_{20}$-alkyle, mono-$C_1$-$C_{20}$-alkylamino-$C_1$-$C_{20}$-alkyle et di-$C_1$-$C_{20}$-alkylamino-$C_1$-$C_{20}$-alkyle ; dans laquelle $R^{12}$ ensemble avec l'un des $R^1$ à $R^4$ forme un noyau hétérocyclique condensé contenant 5 à 8 chaînons ; dans laquelle chaque Y est indépendamment un ou plusieurs groupes choisis parmi les suivants : hydrido, hydroxy, $C_1$-$C_{20}$-alkyle, $C_3$-$C_{10}$-cycloalkyle, $C_3$-$C_{10}$-cycloalkyl-$C_1$-$C_{20}$-alkyle, phén-$C_1$-$C_{20}$-alkyle, phényle, $C_1$-$C_{10}$-alcoxy, phén-$C_1$-$C_{10}$-alcoxy, phénoxy, $C_1$-$C_{10}$-alcoxyalkyle, $C_1$-$C_{20}$-haloalkyle, $C_1$-$C_{20}$-hydroxyalkyle, halo, cyano, amino, mono-$C_1$-$C_{20}$-alkylamino, di-$C_1$-$C_{20}$-alkylamino, carboxy, carboxy-$C_1$-$C_{20}$-alkyle et alcanoyle ; ou un sel pharmaceutiquement acceptable dudit composé.

**3.** Composé selon la revendication 2, selon laquelle chacun des $R^1$ à $R^4$ est indépendamment choisi parmi les suivants : hydrido, $C_1$-$C_5$-alkyle, cycloalkyle de 3 à 8 atomes de carbone, cycloalkylalkyle de 4 à 8 atomes de carbone, phényl-$C_1$-$C_5$-alkyle, phényle, $C_1$-$C_5$-alcoxy-$C_1$-$C_5$-alkyle, hydroxy-$C_1$-$C_5$-alkyle et halo ; dans laquelle chacun des $R^5$ et $R^6$ est choisi indépendamment parmi les suivants : hydrido, $C_1$-$C_5$-alkyle, phényl-$C_1$-$C_5$-alkyle, phényle et $C_1$-$C_5$-alcoxy-$C_1$-$C_5$-alkyle ; dans laquelle $R^5$ et $R^6$ peuvent être pris ensemble pour former un groupe oxo ; dans laquelle chacun des $R^7$ à $R^{11}$ est choisi indépendamment parmi les suivants : hydrido : $C_1$-$C_5$-alkyle, hydroxy, cycloalkyle de 3 à 8 atomes de carbone, cycloalkylalkyle de 4 à 8 atomes de carbone, phényl-$C_1$-$C_5$-alkyle, phényle, $C_1$-$C_5$-alcoxy, $C_1$-$C_5$-alcoxy-$C_1$-$C_5$-alkyle, phénoxy, phényl-$C_1$-$C_{10}$-alcoxy, hydroxy-$C_1$-$C_5$-alkyle, halo et halo-$C_1$-$C_5$-alkyle ; dans laquelle $R^8$ et $R^9$ peuvent être pris ensemble pour former un groupe oxo ; dans laquelle n est un nombre choisi de 0 à 5 inclus ; dans laquelle chacun des p et q est égal à 2 ou 3 ; dans laquelle X est choisi parmi O, S, $\rangle N$-$R^{12}$ ; dans laquelle $R^{12}$ peut être choisi parmi les suivants : hydrido, $C_1$-$C_5$-alkyle, cycloalkyle de 3 à 8 atomes de carbone, cycloalkylalkyle de 4 à 8 atomes de carbone, phényle, phényl-$C_1$-$C_5$-alkyle, $C_1$-$C_5$-alcoxy-$C_1$-$C_5$-alkyle, hydroxy-$C_1$-$C_5$-alkyle, alcanoyle inférieur et hétéroaryle, choisi parmi les noyaux hétérocycliques saturés, partiellement insaturés et totalement insaturés contenant 5 à 7 chaînons dont 1 ou 2 chaînons sont choisis parmi l'atome

d'oxygène et l'atome d'azote ; dans laquelle $R^{12}$ ensemble avec l'un des $R^1$ à $R^4$ peut former un noyau hétérocyclique condensé contenant 5 ou 6 chaînons ; dans laquelle chaque Y désigne indépendamment un ou plusieurs groupes choisis parmi les suivants : hydrido, hydroxy, $C_1$-$C_5$-alkyle, cycloalkyle de 3 à 8 atomes de carbone, cycloalkylalkyle de 4 à 8 atomes de carbone, phényl-$C_1$-$C_5$-alkyle, phényle, $C_1$-$C_5$-alcoxy, phénoxy, $C_1$-$C_5$-alcoxy-$C_1$-$C_5$-alkyle, halo-$C_1$-$C_5$-alkyle, hydroxy-$C_1$-$C_5$-alkyle, halo, carboxy, carboxy-$C_1$-$C_5$-alkyle et alcanoyle inférieur; ou un sel pharmaceutiquement acceptable dudit composé.

**4.** Composé selon la revendication 3 de formule

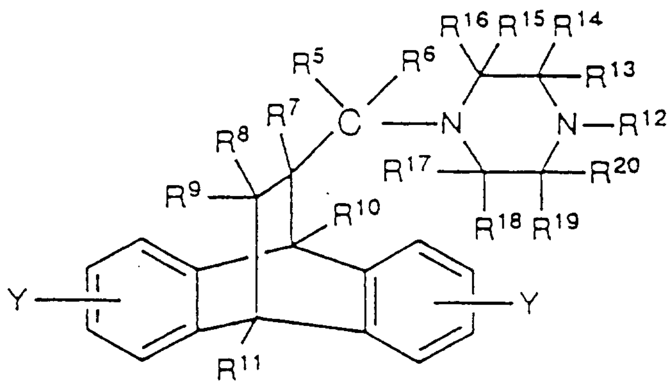

dans laquelle chacun des $R^5$ et $R^6$ est choisi indépendamment parmi les suivants : hydrido, $C_1$-$C_5$-alkyle, benzyle et phényle ; dans laquelle chacun des $R^7$ à $R^{11}$ est choisi indépendamment parmi les suivnats : hydrido, $C_1$-$C_5$-alkyle, hydroxy, benzyle, phényle, $C_1$-$C_5$-alcoxy, phénoxy, benzyloxy, halo et halo-$C_1$-$C_5$-alkyle ; dans laquelle $R^{12}$ peut être choisi parmi les suivants : hydrido, $C_1$-$C_5$-alkyle, cycloalkyle de 5 à 6 atomes de carbone, cycloalkylalkyle de 6 à 7 atomes de carbone, phényle, benzyle, hydroxy-$C_1$-$C_5$-alkyle et hétéroalkyle choisi parmi les noyaux hétérocycliques saturés ou totalement insaturés contenant 5 à 7 chaînons dont 1 ou 2 chaînons sont des atomes d'azote ; dans laquelle chaque Y est indépendamment un ou plusieurs groupes choisis parmi les suivants : hydrido, hydroxy, $C_1$-$C_5$-alkyle, benzyle, phényle, $C_1$-$C_5$-alcoxy, phénoxy, halo-$C_1$-$C_5$-alkyle, halo et alcanoyle inférieur; et dans laquelle chacun des $R^{13}$ à $R^{20}$ est choisi indépendamment parmi les suivants : hydrido, $C_1$-$C_5$-alkyle, benzyle, phényle et halo ; dans laquelle $R^{12}$ ensemble avec l'un des $R^{13}$, $R^{14}$, $R^{19}$ ou $R^{20}$ peut former un noyau hétérocyclique condensé contenant 5 ou 6 chaînons ; ou un sel pharmaceutiquement acceptable dudit composé.

**5.** Composé selon la revendication 4, selon laquelle chacun des $R^5$ et $R^6$ est indépendamment un groupe hydrido ou méthyle ; dans laquelle chacun des $R^7$ à $R^{11}$ est choisi indépendamment parmi les suivants : hydrido, méthyle, éthyle, hydroxy, méthoxy, halo et trihalométhyle ; dans laquelle $R^{12}$ est choisi parmi les suivants : hydrido, méthyle, éthyle, hydroxyéthyle, benzyle, pyridyle et pyrimidyle; dans laquelle chacun des $R^{13}$ à $R^{20}$ est choisi indépendamment parmi les suivants : hydrido, méthyle et éthyle ; dans laquelle $R^{12}$ ensemble avec l'un des $R^{13}$, $R^{14}$, $R^{19}$ ou $R^{20}$ peut former un noyau hétérocyclique condensé contenant 5 ou 6 chaînons ; et dans laquelle chaque Y désigne un ou plusieurs groupes choisis parmi les suivants : hydrido, méthyle, éthyle, hydroxy, méthoxy, trihalométhyle et halo.

**6.** Composé selon la revendication 5 choisi parmi les suivants : 4-[(9,10-dihydro-9,10-éthanoanthracényl)-méthyl]-1-(2-pyrimidyl)pipérazine ; 4-[(9,10-dihydro-9,10-éthanoanthracényl)méthyl]-1,4-diazabicyclo-[4.3.0]-nonane ; et 4-[(9,10-dihydro-9,10-éthanoanthracényl)-méthyl]-1-benzylpipérazine.

**7.** Un procédé de préparation d'une composition pharmaceutique, caractérisé en ce qu'une quantité thérapeutique efficace d'un composé actif est mélangée avec un support ou diluant pharmaceutique acceptable, ledit composé actif étant choisi dans une famille de composés préparée selon l'une des revendications 1, 2, 3, 4, 5 ou 6.

8. Utilisation d'une quantité thérapeutique efficace d'un composé selon l'une quelconque des revendications 1, 2, 4, 5 ou 6 pour la préparation d'un médicament pour le traitement d'un désordre du système nerveux central.

9. Utilisation selon la revendication 8, selon laquelle ledit désordre du système nerveux central est choisi parmi un désordre psychotique, un désordre convulsivant et un désordre dystonique.

10. Utilisation selon la revendication 9, selon laquelle ledit désordre du système nerveux central est un désordre psychotique.

11. Un procédé de préparation d'un composé de formule :

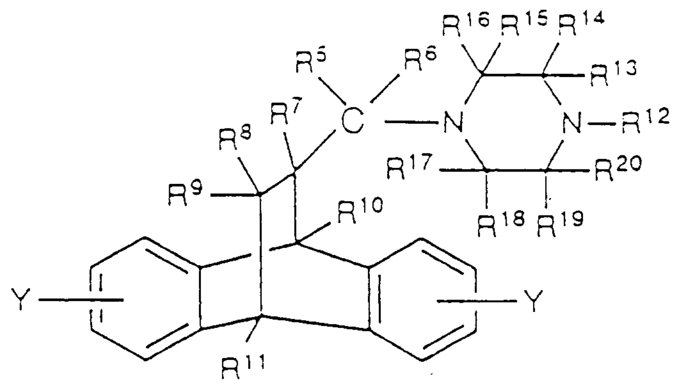

dans laquelle chacun des $R^5$ et $R^6$ est choisi indépendamment parmi les suivants : hydrido, $C_1$-$C_5$-alkyle, benzyle et phényle ; dans laquelle chacun des $R^7$ à $R^{11}$ est choisi indépendamment parmi les suivants : hydrido, $C_1$-$C_5$-alkyle, hydroxy, benzyle, phényle, $C_1$-$C_5$-alcoxy, phénoxy, benzyloxy, halo et halo-$C_1$-$C_5$-alkyle; dans laquelle $R^{12}$ peut être choisi parmi les suivants : hydrido, $C_1$-$C_5$-alkyle, cycloalkyle de 5 ou 6 atomes de carbone, cycloalkylalkyle de 6 ou 7 atomes de carbone, phényle, benzyle, hydroxy-$C_1$-$C_5$-alkyle et hétéroaryle choisi parmi les noyaux hétérocycliques saturés ou totalement insaturés contenant 5 à 7 chaînons dont 1 ou 2 chaînons sont des atomes d'azote ; dans laquelle chaque Y est indépendamment un ou plusieurs groupes choisis parmi les suivants : hydrido, hydroxy, $C_1$-$C_5$-alkyle, benzyle, phényle, $C_1$-$C_5$-alcoxy, phénoxy, halo-$C_1$-$C_5$-alkyle, halo et alcanoyle inférieur; et dans laquelle chacun des $R^{13}$ à $R^{20}$ est choisi indépendamment parmi les suivants : hydrido, $C_1$-$C_5$-alkyle, benzyle, phényle et halo ; dans laquelle $R^{12}$ ensemble avec l'un des $R^{13}$, $R^{14}$, $R^{19}$ et $R^{20}$ peut former un noyau hétérocyclique condensé contenant 5 ou 6 chaînons ; ou un sel pharmaceutiquement acceptable dudit composé;
ladite méthode comprenant la réaction d'un composé de formule :

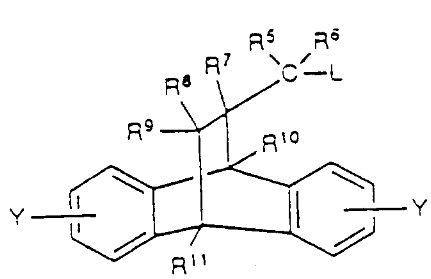

dans laquelle chacun des $R^5$ à $R^{11}$ et Y est défini comme précédemment ; et dans laquelle L est choisi parmi les suivants : halo, hydroxy, para-toluènesulfonyloxy,méthylsulfonyloxy et para-bromotoluènesulfonyloxy ; avec une amine de formule :

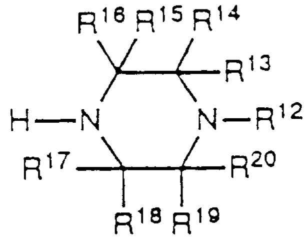

dans laquelle chacun des $R^{12}$ à $R^{20}$ est comme défini précédemment.

**12.** Le procédé selon la revendication 11, selon laquelle chacun des $R^5$ et $R^6$ désignent indépendamment un groupe hydrido ou méthyle ; dans laquelle chacun des $R^7$ à $R^{11}$ est choisi indépendamment parmi les suivants : hydrido, méthyle, éthyle, hydroxy, méthoxy, halo et trihalométhyle ; dans laquelle $R^{12}$ est choisi parmi les suivants : hydrido, méthyle, éthyle, hydroxyéthyle, benzyle, pyridyle et pyrimidyle ; dans laquelle chacun des $R^{13}$ à $R^{20}$ est choisi indépendamment parmi les suivants : hydrido, méthyle, éthyle et trihalométhyle ; dans laquelle $R^{12}$ ensemble avec l'un des $R^{13}$, $R^{14}$, $R^{19}$ ou $R^{20}$ peut former un noyau hétérocyclique condensé contenant 5 ou 6 chaînons ; dans laquelle chaque Y est indépendamment un ou plusieurs groupes choisis parmi les suivants : hydrido, méthyle, éthyle, hydroxy, méthoxy, trihalométhyle et halo; et dans laquelle L est choisi parmi les groupes halo et hydroxy.

**13.** Le procédé selon la revendication 12, selon laquelle L est un groupe halo.

**14.** Le procédé selon la revendication 13, selon laquelle L est un groupe chloro ou bromo.

**15.** Le procédé selon la revendication 12, selon laquelle L est un groupe hydroxy.

**16.** Le procédé selon la revendication 11, selon laquelle un composé de formule :

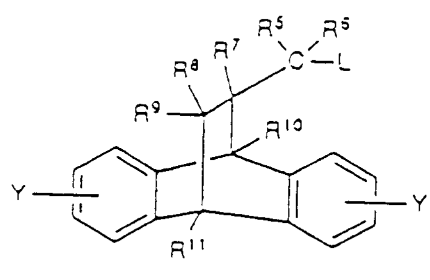

est préparé en mélangeant un alcool de formule :

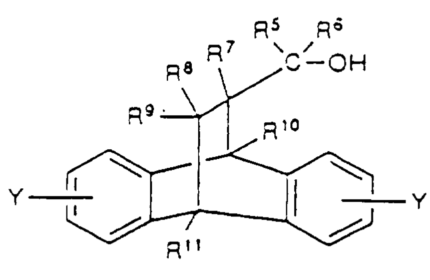

dans laquelle chacun des $R^5$ à $R^{11}$ et L est comme défini précédemment, avec un réactif choisi parmi le chlorure de thionyle, l'oxychlorure de phosphore, le dibromure de triphénylphosphine, le chlorure de méthanesulfonyle et le chlorure de p-toluènesulfonyle.

**17.** Le procédé selon la revendication 11, selon laquelle le produit est choisi parmi les suivants : 4-[(9,10-dihydro-9,10-éthanoanthracényl)méthyl]-1-méthylpipérazine ; 4-[(9,10-dihydro-9,10-éthanoanthracényl)-

méthyl]-1-(2-hydroxyéthyl)-pipérazine ; 4-[(9,10-dihydro-9,10-éthanoanthracényl)méthyl]-1-(2-pyrimidyl)-pipérazine ; 4-[(9,10-dihydro-9,10-éthanoanthracényl)méthyl]-1,4-diazabicyclo[4.3.0]-nonane ; et 4-[-(9,10-dihydro-9,10-éthanoanthracényl)méthyl]-1-benzylpipérazine.

**18.** Le procédé selon la revendication 17, selon laquelle ledit produit est choisi parmi les suivants : 4-[-(9,10-dihydro-9,10-éthanoanthracényl)méthyl]-1-méthylpipérazine ; 4-[(9,10-dihydro-9,10-éthanoanthracényl)méthyl]-1-(2-hydroxyéthyl)-pipérazine ; et 4-[(9,10-dihydro-9,10-éthanoanthracényl)méthyl]-1-benzylpipérazine.